# EUROPÄISCHE PATENTSCHRIFT

(11) **EP 0 915 853 B1**
(45) Veröffentlichungstag und Bekanntmachung des Hinweises auf die Patenterteilung: **06.11.2002**
(21) Anmeldenummer: 97933685.6
(22) Anmeldetag: 21.07.1997
(51) Int. Cl.: C07D 231/20, A01N 43/56

(54) **SUBSTITUIERTE 3-PHENYLPYRAZOLE**
SUBSTITUTED 3-PHENYL PYRAZOLES
3-PHENYLPYRAZOLES SUBSTITUES

(30) Priorität: 01.08.1996 DE 19631008
(43) Veröffentlichungstag der Anmeldung: 19.05.1999
(73) Patentinhaber: BASF AKTIENGESELLSCHAFT, 67056 Ludwigshafen (DE)
(72) Erfinder: ZAGAR, Cyrill, D-67061 Ludwigshafen (DE); HAMPRECHT, Gerhard, D-69469 Weinheim (DE); MENGES, Markus, D-68161 Mannheim (DE); MENKE, Olaf, D-67317 Altleiningen (DE); SCHÄFER, Peter, D-67308 Ottersheim (DE); WESTPHALEN, Karl-Otto, D-67346 Speyer (DE); MISSLITZ, Ulf, D-67433 Neustadt (DE); WALTER, Helmut, D-67283 Obrigheim (DE)
(86) Internationale Anmeldenummer: EP9703901
(87) Internationale Veröffentlichungsnummer: WO98005649

(56) Entgegenhaltungen:
- DE-A- 4 419 517
- DE-A- 4 424 791
- US-A- 5 281 571

## Beschreibung

Die vorliegende Erfindung betrifft neue substituierte 3-Phenylpyrazole der Formel I 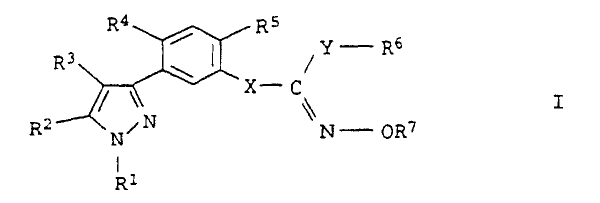 in der die Variablen folgende Bedeutungen haben:
- R¹: C₁-C₄-Alkyl oder C₁-C₄-Halogenalkyl;
- R²: Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
- R³: Wasserstoff, Cyano, Halogen, C₁-C₄-Alkyl oder C₁-C₄-Halogenalkyl;
- R⁴: Wasserstoff oder Halogen;
- R⁵: Cyano, Halogen, C₁-C₄-Alkyl, C₁-C₄-Halogenalkyl, C₁-C₄-Alkoxy oder C₁-C₄-Halogenalkoxy;
- R⁶, R⁷: unabhängig voneinander C₁-C₆-Alkyl, C₁-C₆-Halogenalkyl, Hydroxy-C₁-C₄-alkyl, Cyano-C₁-C₄-alkyl, C₁-C₄-Alkoxy-C₁-C₄-alkyl, C₁-C₄-Halogenalkoxy-C₁-C₄-alkyl, C₃-C₄-Alkenyloxy-C₁-C₄-alkyl, C₃-C₄-Alkinyloxy-C₁-C₄-alkyl, C₃-C₈-Cycloalkyloxy-C₁-C₄-alkyl, Amino-C₁-C₄-alkyl, C₁-C₄-Alkylamino-C₁-C₄-alkyl, Di-(C₁-C₄-alkyl)amino-C₁-C₄-alkyl, C₁-C₄-Alkylthio-C₁-C₄-alkyl, C₁-C₄-Halogenalkylthio-C₁-C₄-alkyl, C₃-C₄-Alkenylthio-C₁-C₄-alkyl, C₃-C₄-Alkinylthio-C₁-C₄-alkyl, C₁-C₄-Alkylsulfinyl-C₁-C₄-alkyl, C₁-C₄-Halogenalkylsulfinyl-C₁-C₄-alkyl, C₃-C₄-Alkenylsulfinyl-C₁-C₄-alkyl, C₃-C₄-Alkinylsulfinyl-C₁-C₄-alkyl, C₁-C₄-Alkylsulfonyl-C₁-C₄-alkyl, C₁-C₄-Halogenalkylsulfonyl-C₁-C₄-alkyl, C₃-C₄-Alkenylsulfonyl-C₁-C₄-alkyl, C₃-C₄-Alkinylsulfonyl-C₁-C₄-alkyl, C₃-C₆-Alkenyl, Cyano-C₃-C₆-alkenyl, C₃-C₆-Halogenalkenyl, C₃-C₆-Alkinyl, Cyano-C₃-C₆-alkinyl, C₃-C₆-Halogenalkinyl, (C₁-C₄-Alkyl)carbonyl, (C₁-C₄-Halogenalkyl)carbonyl, (C₁-C₄-Alkoxy)carbonyl, Aminocarbonyl, (C₁-C₄-Alkyl)aminocarbonyl, Di(C₁-C₄-alkyl)aminocarbonyl, (C₁-C₄-Alkoxy-C₁-C₄-alkyl)carbonyl, C₁-C₄-Alkylsulfonyl, C₁-C₄-Halogenalkylsulfonyl, (C₁-C₄-Alkyl)carbonyl-C₁-C₄-alkyl, (C₁-C₄-Halogenalkyl)carbonyl-C₁-C₄-alkyl, (C₃-C₈-Cycloalkyl)carbonyl-C₁-C₄-alkyl, (C₁-C₄-Alkoxy)imino-C₁-C₄-alkyl, (C₃-C₄-Alkenyloxy)imino-C₁-C₄-alkyl, Hydroxycarbonyl-C₁-C₄-alkyl, (C₁-C₄-Alkoxy)carbonyl-C₁-C₄-alkyl, das eine (C₁-C₄-Alkoxy)imino-Gruppe tragen kann, (C₁-C₄-Halogenalkoxy)carbonyl-C₁-C₄-alkyl, (C₁-C₄-Alkylthio)carbonyl-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl,
C₃-C₈-Cycloalkyl, C₃-C₈-Cycloalkyl-C₁-C₄-alkyl, Phenyl, Phenyl-C₁-C₄-alkyl oder 3- bis 7-gliedriges Heterocyclyl oder Heterocyclyl-C₁-C₄-alkyl, wobei alle Cycloalkylringe und Heterocyclen gewünschtenfalls ein Carbonyl- oder Thiocarbonyl-Ringglied enthalten können,
und wobei jeder Cycloalkyl-, Phenyl- oder Heterocyclylring unsubstituiert sein oder ein bis vier Substituenten tragen kann, jeweils ausgewählt aus der Gruppe bestehend aus Cyano, Nitro, Amino, Hydroxy, Carboxy, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy und Di(C₁-C₄-alkyl)amino;
- X: eine chemische Bindung, eine 1,2-Ethindiyl-Gruppe oder eine über die mit Stern gekennzeichnete Bindung an den Phenylring gebundene Gruppe *-CH₂-CH(R⁸)- oder *-CH=C(R⁸)-, wobei R⁸ für Wasserstoff, Cyano, Nitro, Halogen, C₁-C₄-Alkyl oder C₁-C₄-Halogenalkyl steht;
- Y: Sauerstoff oder Schwefel;
sowie die landwirtschaftlich brauchbaren Salze der Verbindungen I.

Außerdem betrifft die Erfindung
- die Verwendung dieser Verbindungen I als Herbizide,
- herbizide Mittel, welche die Verbindungen I als wirksame Substanzen enthalten,
- Verfahren zur Herstellung der Verbindungen I und von herbiziden Mitteln, unter Verwendung der Verbindungen I, sowie
- Verfahren zur Bekämpfung von unerwünschtem Pflanzenwuchs mit den Verbindungen I.

3-Phenylpyrazole mit herbizider Wirkung wurden bereits in der EP-A 443 059, WO 92/06 962, JP-A 03/151 367, WO 92/02 509, WO 96/15 115, WO 95/32 188, WO 95/33 728, WO 96/01 254, WO 96/02518 und WO 96/40 643 beschrieben.

Die herbiziden Eigenschaften der bekannten Herbizide bezüglich der Schadpflanzen sind jedoch nicht immer völlig befriedigend.

Aufgabe der vorliegenden Erfindung war es deshalb, neue herbizid wirksame Verbindungen bereitzustellen, mit denen sich unerwünschte Pflanzen besser als bisher bekämpfen lassen.

Demgemäß wurden die vorliegenden substituierten 3-Phenylpyrazole der Formel I sowie deren herbizide Wirkung gefunden.

Ferner wurde herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Je nach Bedeutung von X sind auch E-/Z-Isomere möglich. Gegenstand der Erfindung sind sowohl die reinen Enantiomeren oder Diastereomeren als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der Verbindungen I nicht negativ beeinträchtigen. So kommen als Kationen insbesondere die Ionen der Alkalimetalie, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat.

Die für die Substituenten R¹ bis R³ und R⁵ bis R⁸ oder als Reste an Cycloalkyl-, Phenyl- oder Heterocyclylringen genannten organischen Molekülteile stellen Sammelbegriffe für individuelle Aufzählungen der einzelnen Gruppenmitglieder dar. Sämtliche Kohlenstoffketten, also alle Alkyl-, Halogenalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Cyanoalkyl-, Hydroxyalkyl-, Hydroxycarbonylalkyl-, Aminoalkyl-, Aminocarbonylalkyl-, Phenylalkyl-, Heterocyclylalkyl-, Alkylcarbonyl-, Halogenalkylcarbonyl-, Alkylcarbonyloxy-, Halogenalkylcarbonyloxy-, Alkoxycarbonyl-, Halogenalkoxycarbonyl-, Alkylthiocarbonyl-, Alkylamino-, Alkenyl-, Halogenalkenyl-, Cyanoalkenyl-, Alkinyl-, Halogenalkinyl-, Cyanoalkinyl-, Alkenyloxy-, Alkinyloxy-, Alkenylthio-, Alkinylthio-, Alkenylsulfinyl-, Alkinylsulfinyl-, Alkenylsulfonyl- und Alkinylsulfonyl-Teile können geradkettig oder verzweigt sein. Halogenierte Substituenten tragen vorzugsweise ein bis fünf gleiche oder verschiedene Halogenatome.

Die Bedeutung Halogen steht jeweils für Fluor, Chlor, Brom oder Jod, insbesondere für Fluor oder Chlor.

Ferner stehen beispielsweise:
- C₁-C₄-Alkyl für: CH₃, C₂H₅, n-Propyl, CH(CH₃)₂, n-Butyl, 1-Methylpropyl, CH₂-CH(CH₃)₂ oder C(CH₃)₃, insbesondere für CH₃ oder C₂H₅;
- C₁-C₆-Alkyl für: C₁-C₄-Alkyl wie vorstehend genannt, oder z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere für CH₃, C₂H₅, n-Propyl, CH(CH₃)₂, n-Butyl, C(CH₃)₃, n-Pentyl oder n-Hexyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂Cl, CH(Cl)₂, C(Cl)₃, CH₂F, CHF₂, CF₃ CHFCl, CF(Cl)₂, CF₂Cl, CF₂Br, 1-Fluorethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 1,2-Dichlorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, l-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, insbesondere für Chlormethyl, Fluormethyl, Difluormethyl, Trifluormethyl, 2-Fluorethyl, 2-Chlorethyl, 1,2-Dichlorethyl, 2,2,2-Trifluorethyl oder Pentafluorethyl;
- C₁-C₆-Halogenalkyl für: C₁-C₆-Alkyl wie vorstehend genannt, das partiell oder vollständig durch Fluor, Chlor und/oder Brom substituiert ist, also z.B. einen der vorstehend genannten C₁-C₄-Halogenalkylreste, oder 5-Fluorpentyl, 5-Chlorpentyl, 5-Brompentyl, 5-Iodpentyl, 5,5,5-Trichlorpentyl, Undecafluorpentyl, 6-Fluorhexyl, 6-Chlorhexyl, 6-Bromhexyl, 6-Iodhexyl, 6,6,6-Trichlorhexyl oder Dodecafluorhexyl, insbesondere für Fluormethyl, Difluormethyl, Trifluormethyl, Chlormethyl, 2-Fluorethyl, 2-Chlorethyl oder 2,2,2-Trifluorethyl;
- C₃-C₈-Cycloalkyl für: Cyclopropyl, Cyclobutyl, Cyclopentyl, Cyclohexyl, Cycloheptyl oder Cyclooctyl, insbesondere für Cyclopentyl oder Cyclohexyl;
- C₃-C₈-Cycloalkyl, das ein Carbonyl- oder Thiocarbonyl-Ringglied enthält, für: z.B. Cyclobutanon-2-yl, Cyclobutanon-3-yl, Cyclopentanon-2-yl, Cyclopentanon-3-yl, Cyclohexanon-2-yl, Cyclohexanon-4-yl, Cycloheptanon-2-yl, Cyclooctanon-2-yl, Cyclobutanthion-2-yl, Cyclobutanthion-3-yl, Cyclopentanthion-2-yl, Cyclopentanthion-3-yl, Cyclohexanthion-2-yl, Cyclohexanthion-4-yl, Cycloheptanthion-2-yl oder Cyclooctanthion-2-yl;
- C₁-C₄-Alkoxy für: OCH₃, OC₂H₅, n-Propoxy, OCH(CH₃)₂, n-Butoxy, 1-Methylpropoxy, 2-Methylpropoxy oder OC(CH₃)₃, insbesondere OCH₃, OC₂H₅, OCH(CH₃)₂ oder OC(CH₃)₃;
- C₁-C₄-Halogenalkoxy: C₁-C₄-Alkoxy wie vorstehend genannt, das partiell oder vollständig durch Fluor, Chlor, Brom und/oder Jod substituiert ist, also z.B. OCH₂Cl, OCH(Cl)₂, OC(Cl)₃, OCH₂F, OCHF₂, OCF₃ OCHFCl, OCF(Cl)₂, OCF₂Cl, OCF₂Br, 1-Fluorethoxy, 2-Fluorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 1,2-Dichlorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, insbesondere für Chlormethoxy, Fluormethoxy, Difluormethoxy, Trifluormethoxy, 2-Fluorethoxy, 2-Chlorethoxy, 1,2-Dichlorethoxy, 2,2,2-Trifluorethoxy oder Pentafluorethoxy;
- C₁-C₄-Alkylthio für: SCH₃, SC₂H₅, n-Propylthio, SCH(CH₃)₂, n-Butylthio, 1-Methylpropylthio, 2-Methylpropylthio oder SC(CH₃)₃, insbesondere für SCH₃ oder SC₂H₅;
- C₁-C₄-Halogenalkylthio für: C₁-C₄-Alkylthio wie vorstehend genannt, das partiell oder vollständig durch Fluor, Chlor, Brom und/oder Jod substituiert ist, also z.B. SCH₂Cl, SCH(Cl)₂, SC(Cl)₃, SCH₂F, SCHF₂, SCF₃, SCHFCl, SCF(Cl)₂, SCF₂Cl, SCF₂Br, 1-Fluorethylthio, 2-Fluorethylthio, 2-Chlorethylthio, 2-Bromethylthio, 2-Iodethylthio, 2,2-Difluorethylthio, 2,2,2-Trifluorethylthio, 1,2-Dichlorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3, 3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutoxy, insbesondere für Chlormethylthio, Fluormethylthio, Difluormethylthio, Trifluormethylthio, 2-Fluorethylthio, 2-Chlorethylthio, 1,2-Dichlorethylthio, 2,2,2-Trifluorethylthio oder Pentafluorethylthio;
- C₁-C₄-Alkylsulfinyl für: SOCH₃, SOC₂H₅, n-Propylsulfinyl, SOCH(CH₃)₂, n-Butylsulfinyl, 1-Methylpropylsulfinyl, 2-Methylpropylsulfinyl oder SOC(CH₃)₃, insbesondere für SOCH₃ oder SOC₂H₅;
- C₁-C₄-Halogenalkylsulfinyl für: einen C₁-C₄-Alkylsulfinylrest wie vorstehend genannt, der partiell oder vollständig durch Fluor, Chlor, Brom und/oder Iod substituiert ist, also z.B. Chlormethylsulfinyl, Dichlormethylsulfinyl, Trichlormethylsulfinyl, Fluormethylsulfinyl, Difluormethylsulfinyl, Trifluormethylsulfinyl, Chlorfluormethylsulfinyl, Dichlorfluormethylsulfinyl, Chlordifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2-Chlor-2-fluorethylsulfinyl, 2-Chlor-2,2-difluorethylsulfinyl, 2,2-Dichlor-2-fluorethylsulfinyl, 1,2-Dichlorethylsulfinyl, 2,2,2-Trichlorethylsulfinyl, Pentafluorethylsulfinyl, 2-Fluorpropylsulfinyl, 3-Fluorpropylsulfinyl, 2,2-Difluorpropylsulfinyl, 2,3-Difluorpropylsulfinyl, 2-Chlorpropylsulfinyl, 3-Chlorpropylsulfinyl, 2,3-Dichlorpropylsulfinyl, 2-Brompropylsulfinyl, 3-Brompropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl oder Nonafluorbutylsulfinyl, insbesondere für Chlormethylsulfinyl, Fluormethylsulfinyl, Difluormethylsulfinyl, Trifluormethylsulfinyl, 2-Fluorethylsulfinyl, 2-Chlorethylsulfinyl, 1,2-Dichlorethylsulfinyl, 2,2,2-Trifluorethylsulfinyl oder Pentafluorethylsulfinyl;
- C₁-C₄-Alkylsulfonyl für: SO₂CH₃, SO₂C₂H₅, n-Propylsulfonyl, SO₂CH(CH₃)₂, n-Butylsulfonyl, 1-Methylpropylsulfonyl, 2-Methylpropylsulfonyl oder SO₂C(CH₃)₃, insbesondere für SO₂CH₃ oder SO₂C₂H₅;
- C₁-C₄-Halogenalkylsulfonyl für: einen C₁-C₄-Alkylsulfonylrest wie vorstehend genannt, der partiell oder vollständig durch Fluor, Chlor, Brom und/oder Iod substituiert ist, also z.B. Chlormethylsulfonyl, Dichlormethylsulfonyl, Trichlormethylsulfonyl, Fluormethylsulfonyl, Difluormethylsulfonyl, Trifluormethylsulfonyl, Chlorfluormethylsulfonyl, Dichlorfluormethylsulfonyl, Chlordifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 1,2-Dichlorethylsulfonyl, 2,2,2-Trichlorethylsulfonyl, Pentafluorethylsulfonyl, 2-Fluorpropylsulfonyl, 3-Fluorpropylsulfonyl, 2,2-Difluorpropylsulfonyl, 2,3-Difluorpropylsulfonyl, 2-Chlorpropylsulfonyl, 3-Chlorpropylsulfonyl, 2,3-Dichlorpropylsulfonyl, 2-Brompropylsulfonyl, 3-Brompropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl, insbesondere für Chlormethylsulfonyl, Fluormethylsulfonyl, Difluormethylsulfonyl, Trifluormethylsulfonyl, 2-Fluorethylsulfonyl, 2-Chlorethylsulfonyl, 1,2-Dichlorethylsulfonyl, 2,2,2-Trifluorethylsulfonyl oder Pentafluorethylsulfonyl;
- Cyano-C₁-C₄-alkyl für: z.B. CH₂CN, 1-Cyanoethyl, 2-Cyanoethyl, 1-Cyanoprop-1-yl, 2-Cyanoprop-1-yl 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 3-Cyanobut-2-yl, 4-Cyanobut-2-yl, 1-(CH₂CN)-eth-1-yl, 1-(CH₂CN)-1-(CH₃)-eth-1-yl oder 1-(CH₂CN)-prop-1-yl, insbesondere für CH₂CN oder 2-Cyanoethyl;
- Hydroxy-C₁-C₄-alkyl für: z.B. CH₂OH, 1-Hydroxyethyl, 2-Hydroxyethyl, 1-Hydroxyprop-1-yl, 2-Hydroxyprop-1-yl, 3-Hydroxyprop-1-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 3-Hydroxybut-2-yl, 3-Hydroxybut-2-yl, 4-Hydroxybut-2-yl, 1-(CH₂OH)eth-1-yl, 1-(CH₂OH)-1-(CH₃)-eth-1-yl oder 1-(CH₂OH)prop-1-yl, insbesondere für CH₂OH oder 2-Hydroxyethyl;
- Hydroxycarbonyl-C₁-C₄-alkyl für: z.B. CH₂COOH, 1-(COOH)ethyl, 2-(COOH)ethyl, 1-(COOH)prop-1-yl, 2-(COOH)prop-1-yl, 3-(COOH)prop-1-yl, 1-(COOH)but-1-yl, 2-(COOH)but-1-yl, 3-(COOH)but-1-yl, 4-(COOH)but-1-yl, 1-(COOH)but-2-yl, 2-(COOH)but-2-yl, 3-(COOH)but-2-yl, 3-(COOH)but-2-yl, 4-(COOH)but-2-yl, 1-(CH₂COOH)eth-1-yl, 1-(CH₂COOH)-1-(CH₃)-eth-1-yl oder 1-(CH₂COOH)prop-1-yl, insbesondere für CH₂-COOH oder 2-Hydroxycarbonylethyl;
- Amino-C₁-C₄-alkyl für: z.B. CH₂NH₂, 1-Aminoethyl, 2-Aminoethyl, 1-Aminoprop-1-yl, 2-Aminoprop-1-yl, 3-Aminoprop-1-yl, 1-Aminobut-1-yl, 2-Aminobut-1-yl, 3-Aminobut-1-yl, 4-Aminobut-1-yl, 1-Aminobut-2-yl, 2-Aminobut-2-yl, 3-Aminobut-2-yl, 3-Aminobut-2-yl, 4-Aminobut-2-yl, 1-(CH₂NH₂)eth-1-yl, 1-(CH₂NH₂)-₁-(CH₃)-eth-1-yl oder 1-(CH₂NH₂)prop-1-yl, insbesondere für CH₂NH₂ oder 2-Aminoethyl;
- Aminocarbonyl-C₁-C₄-alkyl für: z.B. CH₂CONH₂, 1-(CONH₂)ethyl, 2-(CONH₂)ethyl, 1-(CONH₂)prop-1-yl, 2-(CONH₂)prop-1-yl, 3-(CONH₂)prop-1-yl, 1-(CONH₂)but-1-yl, 2-(CONH₂)but-1-yl, 3-(CONH₂)but-1-yl, 4-(CONH₂)but-1-yl, 1-(CONH₂)but-2-yl, 2-(CONH₂)but-2-yl, 3-(CONH₂)but-2-yl, 3-(CONH₂)but-2-yl, 4-(CONH₂)but-2-yl, 1-(CH₂CONH₂)eth-1-yl, 1-(CH₂CONH₂)-1-(CH₃)-eth-1-yl oder 1-(CH₂CONH₂)prop-1-yl, insbesondere für CH₂CONH₂ oder 2-Aminocarbonylethyl;
- Phenyl-C₁-C₄-alkyl für: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl oder 1-(Phenylmethyl)-prop-1-yl, insbesondere für Benzyl oder 2-Phenylethyl;
- C₃-C₈-Cycloalkyl-C₁-C₄-alkyl für: Cyclopropylmethyl, 1-Cyclopropyl-ethyl, 2-Cyclopropyl-ethyl, 1-Cyclopropyl-prop-l-yl, 2-Cyclopropyl-prop-1-yl, 3-Cyclopropyl-prop-1-yl, 1-Cyclopropyl-but-1-yl, 2-Cyclopropyl-but-1-yl, 3-Cyclopropyl-but-1-yl, 4-Cyclopropyl-but-1-yl, 1-Cyclopropyl-but-2-yl, 2-Cyclopropyl-but-2-yl, 3-Cyclopropyl-but-2-yl, 3-Cyclopropyl-but-2-yl, 4-Cyclopropyl-but-2-yl, 1-(Cyclopropylmethyl)eth-1-yl, 1-(Cyclopropylmethyl)-1-(CH₃)-eth-1-yl, 1-(Cyclopropylmethyl)prop-1-yl, Cyclobutylmethyl, 1-Cyclobutyl-ethyl, 2-Cyclobutyl-ethyl, 1-Cyclobutyl-prop-l-yl, 2-Cyclobutyl-prop-1-yl, 3-Cyclobutyl-prop-1-yl, 1-Cyclobutyl-but-1-yl, 2-Cyclobutyl-but-1-yl, 3-Cyclobutyl-but-1-yl, 4-Cyclobutyl-but-1-yl, 1-Cyclobutyl-but-2-yl, 2-Cyclobutylbut-2-yl, 3-Cyclobutyl-but-2-yl, 3-Cyclobutyl-but-2-yl, 4-Cyclobutyl-but-2-yl, 1-(Cyclobutylmethyl)eth-1-yl, 1-(Cyclobutylmethyl)-1-(CH₃)-eth-1-yl, 1-(Cyclobutylmethyl)prop-1-yl, Cyclopentylmethyl, 1-Cyclopentyl-ethyl, 2-Cyclopentyl-ethyl, 1-Cyclopentyl-prop-1-yl, 2-Cyclopentyl-prop-1-yl, 3-Cyclopentyl-prop-1-yl, 1-Cyclopentyl-but-1-yl, 2-Cyclopentyl-but-1-yl, 3-Cyclopentyl-but-1-yl, 4-Cyclopentyl-but-1-yl, 1-Cyclopentyl-but-2-yl, 2-Cyclopentylbut-2-yl, 3-Cyclopentyl-but-2-yl, 3-Cyclopentyl-but-2-yl, 4-Cyclopentyl-but-2-yl, 1-(Cyclopentylmethyl)eth-1-yl, 1-(Cyclopentylmethyl)-1-(CH₃)-eth-1-yl, 1-(Cyclopentylmethyl)prop-1-yl, Cyclohexylmethyl, 1-Cyclohexyl-ethyl, 2-Cyclohexyl-ethyl, 1-Cyclohexyl-prop-1-yl, 2-Cyclohexyl-prop-1-yl, 3-Cyclohexyl-prop-1-yl, 1-Cyclohexyl-but-1-yl, 2-Cyclohexyl-but-1-yl, 3-Cyclohexyl-but-1-yl, 4-Cyclohexyl-but-1-yl, 1-Cyclohexyl-but-2-yl, 2-Cyclohexyl-but-2-yl, 3-Cyclohexyl-but-2-yl, 3-Cyclohexyl-but-2-yl, 4-Cyclohexylbut-2-yl, 1-(Cyclohexylmethyl)eth-1-yl, 1-(Cyclohexylmethyl)-1-(CH₃)-eth-1-yl, 1-(Cyclohexylmethyl)prop-1-yl, Cycloheptylmethyl, 1-Cycloheptyl-ethyl, 2-Cycloheptyl-ethyl, 1-Cycloheptyl-prop-1-yl, 2-Cycloheptyl-prop-1-yl, 3-Cycloheptyl-prop-1-yl, 1-Cycloheptyl-but-1-yl, 2-Cycloheptyl-but-1-yl, 3-Cycloheptyl-but-1-yl, 4-Cycloheptyl-but-1-yl, 1-Cycloheptyl-but-2-yl, 2-Cycloheptyl-but-2-yl, 3-Cycloheptyl-but-2-yl, 3-Cycloheptyl-but-2-yl, 4-Cycloheptyl-but-2-yl, 1-(Cycloheptylmethyl)eth-1-yl, 1-(Cycloheptylmethyl)-1-(CH₃)-eth-1-yl, 1-(Cycloheptylmethyl)prop-1-yl, Cyclooctylmethyl, 1-Cyclooctyl-ethyl, 2-Cyclooctyl-ethyl, 1-Cyclooctyl-prop-1-yl, 2-Cyclooctyl-prop-1-yl, 3-Cyclooctyl-prop-1-yl, 1-Cyclooctyl-but-1-yl, 2-Cyclooctyl-but-1-yl, 3-Cyclooctyl-but-1-yl, 4-Cyclooctyl-but-1-yl, 1-Cyclooctyl-but-2-yl, 2-Cyclooctyl-but-2-yl, 3-Cyclooctyl-but-2-yl, 3-Cyclooctylbut-2-yl, 4-Cyclooctyl-but-2-yl, 1-(Cyclooctylmethyl)eth-1-yl, 1-(Cyclooctylmethyl)-1-(CH₃)-eth-1-yl oder 1-(Cyclooctylmethyl)prop-1-yl, insbesondere für C₃-C₆-Cycloalkylmethyl;
- C₃-C₈-Cycloalkyl-C₁-C₄-alkyl, das ein Carbonyl- oder Thiocarbonyl-Ringglied enthält, für: z.B. Cyclobutanon-2-ylmethyl, Cyclobutanon-3-ylmethyl, Cyclopentanon-2-ylmethyl, Cyclopentanon-3-ylmethyl, Cyclohexanon-2-ylmethyl, Cyclohexanon-4-ylmethyl, Cycloheptanon-2-ylmethyl, Cyclooctanon-2-ylmethyl, Cyclobutanthion-2-ylmethyl, Cyclobutanthion-3-ylmethyl, Cyclopentanthion-2-ylmethyl, Cyclopentanthion-3-ylmethyl, Cyclohexanthion-2-ylmethyl, Cyclohexanthion-4-ylmethyl, Cycloheptanthion-2-ylmethyl, Cyclooctanthion-2-ylmethyl, 1-(Cyclobutanon-2-yl)ethyl, 1-(Cyclobutanon-3-yl)ethyl, 1-(Cyclopentanon-2-yl)ethyl, 1-(Cyclopentanon-3-yl)ethyl, 1-(Cyclohexanon-2-yl)ethyl, 1-(Cyclohexanon-4-yl)ethyl, 1-(Cycloheptanon-2-yl)ethyl, 1-(Cyclooctanon-2-yl)ethyl, 1-(Cyclobutanthion-2-yl)ethyl, 1-(Cyclobutanthion-3-yl)ethyl, 1-(Cyclopentanthion-2-yl)ethyl, 1-(Cyclopentanthion-3-yl)ethyl, 1-(Cyclohexanthion-2-yl)-ethyl, 1-(Cyclohexanthion-4-yl)ethyl, 1-(Cycloheptanthion-2-yl)ethyl, 1-(Cyclooctanthion-2-yl)ethyl, 2-(Cyclobutanon-2-yl)ethyl, 2-(Cyclobutanon-3-yl)ethyl, 2-(Cyclopentanon-2-yl)ethyl, 2-(Cyclopentanon-3-yl)ethyl, 2-(Cyclohexanon-2-yl)ethyl, 2-(Cyclohexanon-4-yl)ethyl, 2-(Cycloheptanon-2-yl)ethyl, 2-(Cyclooctanon-2-yl)ethyl, 2-(Cyclobutanthion-2-yl)ethyl, 2-(Cyclobutanthion-3-yl)ethyl, 2-(Cyclopentanthion-2-yl)ethyl, 2-(Cyclopentanthion-3-yl)ethyl, 2-(Cyclohexanthion-2-yl)ethyl, 2-(Cyclohexanthion-4-yl)ethyl, 2-(Cycloheptanthion-2-yl)ethyl, 2-(Cyclooctanthion-2-yl)ethyl, 3-(Cyclobutanon-2-yl)propyl, 3-(Cyclobutanon-3-yl)propyl, 3-(Cyclopentanon-2-yl)propyl, 3-(Cyclopentanon-3-yl)propyl, 3-(Cyclohexanon-2-yl)propyl, 3-(Cyclohexanon-4-yl)propyl, 3-(Cycloheptanon-2-yl)propyl, 3-(Cyclooctanon-2-yl)propyl, 3-(Cyclobutanthion-2-yl)propyl, 3-(Cyclobutanthion-3-yl)propyl, 3-(Cyclopentanthion-2-yl)propyl, 3-(Cyclopentanthion-3-yl)propyl, 3-(Cyclohexanthion-2-yl)propyl, 3-(Cyclohexanthion-4-yl)propyl, 3-(Cycloheptanthion-2-yl)propyl, 3-(Cyclooctanthion-2-yl)propyl, 4-(Cyclobutanon-2-yl)butyl, 4-(Cyclobutanon-3-yl)butyl, 4-(Cyclopentanon-2-yl)butyl, 4-(Cyclopentanon-3-yl)butyl, 4-(Cyclohexanon-2-yl)butyl, 4-(Cyclohexanon-4-yl)butyl, 4-(Cycloheptanon-2-yl)butyl, 4-(Cyclooctanon-2-yl)butyl, 4-(Cyclobutanthion-2-yl)butyl, 4-(Cyclobutanthion-3-yl)butyl, 4-(Cyclopentanthion-2-yl)butyl, 4-(Cyclopentanthion-3-yl)butyl, 4-(Cyclohexanthion-2-yl)butyl, 4-(Cyclohexanthion-4-yl)butyl, 4-(Cycloheptanthion-2-yl)butyl oder 4-(Cyclooctanthion-2-yl)butyl;
- Heterocyclyl-C₁-C₄-alkyl für: Heterocyclylmethyl, 1-(Heterocyclyl)ethyl, 2-(Heterocyclyl)ethyl, 1-(Heterocyclyl)prop-1-yl, 2-(Heterocyclyl)prop-1-yl, 3-(Heterocyclyl)prop-1-yl, 1-(Heterocyclyl)but-1-yl, 2-(Heterocyclyl)but-1-yl, 3-(Heterocyclyl)but-1-yl, 4-(Heterocyclyl)but-1-yl, 1-(Heterocyclyl)but-2-yl, 2-(Heterocyclyl)but-2-yl, 3-(Heterocyclyl)but-2-yl, 3-(Heterocyclyl)but-2-yl, 4-(Heterocyclyl)but-2-yl, 1-(Heterocyclylmethyl)eth-1-yl, 1-(Heterocyclylmethyl)-1-(CH₃)-eth-1-yl oder 1-(Heterocyclylmethyl)prop-1-yl, insbesondere für Heterocyclylmethyl oder 2-(Heterocyclyl)ethyl;
- C₁-C₄-Alkoxy-C₁-C₄-alkyl sowie der Alkoxyalkyl-Teil von (C₁-C₄-Alkoxy-C₁-C₄-alkyl)carbonyl für: durch C₁-C₄-Alkoxy wie vorstehend genannt substituiertes C₁-C₄-Alkyl, also z.B. für CH₂OCH₃, CH₂OC₂H₅, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(OCH₃)propyl, 2-(OC₂H₅)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(OCH₃)propyl, 3-(OC₂H₅)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3(1,1-Dimethylethoxy)propyl, 2-(OCH₃)butyl, 2-(OC₂H₅)butyl, 2-(n-Propoxy)-butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(OCH₃)butyl, 3-(OC₂H₅)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(OCH₃)butyl, 4-(OC₂H₅)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, insbesondere für Methoxymethyl oder 2-Methoxyethyl;
- (C₁-C₄-Alkoxy)imino-C₁-C₄-alkyl für: durch C₁-C₄-Alkoxyimino wie Methoxyimino, Ethoxyimino, n-Propoxyimino, 1-Methylethoxyimino, n-Butoxyimino, 1-Methylpropoxyimino, 2-Methylpropoxyimino oder 1,1-Dimethylethoxyimino, insbesondere Methoxyimino oder Ethoxyimino, substituiertes C₁-C₄-Alkyl, also z.B. für 2-(Methoxyimino)ethyl, 2-(Ethoxyimino)ethyl, 2-(n-Propoxyimino)ethyl, 2-(1-Methylethoxyimino)ethyl, 2-(n-Butoxyimino)ethyl, 2-(1-Methylpropoxyimino)ethyl, 2-(2-Methylpropoxyimino)ethyl, 2-(1,1-Dimethylethoxyimino)ethyl, 2-(Methoxyimino)propyl, 2-(Ethoxyimino)propyl, 2-(n-Propoxyimino)propyl, 2-(1-Methylethoxyimino)propyl, 2-(n-Butoxyimino)propyl, 2-(1-Methylpropoxyimino)propyl, 2-(2-Methylpropoxyimino)propyl, 2-(1,1-Dimethylethoxyimino)propyl, 3-(Methoxyimino)propyl, 3-(Ethoxyimino)propyl, 3-(n-Propoxyimino)propyl, 3-(1-Methylethoxyimino)propyl, 3-(n-Butoxyimino)propyl, 3-(1-Methylpropoxyimino)propyl, 3-(2-Methylpropoxyimino)propyl, 3-(1,1-Dimethylethoxyimino)propyl, 2-(Methoxyimino)butyl, 2-(Ethoxyimino)butyl, 2-(n-Propoxyimino)butyl, 2-(1-Methylethoxyimino)butyl, 2-(n-Butoxyimino)butyl, 2-(1-Methylpropoxyimino)butyl, 2-(2-Methylpropoxyimino)butyl, 2-(1,1-Dimethylethoxyimino)butyl, 3-(Methoxyimino)butyl, 3-(Ethoxyimino)butyl, 3-(n-Propoxyimino)butyl, 3-(1-Methylethoxyimino)butyl, 3-(n-Butoxyimino)butyl, 3-(1-Methylpropoxyimino)butyl, 3-(2-Methylpropoxyimino)butyl, 3-(1,1-Dimethylethoxyimino)butyl, 4-(Methoxy)butyl, 4-(Ethoxyimino)butyl, 4-(n-Propoxyimino)butyl, 4-(1-Methylethoxyimino)butyl, 4-(n-Butoxyimino)butyl, 4-(1-Methylpropoxyimino)butyl, 4-(2-Methylpropoxyimino)butyl oder 4-(1,1-Dimethylethoxyimino)butyl, insbesondere für 2-(Methoxyimino)ethyl oder 2-(Ethoxyimino)ethyl;
- C₁-C₄-Halogenalkoxy-C₁-C₄-alkyl für: durch C₁-C₄-Halogenalkoxy wie vorstehend genannt substituiertes C₁-C₄-Alkyl, also z.B. für 2-(Difluormethoxy)ethyl, 2-(Trifluormethoxy)ethyl oder 2-(Pentafluorethoxy)ethyl;
- C₃-C₈-Cycloalkyloxy-C₁-C₄-alkyl für: Cyclopropyloxymethyl, 1-Cyclopropyloxy-ethyl, 2-Cyclopropyloxy-ethyl, 1-Cyclopropyloxy-prop-1-yl, 2-Cyclopropyloxy-prop-1-yl, 3-Cyclopropyloxy-prop-1-yl, 1-Cyclopropyloxy-but-1-yl, 2-Cyclopropyloxy-but-1-yl, 3-Cyclopropyloxy-but-1-yl, 4-Cyclopropyloxy-but-1-yl, 1-Cyclopropyloxy-but-2-yl, 2-Cyclopropyloxybut-2-yl, 3-Cyclopropyloxy-but-2-yl, 3-Cyclopropyloxy-but-2-yl, 4-Cyclopropyloxy-but-2-yl, 1-(Cyclopropyloxymethyl)eth-1-yl, 1-(Cyclopropyloxymethyl)-1-(CH₃)-eth-1-yl, 1-(Cyclopropylmethyloxy)prop-1-yl, Cyclobutyloxymethyl, 1-Cyclobutyloxy-ethyl, 2-Cyclobutyloxy-ethyl, 1-Cyclobutyloxy-prop-1-yl, 2-Cyclobutyloxy-prop-1-yl, 3-Cyclobutyloxy-prop-1-yl, 1-Cyclobutyloxy-but-1-yl, 2-Cyclobutyloxy-but-1-yl, 3-Cyclobutyloxy-but-1-yl, 4-Cyclobutyloxy-but-1-yl, 1-Cyclobutyloxy-but-2-yl, 2-Cyclobutyloxy-but-2-yl, 3-Cyclobutyloxy-but-2-yl, 3-Cyclobutyloxy-but-2-yl, 4-Cyclobutyloxybut-2-yl, 1-(Cyclobutyloxymethyl)eth-1-yl, 1-(Cyclobutyloxymethyl)-1-(CH₃)-eth-1-yl, 1-(Cyclobutyloxymethyl)prop-1-yl, Cyclopentyloxymethyl, 1-Cyclopentyloxy-ethyl, 2-Cyclopentyloxy-ethyl, 1-Cyclopentyloxy-prop-1-yl, 2-Cyclopentyloxy-prop-1-yl, 3-Cyclopentyloxy-prop-1-yl, 1-Cyclopentyloxy-but-1-yl, 2-Cyclopentyloxy-but-1-yl, 3-Cyclopentyloxy-but-1-yl, 4-Cyclopentyloxy-but-1-yl, 1-Cyclopentyloxy-but-2-yl, 2-Cyclopentyloxy-but-2-yl, 3-Cyclopentyloxy-but-2-yl, 3-Cyclopentyloxy-but-2-yl, 4-Cyclopentyloxy-but-2-yl, 1-(Cyclopentyloxymethyl)eth-1-yl, 1-(Cyclopentyloxymethyl)-1-(CH₃)-eth-1-yl, 1-(Cyclopentyloxymethyl)prop-1-yl, Cyclohexyloxymethyl, 1-Cyclohexyloxy-ethyl, 2-Cyclohexyloxy-ethyl, 1-Cyclohexyloxy-prop-1-yl, 2-Cyclohexyloxy-prop-1-yl, 3-Cyclohexyloxy-prop-1-yl, 1-Cyclohexyloxy-but-1-yl, 2-Cyclohexyloxy-but-1-yl, 3-Cyclohexyloxy-but-1-yl, 4-Cyclohexyloxy-but-1-yl, 1-Cyclohexyloxy-but-2-yl, 2-Cyclohexyloxy-but-2-yl, 3-Cyclohexyloxy-but-2-yl, 3-Cyclohexyloxy-but-2-yl, 4-Cyclohexyloxy-but-2-yl, 1-(Cyclohexyloxymethyl)eth-1-yl, 1-(Cyclohexyloxymethyl)-1-(CH₃)-eth-1-yl, 1-(Cyclohexyloxymethyl)prop-1-yl, Cycloheptyloxymethyl, 1-Cycloheptyloxy-ethyl, 2-Cycloheptyloxy-ethyl, 1-Cycloheptyloxy-prop-1-yl, 2-Cycloheptyloxy-prop-1-yl, 3-Cycloheptyloxy-prop-1-yl, 1-Cycloheptyloxy-but-1-yl, 2-Cycloheptyloxy-but-1-yl, 3-Cycloheptyloxy-but-1-yl, 4-Cycloheptyloxy-but-1-yl, 1-Cycloheptyloxybut-2-yl, 2-Cycloheptyloxy-but-2-yl, 3-Cycloheptyloxybut-2-yl, 3-Cycloheptyloxy-but-2-yl, 4-Cycloheptyloxy-but-2-yl, 1-(Cycloheptyloxymethyl)eth-1-yl, 1-(Cycloheptyloxymethyl)-1-(CH₃)-eth-1-yl, 1-(Cycloheptyloxymethyl)prop-1-yl, Cyclooctyloxymethyl, 1-Cyclooctyloxy-ethyl, 2-Cyclooctyloxy-ethyl, 1-Cyclooctyloxy-prop-1-yl, 2-Cyclooctyloxyprop-1-yl, 3-Cyclooctyloxy-prop-1-yl, 1-Cyclooctyloxy-but-1-yl, 2-Cyclooctyloxy-but-1-yl, 3-Cyclooctyloxy-but-1-yl, 4-Cyclooctyloxy-but-1-yl, 1-Cyclooctyloxy-but-2-yl, 2-Cyclooctyloxy-but-2-yl, 3-Cyclooctyloxy-but-2-yl, 3-Cyclooctyloxybut-2-yl, 4-Cyclooctyloxy-but-2-yl, 1-(Cyclooctyloxymethyl)-eth-1-yl, 1-(Cyclooctyloxymethyl)-1-(CH₃)-eth-1-yl oder 1-(Cyclooctyloxymethyl)prop-1-yl, insbesondere für C₃-C₆-Cycloalkoxymethyl oder 2-(C₃-C₆-Cycloalkoxy)ethyl;
- C₁-C₄-Alkylthio-C₁-C₄-alkyl für: durch C₁-C₄-Alkylthio wie vorstehend genannt substituiertes C₁-C₄-Alkyl, also z.B. für CH₂SCH₃, CH₂SC₂H₅, n-Propylthiomethyl, CH₂SCH(CH₃)₂, n-Butylthiomethyl, (1-Methylpropylthio)methyl, (2-Methylpropylthio)methyl, CH₂SC(CH₃)₃, 2-Methylthioethyl, 2-Ethylthioethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(SCH₃)propyl, 3-(SCH₃)propyl, 2-(SC₂H₅)propyl, 3-(SC₂H₅)propyl, 3-(Propylthio)propyl, 3-(Butylthio)propyl, 4-(SCH₃)butyl, 4-(SC₂H₅)butyl, 4-(n-Propylthio)butyl oder 4-(n-Butylthio)butyl, insbesondere für CH₂SCH₃ oder 2-(Methylthio)ethyl;
- C₁-C₄-Halogenalkylthio-C₁-C₄-alkyl für: durch C₁-C₄-Halogenalkylthio wie vorstehend genannt substituiertes C₁-C₄-Alkyl, also z.B. für 2-(Difluormethylthio)ethyl, 2-(Trifluormethylthio)ethyl oder 2-(Pentafluorethyithio)ethyl;
- C₁-C₄-Alkylsulfinyl-C₁-C₄-alkyl für: durch C₁-C₄-Alkylsulfinyl wie vorstehend genannt substituiertes C₁-C₄-Alkyl, also z.B. für CH₂SOCH₃, CH₂SOC₂H₅, n-Propylsulfinylmethyl, (1-Methylethylsulfinyl)methyl, n-Butylsulfinylmethyl, (1-Methylpropylsulfinyl)methyl, (2-Methylpropylsulfinyl)methyl, (1,1-Dimethylethylsulfinyl)methyl, 2-Methylsulfinylethyl, 2-Ethylsulfinylethyl, 2-(n-Propylsulfinyl)ethyl, 2-(1-Methylethylsulfinyl)ethyl, 2-(n-Butylsulfinyl)ethyl, 2-(1-Methylpropylsulfinyl)ethyl, 2-(2-Methylpropylsulfinyl)ethyl, 2-(1,1-Dimethylethylsulfinyl)ethyl, 2-(SOCH₃)propyl, 3-(SOCH₃)propyl, 2-(SOC₂H₅)propyl, 3-(SOC₂H₅)propyl, 3-(Propylsulfinyl)propyl, 3-(Butylsulfinyl)propyl, 4-(SOCH₃)butyl, 4-(SOC₂H₅)butyl, 4-(n-Propylsulfinyl)butyi oder 4-(n-Butylsulfinyl)butyl, insbesondere für 2-(SOCH₃)ethyl;
- C₁-C₄-Halogenalkylsulfinyl-C₁-C₄-alkyl für: durch C₁-C₄-Halogenalkylsulfinyl wie vorstehend genannt substituiertes C₁-C₄-Alkyl, also z.B. für 2-(2,2,2-Trifluorethylsulfinyl)ethyl;
- C₁-C₄-Alkylsulfonyl-C₁-C₄-alkyl für: durch C₁-C₄-Alkylsulfonyl wie vorstehend genannt substituiertes C₁-C₄-Alkyl, also z.B. für CH₂SO₂CH₃, CH₂SO₂C₂H₅, n-Propylsulfonylmethyl, (1-Methylethylsulfonyl)methyl, n-Butylsulfonylmethyl, (1-Methylpropylsulfonyl)methyl, (2-Methylpropylsulfonyl)methyl, (1,1-Dimethylethylsulfonyl)methyl, 2-Methylsulfonylethyl, 2-Ethylsulfonylethyl, 2-(n-Propylsulfonyl)ethyl, 2-(1-Methylethylsulfonyl)ethyl, 2-(n-Butylsulfonyl)ethyl, 2-(1-Methylpropylsulfonyl)ethyl, 2-(2-Methylpropylsulfonyl)ethyl, 2-(1,1-Dimethylethylsulfonyl)ethyl, 2-(SO₂CH₃)propyl, 3-(SO₂CH₃)propyl, 2-(SO₂C₂H₅)propyl, 3-(SO₂C₂H₅)propyl, 3-(Propylsulfonyl)-propyl, 3-(Butylsulfonyl)propyl, 4-(SO₂CH₃)butyl, 4-(SO₂C₂H₅)butyl, 4-(n-Propylsulfonyl)butyl oder 4-(n-Butylsulfonyl)butyl, insbesondere für 2-(SO₂CH₃)ethyl oder 2-(SO₂C₂H₅)ethyl;
- C₁-C₄-Halogenalkylsulfonyl-C₁-C₄-alkyl für: durch C₁-C₄-Halogenalkylsulfonyl wie vorstehend genannt substituiertes C₁-C₄-Alkyl, also z.B. für 2-(2,2,2-Trifluorethylsulfonyl)ethyl;
- (C₁-C₄-Alkyl)carbonyl für: COCH₃, COC₂H₅, n-Propylcarbonyl, COCH(CH₃)₂, n-Butylcarbonyl, 1-Methylpropylcarbonyl, 2-Methylpropylcarbonyl oder COC(CH₃)₃, insbesondere für COCH₃, COC₂H₅ oder COC(CH₃)₃;
- (C₁-C₄-Halogenalkyl)carbonyl: (C₁-C₄-Alkyl)carbonyl wie vorstehend genannt, das partiell oder vollständig durch Fluor, Chlor, Brom und/oder Jod substituiert ist, also z.B. COCH₂Cl, COCH(Cl)₂, COC(Cl)₃, COCH₂F, COCHF₂, COCF₃ COCHFCl, COCF(Cl)₂, COCF₂Cl, COCF₂Br, 1-Fluorethylcarbonyl, 2-Fluorethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 1,2-Dichlorethylcarbonyl, 2,2,2-Trichlorethylcarbonyl, COC₂F₅, 3-Chlorpropylcarbonyl, Heptafluorpropylcarbonyl, 1-(Fluormethyl)-2-fluorethylcarbonyl, l-(Chlormethyl)-2-chlorethylcarbonyl, 1-(Brommethyl)-2-bromethylcarbonyl, 4-Fluorbutylcarbonyl, 4-Chlorbutylcarbonyl, 4-Brombutylcarbonyl oder Nonafluorbutylcarbonyl, insbesondere für COCH₂Cl, COCH₂F, COCHF₂, COCF₃, 2-Fluorethylcarbonyl, 2-Chlorethylcarbonyl, 1,2-Dichlorethylcarbonyl, 2,2,2-Trifluorethylcarbonyl oder COC₂F₅;
- (C₁-C₄-Alkyl)carbonyl-C₁-C₄-alkyl für: durch (C₁-C₄-Alkyl)carbonyl wie vorstehend genannt substituiertes C₁-C₄-Alkyl, also z.B. für CH₂COCH₃, CH₂COC₂H₅ oder CH₂COC(CH₃)₃;
- (C₁-C₄-Halogenalkyl)carbonyl-C₁-C₄-alkyl für: durch (C₁-C₄-Halogenalkyl)carbonyl wie vorstehend genannt substituiertes C₁-C₄-Alkyl, also z.B. für CH₂COCF₃ oder CH₂COCH₂Cl;
- (C₃-C₈-Cycloalkyl)carbonyl-C₁-C₄-alkyl für: Cyclopropyl-CO-CH₂-, 1-(Cyclopropyl-CO-)ethyl, 2-(Cyclopropyl-CO-)ethyl, 1-(Cyclopropyl-CO-)prop-1-yl, 2-(Cyclopropyl-CO-)prop-1-yl, 3-(Cyclopropyl-CO-)prop-1-yl, 1-(Cyclopropyl-CO-)but-1-yl, 2-(Cyclopropyl-CO-)but-1-yl, 3-(Cyclopropyl-CO-)but-1-yl, 4-(Cyclopropyl-CO-)but-1-yl, 1-(Cyclopropyl-CO-)but-2-yl, 2-(Cyclopropyl-CO-)but-2-yl, 3-(Cyclopropyl-CO-)but-2-yl, 3-(Cyclopropyl-CO-)but-2-yl, 4-(Cyclopropyl-CO-)but-2-yl, 1-(Cyclopropyl-CO-CH₂-)eth-1-yl, 1-(Cyclopropyl-CO-CH₂-)-1-(CH₃)-eth-1-yl, 1-(Cyclopropyl-CO-CH₂-)prop-1-yl, Cyclobutyl-CO-CH₂-, 1-(Cyclobutyl-CO-)ethyl, 2-(Cyclobutyl-CO-)ethyl, 1-(Cyclobutyl-CO-)prop-1-yl, 2-(Cyclobutyl-CO-)prop-1-yl, 3-(Cyclobutyl-CO-)prop-1-yl, 1-(Cyclobutyl-CO-)but-1-yl, 2-(Cyclobutyl-CO-)but-1-yl, 3-(Cyclobutyl-CO-)but-1-yl, 4-(Cyclobutyl-CO-)but-1-yl, 1-(Cyclobutyl-CO-)but-2-yl, 2-(Cyclobutyl-CO-)but-2-yl, 3-(Cyclobutyl-CO-)but-2-yl, 3-(Cyclobutyl-CO-)but-2-yl, 4-(Cyclobutyl-CO-)but-2-yl, 1-(Cyclobutyl-CO-CH₂-)eth-1-yl, 1-(Cyclobutyl-CO-CH₂-)-1-(CH₃)-eth-1-yl, 1-(Cyclobutyl-CO-CH₂-)prop-1-yl, Cyclopentyl-CO-CH₂-, 1-(Cyclopentyl-CO-)ethyl, 2-(Cyclopentyl-CO-)ethyl, 1-(Cyclopentyl-CO-)prop-1-yl, 2-(Cyclopentyl-CO-)prop-1-yl, 3-(Cyclopentyl-CO-)prop-1-yl, 1-(Cyclopentyl-CO-)but-1-yl, 2-(Cyclopentyl-CO-)but-1-yl, 3-(Cyclopentyl-CO-)but-1-yl, 4-(Cyclopentyl-CO-)but-1-yl, 1-(Cyclopentyl-CO-)but-2-yl, 2-(Cyclopentyl-CO-)but-2-yl, 3-(Cyclopentyl-CO-)but-2-yl, 3-(Cyclopentyl-CO-)but-2-yl, 4-(Cyclopentyl-CO-)but-2-yl, 1-(Cyclopentyl-CO-CH₂-)eth-1-yl, 1-(Cyclopentyl-CO-CH₂-)-1-(CH₃)-eth-1-yl, 1- (Cyclopentyl-CO-CH₂-)prop-1-yl, Cyclohexyl-CO-CH₂-, 1-(Cyclohexyl-CO-)ethyl, 2-(Cyclohexyl-CO-)ethyl, 1-(Cyclohexyl-CO-)prop-1-yl, 2-(Cyclohexyl-CO-)prop-1-yl, 3-(Cyclohexyl-CO-)prop-1-yl, 1-(Cyclohexyl-CO-)but-1-yl, 2-(Cyclohexyl-CO-)but-1-yl, 3-(Cyclohexyl-CO-)but-1-yl, 4-(Cyclohexyl-CO-)but-1-yl, 1-(Cyclohexyl-CO-)but-2-yl, 2-(Cyclohexyl-CO-)but-2-yl, 3-(Cyclohexyl-CO-)but-2-yl, 3-(Cyclohexyl-CO-)but-2-yl, 4-(Cyclohexyl-CO-)but-2-yl, 1-(Cyclohexyl-CO-CH₂-)eth-1-yl, 1-(Cyclohexyl-CO-CH₂-)-1-(CH₃)-eth-1-yl, 1-(cyclohexyl-CO-CH₂-)prop-1-yl, Cycloheptyl-CO-CH₂-, 1-(Cycloheptyl-CO-)ethyl, 2-(Cycloheptyl-CO-)ethyl, 1-(Cycloheptyl-CO-)prop-1-yl, 2-(Cycloheptyl-CO-)prop-1-yl, 3-(Cycloheptyl-CO-)prop-1-yl, 1-(Cycloheptyl-CO-)but-1-yl, 2-(Cycloheptyl-CO-)but-1-yl, 3-(Cycloheptyl-CO-)but-1-yl, 4-(Cycloheptyl-CO-)but-1-yl, 1-(Cycloheptyl-CO-)but-2-yl, 2-(Cycloheptyl-CO-)but-2-yl, 3-(Cycloheptyl-CO-)but-2-yl, 3-(Cycloheptyl-CO-)but-2-yl, 4-(Cycloheptyl-CO-)but-2-yl, 1-(Cycloheptyl-CO-CH₂-)eth-1-yl, 1-(Cycloheptyl-CO-CH₂-)-1-(CH₃)-eth-1-yl, 1-(Cycloheptyl-CO-CH₂-)prop-1-yl, Cyclooctyl-CO-CH₂-, 1-(Cyclooctyl-CO-)ethyl, 2-(Cyclooctyl-CO-)ethyl, 1-(Cyclooctyl-CO-)prop-1-yl, 2-(Cyclooctyl-CO-)prop-1-yl, 3-(Cyclooctyl-CO-)prop-1-yl, 1-(Cyclooctyl-CO-)but-1-yl, 2-(Cyclooctyl-CO-)but-1-yl, 3-(Cyclooctyl-CO-)but-1-yl, 4-(Cyclooctyl-CO-)but-1-yl, 1-(Cyclooctyl-CO-)but-2-yl, 2-(Cyclooctyl-CO-)but-2-yl, 3-(Cyclooctyl-CO-)but-2-yl, 3-(Cyclooctyl-CO-)but-2-yl, 4-(Cyclooctyl-CO-)but-2-yl, 1-(Cyclooctyl-CO-CH₂-)-eth-1-yl, 1-(Cyclooctyl-CO-CH₂-)-1-(CH₃)-eth-1-yl oder 1-(Cyclooctyl-CO-CH₂-)prop-1-yl, insbesondere für (C₃-C₆-Cycloalkyl)carbonylmethyl;
- C₁-C₄-Alkylcarbonyloxy für: O-COCH₃, O-COC₂H₅, n-Propylcarbonyloxy, O-COCH(CH₃)₂, n-Butylcarbonyloxy, 1-Methylpropylcarbonyloxy, 2-Methylpropylcarbonyloxy oder O-COC(CH₃)₃, insbesondere für O-COCH₃, O-COC₂H₅ oder O-COC(CH₃)₃;
- (C₁-C₄-Halogenalkyl)carbonyloxy für: (C₁-C₄-Alkyl)carbonyloxy wie vorstehend genannt, das partiell oder vollständig durch Fluor, Chlor, Brom und/oder Jod substituiert ist, also z.B. für O-COCH₂Cl, O-COCH(Cl)₂, O-COC(Cl)₃, O-COCH₂F, O-COCHF₂, O-COCF₃, O-COCHFCl, O-COCF(Cl)₂, O-COCF₂Cl, O-COCF₂Br, 1-Fluorethylcarbonyloxy, 2-Fluorethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 1,2-Dichlorethylcarbonyloxy, 2,2,2-Trichlorethylcarbonyloxy, O-COC₂F₅, 3-Chlorpropylcarbonyloxy, Heptafluorpropylcarbonyloxy, 1-(Fluormethyl)-2-fluorethylcarbonyloxy, 1-(Chlormethyl) -2-chlorethylcarbonyloxy, 1-(Brommethyl)-2-bromethylcarbonyloxy, 4-Fluorbutylcarbonyloxy, 4-Chlorbutylcarbonyloxy, 4-Brombutylcarbonyloxy oder Nonafluorbutylcarbonyloxy, insbesondere für O-COCH₂Cl, O-COCH₂F, O-COCHF₂, O-COCF₃, 2-Fluorethylcarbonyloxy, 2-Chlorethylcarbonyloxy, 1,2-Dichlorethylcarbonyloxy, 2,2,2-Trifluorethylcarbonyloxy oder O-COC₂F₅;
- (C₁-C₄-Alkoxy)carbonyl für: COOCH₃, COOC₂H₅, n-Propoxycarbonyl, COOCH(CH₃)₂, n-Butoxycarbonyl, 1-(Methylpropoxy)carbonyl, 2-(Methylpropoxy)carbonyl oder COOC(CH₃)₃, insbesondere für COOCH₃, COOC₂H₅ oder COOC(CH₃)₃;
- (C₁-C₄-Alkoxy)carbonyl-C₁-C₄-alkyl für: durch (C₁-C₄-Alkoxy)carbonyl wie vorstehend genannt substituiertes C₁-C₄-Alkyl, also z.B. für CH₂COOCH₃, CH₂COOC₂H₅, CH₂CH₂COOCH₃, CH₂CH₂COOC₂H₅, CH(CH₃)COOCH₃ oder CH(CH₃)COOC₂H₅;
- (C₁-C₄-Halogenalkoxy)carbonyl-C₁-C₄-alkyl für: (C₁-C₄-Alkoxy)carbonyl-C₁-C₄-alkyl wie vorstehend genannt, dessen AlkoxyTeil partiell oder vollständig durch Fluor, Chlor, Brom und/oder Jod substituiert ist, also z.B. CH₂COOCH₂CF₃ oder CH(CH₃)COOCH₂CF₃;
- (C₁-C₄-Alkylthio)carbonyl-C₁-C₄-alkyl für: durch (C₁-C₄-Alkylthio)carbonyl wie COSCH₃, COSC₂H₅, n-Propylthiocarbonyl, 1-Methylethylthiocarbonyl, n-Butylthiocarbonyl, 1-Methylpropylthiocarbonyl, 2-Methylpropylthiocarbonyl oder 1,1-Dimethylethylthiocarbonyl, insbesondere COSCH₃ oder COSC₂H₅, substituiertes C₁-C₄-Alkyl, also z.B. für CH₂COSCH₃ oder CH₂COSC₂H₅;
- Di(C₁-C₄-alkyl)amino sowie der Dialkylamino-Teil von Di(C₁-C₄-alkyl)-aminocarbonyl für: z.B. N(CH₃)₂, N(C₂H₅)₂, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino, insbesondere für N(CH₃)₂ oder N(C₂H₅)₂;
- Di(C₁-C₄-alkyl)amino-C₁-C₄-alkyl für: durch Di(C₁-C₄-alkyl)amino wie vorstehend genannt substituiertes C₁-C₄-Alkyl, also z.B. für CH₂N(CH₃)₂, CH₂N(C₂H₅)₂, N,N-Dipropylaminomethyl, N,N-Di(1-methylethyl)aminomethyl, N,N-Dibutylaminomethyl, N,N-Di(1-methylpropyl)aminomethyl, N,N-Di(2-methylpropyl)aminomethyl, N,N-Di(1,1-dimethylethyl)aminomethyl, N-Ethyl-N-methylaminomethyl, N-Methyl-N-propylaminomethyl, N-Methyl-N-(1-methylethyl)aminomethyl, N-Butyl-N-methylaminomethyl, N-Methyl-N-(1-methylpropyl)aminomethyl, N-Methyl-N-(2-methylpropyl)aminomethyl, N-(1,1-Dimethylethyl)-N-methylaminomethyl, N-Ethyl-N-propylaminomethyl, N-Ethyl-N-(1-methylethyl)aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-(1,1-dimethylethyl)aminomethyl, N-(1-Methylethyl)-N-propylaminomethyl, N-Butyl-N-propylaminomethyl, N-(1-Methylpropyl)-N-propylaminomethyl, N-(2-Methylpropyl)-N-propylaminomethyl, N-(1,1-Dimethylethyl)-N-propylaminomethyl, N-Butyl-N-(1-methylethyl)aminomethyl, N-(1-Methylethyl)-N-(1-methylpropyl)aminomethyl, N-(1-Methylethyl)-N-(2-methylpropyl)aminomethyl, N-(1,1-Dimethylethyl)-N-(1-methylethyl)aminomethyl, N-Butyl-N-(1-methylpropyl)aminomethyl, N-Butyl-N-(2-methylpropyl)aminomethyl, N-Butyl-N-(1,1-dimethylethyl)aminomethyl, N-(1-Methylpropyl)-N-(2-methylpropyl)aminomethyl, N-(1,1-Dimethylethyl)-N-(1-methylpropyl)aminomethyl, N-(1,1-Dimethylethyl)-N-(2-methylpropyl)aminomethyl, N,N-Dimethylaminoethyl, N,N-Diethylaminoethyl, N,N-Di(n-propyl)aminoethyl, N,N-Di-(1-methylethyl)aminoethyl, N,N-Dibutylaminoethyl, N,N-Di(1-methylpropyl)aminoethyl, N,N-Di-(2-methylpropyl)aminoethyl, N,N-Di(1,1-dimethylethyl)aminoethyl, N-Ethyl-N-methylaminoethyl, N-Methyl-N-propylaminoethyl, N-Methyl-N- (1-methylethyl)aminoethyl, N-Butyl-N-methylaminoethyl, N-Methyl-N-(1-methylpropyl)aminoethyl, N-Methyl-N-(2-methylpropyl)aminoethyl, N-(1,1-Dimethylethyl)-N-methylaminoethyl, N-Ethyl-N-propylaminoethyl, N-Ethyl-N-(1-methyl-ethyl)aminoethyl, N-Butyl-N-ethylaminoethyl, N-Ethyl-N-(1-methylpropyl)aminoethyl, N-Ethyl-N-(2-methylpropyl)aminoethyl, N-Ethyl-N-(1,1-dimethylethyl)aminoethyl, N-(1-Methylethyl)-N-propylaminoethyl, N-Butyl-N-propylaminoethyl, N-(1-Methylpropyl)-N-propylaminoethyl, N-(2-Methylpropyl)-N-propylaminoethyl, N-(1,1-Dimethylethyl)-N-propylaminoethyl, N-Butyl-N-(1-methylethyl)-aminoethyl, N-(1-Methylethyl)-N-(1-methylpropyl)aminoethyl, N-(1-Methylethyl)-N-(2-methylpropyl)aminoethyl, N-(1,1-Dimethylethyl)-N-(1-methylethyl)aminoethyl, N-Butyl-N-(1-methylpropyl)aminoethyl, N-Butyl-N-(2-methylpropyl)aminoethyl, N-Butyl-N-(1,1-dimethylethyl)aminoethyl, N-(1-Methylpropyl)-N-(2-methyl-propyl)aminoethyl, N-(1,1-Dimethylethyl)-N-(1-methylpropyl)aminoethyl oder N-(1,1-Dimethylethyl)-N-(2-methylpropyl)aminoethyl, insbesondere für N,N-Dimethylaminoethyl oder N,N-Diethylaminoethyl;
- Di(C₁-C₄-alkyl)aminocarbonyl für: einen über eine Carbonyl-Brücke gebundenen Di(C₁-C₄-alkyl)amino-Rest, wie vorstehend genannt, also z.B. für CON(CH₃)₂, CON(C₂H₅)₂, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)-aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl;
- Di(C₁-C₄-alkyl)aminocarbonyl-C₁-C₄-alkyl für: durch Di(C₁-C₄-alkyl)aminocarbonyl wie vorstehend genannt substituiertes C₁-C₄-Alkyl,
   also z.B. für CH₂-CON(CH₃)₂, CH₂-CON(C₂H₅)₂, N,N-Dipropylamino-COCH₂-, N,N-Di-(1-methylethyl)amino-COCH₂-, N,N-Dibutylamino-COCH₂-, N,N-Di-(1-methylpropyl)amino-COCH₂-, N,N-Di-(2-methylpropyl)amino-COCH₂-, N,N-Di-(1,1-dimethylethyl)amino-COCH₂-, N-Ethyl-N-methylamino-COCH₂-, N-Methyl-N-propylamino-COCH₂-, N-Methyl-N-(1-methylethyl)amino-COCH₂-, N-Butyl-N-methylamino-COCH₂-, N-Methyl-N-(1-methylpropyl)amino-COCH₂-, N-Methyl-N-(2-methylpropyl)amino-COCH₂-, N-(1,1-Dimethylethyl)-N-methylamino-COCH₂-, N-Ethyl-N-propylamino-COCH₂-, N-Ethyl-N-(1-methylethyl)amino-COCH₂-, N-Butyl-N-ethylamino-COCH₂-, N-Ethyl-N-(1-methylpropyl)amino-COCH₂-, N-Ethyl-N-(2-methylpropyl)amino-COCH₂-, N-Ethyl-N-(1,1-dimethylethyl)amino-COCH₂-, N-(1-Methylethyl)-N-propylamino-COCH₂-, N-Butyl-N-propylamino-COCH₂-, N-(1-Methylpropyl)-N-propylamino-COCH₂-, N-(2-Methylpropyl)-N-propylamino-COCH₂-, N-(1,1-Dimethylethyl)-N-propylamino-COCH₂-, N-Butyl-N-(1-methylethyl)amino-COCH₂-, N-(1-Methylethyl)-N-(1-methylpropyl)amino-COCH₂-, N-(1-Methylethyl)-N-(2-methylpropyl)amino-COCH₂-, N-(1,1-Dimethylethyl)-N-(1-methylethyl)amino-COCH₂-, N-Butyl-N-(1-methylpropyl)amino-COCH₂-, N-Butyl-N-(2-methylpropyl)amino-COCH₂-, N-Butyl-N-(1,1-dimethylethyl)amino-COCH₂-, N-(1-Methylpropyl)-N-(2-methylpropyl)amino-COCH₂-, N-(1,1-Dimethylethyl)-N-(1-methylpropyl)amino-COCH₂-, N-(1,1-Dimethylethyl)-N-(2-methylpropyl)amino-COCH₂-, C₂H₄-CON(CH₃)₂, C₂H₄-CON(C₂H₅)₂, N,N-Di(n-propyl)amino-COC₂H₄-, N,N-Di-(1-methylethyl)amino-COC₂H₄-, N,N-Dibutylamino-COC₂H₄-, N,N-Di-(1-methylpropyl)amino-COC₂H₄-, N,N-Di(2-methylpropyl)amino-COC₂H₄-, N,N-Di(1,1-dimethylethyl)amino-COC₂H₄-, N-Ethyl-N-methylamino-COC₂H₄-, N-Methyl-N-propylamino-COC₂H₄-, N-Methyl-N-(1-methylethyl)amino-COC₂H₄-, N-Butyl-N-methylamino-COC₂H₄-, N-Methyl-N-(1-methylpropyl)amino-COC₂H₄-, N-Methyl-N-(2-methylpropyl)amino-COC₂H₄-, N-(1,1-Dimethylethyl)-N-methylamino-COC₂H₄-, N-Ethyl-N-propylamino-COC₂H₄-, N-Ethyl-N-(1-methylethyl)amino-COC₂H₄-, N-Butyl-N-ethylamino-COC₂H₄-, N-Ethyl-N-(1-methylpropyl)amino-COC₂H₄-, N-Ethyl-N-(2-methylpropyl)amino-COC₂H₄-, N-Ethyl-N-(1,1-dimethylethyl)amino-COC₂H₄-, N-(1-Methylethyl)-N-propylamino-COC₂H₄-, N-Butyl-N-propylamino-COC₂H₄-, N-(1-Methylpropyl)-N-propylamino-COC₂H₄-, N-(2-Methylpropyl)-N-propylamino-COC₂H₄-, N-(1,1-Dimethylethyl)-N-propylamino-COC₂H₄-, N-Butyl-N-(1-methylethyl)amino-COC₂H₄-, N-(1-Methylethyl)-N-(1-methylpropyl)amino-COC₂H₄-, N-(1-Methylethyl)-N-(2-methylpropyl)amino-COC₂H₄-, N-(1,1-Dimethylethyl)-N-(1-methylethyl)amino-COC₂H₄-, N-Butyl-N-(1-methylpropyl)amino-COC₂H₄-, N-Butyl-N-(2-methylpropyl)amino-COC₂H₄-, N-Butyl-N-(1,1-dimethylethyl)amino-COC₂H₄-, N-(1-Methylpropyl)-N-(2-methyl-propyl)amino-COC₂H₄-, N-(1,1-Dimethylethyl)-N-(1-methylpropyl)amino-COC₂H₄- oder N-(1,1-Dimethylethyl)-N-(2-methylpropyl)amino-COC₂H₅-, insbesondere für CH₂-CON(CH₃)₂, CH₂-CON(C₂H₅)₂, CH(CH₃)-CON(CH₃)₂ oder CH(CH₃)CON(C₂H₅)₂;
- C₁-C₄-Alkylamino-C₁-C₄-alkyl für: durch C₁-C₄-Alkylamino wie H₃C-NH-, H₅C₂-NH-, n-Propyl-NH-, 1-Methylethyl-NH-, n-Butyl-NH-, 1-Methylpropyl-NH-, (H₃C)₂CH-CH₂-NH- und 1,1-Dimethylethyl-NH-, vorzugsweise H₃C-NH- oder H₃C₂-NH-, substituiertes C₁-C₄-Alkyl, also beispielsweise für CH₂CH₂-NH-CH₃, CH₂CH₂-N(CH₃)₂, CH₂CH₂-NH-C₂H₅ oder CH₂CH₂-N(C₂H₅)₂;
- (C₁-C₄-Alkylamino)carbonyl für: H₃C-NH-CO-, H₅C₂-NH-CO-, n-Propyl-NH-CO-, 1-Methylethyl-NH-CO-, n-Butyl-NH-CO-, 1-Methylpropyl-NH-CO-, -CONH-CH₂-CH(CH₃)₂ oder -CONH-C(CH₃)₃, insbesondere für -CONH-CH₃ oder -CONH-C₂H₅;
- (C₁-C₄-Alkylamino)carbonyl-C₁-C₄-alkyl für: durch (C₁-C₄-Alkylamino)carbonyl wie vorstehend genannt substituiertes C₁-C₄-Alkyl, also beispielsweise für CH₂-CONH-CH₃, CH₂-CONH-C₂H₅, CH(CH₃)-CONH-CH₃ oder CH(CH₃)-CONH-C₂H₅;
- C₃-C₆-Alkenyl für: Prop-1-en-1-yl, Prop-2-en-1-yl, 1-Methylethenyl, n-Buten-1-yl, n-Buten-2-yl, n-Buten-3-yl, 1-Methylprop-1-en-1-yl, 2-Methylprop-1-en-1-yl, 1-Methylprop-2-en-1-yl, 2-Methylprop-2-en-1-yl, n-Penten-1-yl, n-Penten-2-yl, n-Penten-3-yl, n-Penten-4-yl, 1-Methylbut-1-en-1-yl, 2-Methylbut-1-en-1-yl, 3-Methylbut-1-en-1-yl, 1-Methylbut-2-en-1-yl, 2-Methylbut-2-en-1-yl, 3-Methylbut-2-en-1-yl, 1-Methylbut-3-en-1-yl, 2-Methylbut-3-en-1-yl, 3-Methylbut-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethylprop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methylpent-1-en-1-yl, 2-Methylpent-1-en-1-yl, 3-Methylpent-1-en-1-yl, 4-Methylpent-1-en-1-yl, 1-Methylpent-2-en-1-yl, 2-Methylpent-2-en-1-yl, 3-Methylpent-2-en-1-yl, 4-Methylpent-2-en-1-yl, 1-Methylpent-3-en-1-yl, 2-Methylpent-3-en-1-yl, 3-Methylpent-3-en-1-yl, 4-Methylpent-3-en-1-yl, 1-Methylpent-4-en-1-yl, 2-Methylpent-4-en-1-yl, 3-Methylpent-4-en-1-yl, 4-Methylpent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethylbut-1-en-1-yl, 1-Ethyl-but-2-en-1-yl, 1-Ethylbut-3-en-1-yl, 2-Ethylbut-1-en-1-yl, 2-Ethylbut-2-en-1-yl, 2-Ethylbut-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl, insbesondere für Allyl oder 2-Buten-1-yl;
- C₃-C₆-Halogenalkenyl für: C₃-C₆-Alkenyl wie vorstehend genannt, das partiell oder vollständig durch Fluor, Chlor, Brom und/oder Jod substituiert ist, also z.B. für 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl, insbesondere für 2-Chlorallyl, 3-Chlorallyl oder 3,3-Dichlorallyl;
- Cyano-C₃-C₆-alkenyl für: z.B. 2-Cyanoallyl, 3-Cyanoallyl, 4-Cyanobut-2-enyl, 4-Cyanobut-3-enyl oder 5-Cyanopent-4-enyl, insbesondere für 3-Cyanoallyl oder 4-Cyanobut-2-enyl;
- C₃-C₆-Alkinyl für: Prop-1-in-1-yl, Propargyl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl, insbesondere für Propargyl;
- C₃-C₆-Halogenalkinyl für: C₃-C₆-Alkinyl wie vorstehend genannt, das partiell oder vollständig durch Fluor, Chlor, Brom und/oder Jod substituiert ist, also z.B. für 1,1-Difluorprop-2-in-1-yl, 4-Fluorbut-2-in-1-yl, 4-Chlorbut-2-in-1-yl, 1,1-Difluorbut-2-in-1-yl, 5-Pluorpent-3-in-1-yl oder 6-Fluorhex-4-in-1-yl, insbesondere für 4-Fluorbut-2-in-1-yl;
- Cyano-C₃-C₆-alkinyl für: z.B. 3-Cyanopropargyl, 4-Cyanobut-2-in-1-yl, 5-Cyanopent-3-in-1-yl und 6-Cyanohex-4-in-1-yl;
- C₃-C₄-Alkenyloxy-C₁-C₄-alkyl für: durch C₃-C₄-Alkenyloxy wie Allyloxy, But-1-en-3-yloxy, But-1-en-4-yloxy, But-2-en-1-yloxy, 1-Methylprop-2-enyloxy oder 2-Methylprop-2-enyloxy substituiertes C₁-C₄-Alkyl, also beispielsweise für Allyloxymethyl, 2-Allyloxyethyl oder But-1-en-4-yloxymethyl, insbesondere für 2-Allyloxyethyl;
- C₃-C₄-Alkinyloxy-C₁-C₄-alkyl für: durch C₃-C₄-Alkinyloxy wie Propargyloxy, But-1-in-3-yloxy, But-1-in-4-yloxy, But-2-in-1-yloxy, 1-Methylprop-2-inyloxy oder 2-Methylprop-2-inyloxy, vorzugsweise Propargyloxy, substituiertes C₁-C₄-Alkyl, also beispielsweise für Propargyloxymethyl oder 2-Propargyloxyethyl, insbesondere für 2-Propargyloxyethyl;
- (C₃-C₄-Alkenyloxy)imino-C₁-C₄-alkyl für: durch (C₃-C₄-Alkenyloxy)imino wie Allyloxy, But-1-en-3-yloxyimino, But-1-en-4-yloxyimino, But-2-en-1-yloxyimino, 1-Methylprop-2-enyloxyimino oder 2-Methylprop-2-enyloxyimino substituiertes C₁-C₄-Alkyl, also beispielsweise für Allyloxyimino-methyl, 2-(Allyloxyimino)ethyl oder But-1-en-4-yloxyimino-methyl, insbesondere für 2-(Allyloxyimino)ethyl oder 2-(2-Buten-1-yloxyimino)ethyl;
- C₃-C₄-Alkenylthio-C₁-C₄-alkyl für: durch C₃-C₄-Alkenylthio wie Allylthio, But-1-en-3-ylthio, But-1-en-4-ylthio, But-2-en-1-ylthio, 1-Methylprop-2-enylthio oder 2-Methylprop-2-enylthio substituiertes C₁-C₄-Alkyl, also beispielsweise für Allylthiomethyl, 2-Allylthioethyl oder But-1-en-4-ylthiomethyl, insbesondere für 2-(Allylthio)ethyl;
- C₃-C₄-Alkinylthio-C₁-C₄-alkyl für: durch C₃-C₄-Alkinylthio wie Propargylthio, But-1-in-3-ylthio, But-1-in-4-ylthio, But-2-in-1-ylthio, 1-Methylprop-2-inylthio oder 2-Methylprop-2-inylthio, vorzugsweise Propargylthio, substituiertes C₁-C₄-Alkyl, also beispielsweise für Propargylthiomethyl oder 2-Propargylthioethyl, insbesondere für 2-(Propargylthio)ethyl;
- C₃-C₄-Alkenylsulfinyl-C₁-C₄-alkyl für: durch C₃-C₄-Alkenylsulfinyl wie Allylsulfinyl, But-1-en-3-ylsulfinyl, But-1-en-4-ylsulfinyl, But-2-en-1-ylsulfinyl, 1-Methylprop-2-enylsulfinyl oder 2-Methylprop-2-enylsulfinyl substituiertes C₁-C₄-Alkyl, also beispielsweise für Allylsulfinylmethyl, 2-Allylsulfinylethyl oder But-1-en-4-yl-sulfinylmethyl, insbesondere für 2-(Allylsulfinyl)ethyl;
- C₃-C₄-Alkinylsulfinyl-C₁-C₄-alkyl für: durch C₃-C₄-Alkinylsulfinyl wie Propargylsulfinyl, But-1-in-3-ylsulfinyl, But-1-in-4-ylsulfinyl, But-2-in-1-ylsulfinyl, 1-Methylprop-2-inylsulfinyl oder 2-Methylprop-2-inylsulfinyl, vorzugsweise Propargylsulfinyl, substituiertes C₁-C₄-Alkyl, also beispielsweise für Propargylsulfinylmethyl oder 2-Propargylsulfinylethyl, insbesondere für 2-(Propargylsulfinyl)ethyl;
- C₃-C₄-Alkenylsulfonyl-C₁-C₄-alkyl für: durch C₃-C₄-Alkenylsulfonyl wie Allylsulfonyl, But-1-en-3-ylsulfonyl, But-1-en-4-ylsulfonyl, But-2-en-1-ylsulfonyl, 1-Methylprop-2-enylsulfonyl oder 2-Methylprop-2-enylsulfonyl substituiertes C₁-C₄-Alkyl, also beispielsweise für Allylsulfonylmethyl, 2-Allylsulfonylethyl oder But-1-en-4-yl-sulfonylmethyl, insbesondere für 2-(Allylsulfonyl)ethyl;
- C₃-C₄-Alkinylsulfonyl-C₁-C₄-alkyl für: durch C₃-C₄-Alkinylsulfonyl wie Propargylsulfonyl, But-1-in-3-ylsulfonyl, But-1-in-4-ylsulfonyl, But-2-in-1-ylsulfonyl, 1-Methylprop-2-inylsulfonyl oder 2-Methylprop-2-inylsulfonyl, vorzugsweise Propargylsulfonyl, substituiertes C₁-C₄-Alkyl, also beispielsweise für Propargylsulfonylmethyl oder 2-Propargylsulfonylethyl, insbesondere für 2-(Propargylsulfonyl)ethyl.

Unter 3- bis 7gliedrigem Heterocyclyl sind sowohl gesättigte, partiell oder vollständig ungesättigte als auch aromatische Heterocyclen mit ein bis drei Heteroatomen, ausgewählt aus einer Gruppe bestehend aus
- ein bis drei Stickstoffatomen,
- einem oder zwei Sauerstoff- und
- einem oder zwei Schwefelatomen,
zu verstehen.

Beispiele für gesättigte Heterocyclen, die ein Carbonyl- oder Thiocarbonyl-Ringglied enthalten können, sind:
Oxiranyl, Thiiranyl, Aziridin-1-yl, Aziridin-2-yl, Diaziridin-1-yl, Diaziridin-3-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-2-yl, 1,3-Dithiepan-2-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl;

Beispiele für ungesättigte Heterocyclen, die ein Carbonyloder Thiocarbonyl-Ringglied enthalten können, sind: Dihydrofuran-2-yl, 1,2-Oxazolin-3-yl, 1,2-Oxazolin-5-yl, 1,3-Oxazolin-2-yl;

Unter den Heteroaromaten sind die 5- und 6-gliedrigen bevorzugt, also z.B. Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Alle Cycloalkyl-, Phenyl- und heterocyclischen Ringe sind vorzugsweise unsubstituiert oder tragen einen Substituenten.

Im Hinblick auf die Verwendung der substituierten 3-Phenylpyrazole I als Herbizide sind diejenigen Verbindungen I bevorzugt, bei denen die Variablen folgende Bedeutungen haben, und zwar jeweils für sich allein oder in Kombination:
- R¹: C₁-C₄-Alkyl, insbesondere Methyl;
- R²: C₁-C₄-Halogenalkyl, C₁-C₄-Halogenalkoxy oder C₁-C₄-Alkylsulfonyl, insbesondere Trifluormethyl, Difluormethoxy oder Methylsulfonyl; besonders bevorzugt ist Difluormethoxy;
- R³: Cyano oder Halogen, insbesondere Chlor, Brom oder Fluor; besonders bevorzugt ist Chlor;
- R⁴: Wasserstoff, Fluor oder Chlor, insbesondere Fluor oder Chlor;
- R⁵: Cyano, Halogen oder C₁-C₄-Halogenalkyl, insbesondere Chlor, Brom oder Trifluormethyl; besonders bevorzugt ist Chlor;
- R⁶: C₁-C₆-Alkyl, C₁-C₆-Halogenalkyl, Cyano-C₁-C₄-alkyl, C₁-C₄-Alkoxy-C₁-C₄-alkyl, C₁-C₄-Alkylthio-C₁-C₄-alkyl, C₁-C₄-Alkylsulfonyl-C₁-C₄-alkyl, C₃-C₆-Alkenyl, C₃-C₆-Halogenalkenyl, C₃-C₆-Alkinyl, (C₁-C₄-Alkyl)carbonyl, (C₁-C₄-Halogenalkyl)carbonyl, (C₁-C₄-Alkoxy)carbonyl, Di(C₁-C₄-alkyl)aminocarbonyl, (C₁-C₄-Alkyl)carbonyl-C₁-C₄-alkyl, (C₃-C₈-Cycloalkyl)carbonyl-C₁-C₄-alkyl, (C₁-C₄-Alkoxy)imino-C₁-C₄-alkyl, Hydroxycarbonyl-C₁-C₄-alkyl, (C₁-C₄-Alkoxy)carbonyl-C₁-C₄-alkyl, das eine (C₁-C₄-Alkoxy)imino-Gruppe tragen kann, Di(C₁-C₄-alkyl)aminocarbonyl-C₁-C₄-alkyl, C₃-C₈-Cycloalkyl, C₃-C₈-Cycloalkyl-C₁-C₄-alkyl oder Phenyl-C₁-C₄-alkyl, insbesondere C₁-C₆-Alkyl, C₁-C₄-Alkoxy-C₁-C₄-alkyl, C₃-C₆-Alkenyl, C₃-C₆-Alkinyl, (C₁-C₄-Alkyl)carbonyl, (C₁-C₄-Alkoxy)carbonyl, Di(C₁-C₄-alkyl)aminocarbonyl, (C₁-C₄-Alkyl)carbonyl-C₁-C₄-alkyl, (C₃-C₈-Cycloalkyl)carbonyl-C₁-C₄-alkyl, (C₁-C₄-Alkoxy)imino-C₁-C₄-alkyl, Hydroxycarbonyl-C₁-C₄-alkyl, (C₁-C₄-Alkoxy)carbonyl-C₁-C₄-alkyl, das eine (C₁-C₄-Alkoxy)imino-Gruppe tragen kann, Di(C₁-C₄-alkyl)aminocarbonyl-C₁-C₄-alkyl oder Phenyl-C₁-C₄-alkyl;
besonders bevorzugt sind C₁-C₆-Alkyl, C₃-C₆-Alkenyl, C₃-C₆-Alkinyl, (C₁-C₄-Alkyl)carbonyl, (C₁-C₄-Alkoxy)carbonyl, Di(C₁-C₄-alkyl)aminocarbonyl, (C₁-C₄-Alkoxy)carbonyl-C₁-C₄-alkyl oder Di(C₁-C₄-alkyl)aminocarbonyl-C₁-C₄-alkyl;
- R⁷: C₁-C₆-Alkyl, C₃-C₆-Alkenyl, C₃-C₆-Alkinyl, (C₁-C₄-Alkoxy)carbonyl-C₁-C₄-alkyl, Di(C₁-C₄-alkyl)aminocarbonyl-C₁-C₄-alkyl, C₃-C₈-Cycloalkyl, C₃-C₈-Cycloalkyl-C₁-C₄-alkyl, Phenyl oder Phenyl-C₁-C₄-alkyl, insbesondere C₁-C₆-Alkyl, C₃-C₆-Alkenyl, C₃-C₆-Alkinyl, (C₁-C₄-Alkoxy)carbonyl-C₁-C₄-alkyl oder Phenyl-C₁-C₄-alkyl;
- X: eine chemische Bindung, eine 1,2-Ethindiyl-Gruppe oder eine über die mit Stern gekennzeichnete Seite an den Phenylring gebundene Gruppe *-CH=C(R⁸)-, wobei R⁸ für Wasserstoff, Cyano, Chlor, Brom, oder Methyl, insbesondere Chlor, steht; besonders bevorzugt steht X für eine chemische Bindung;
- Y: Sauerstoff.

Ganz besonders bevorzugt sind die in der folgenden Tabelle 1 aufgeführten Verbindungen Ia (≙ I mit R¹ = Methyl, R² = Difluormethoxy, R³ und R⁵ = Chlor, X = chemische Bindung und Y = Sauerstoff):

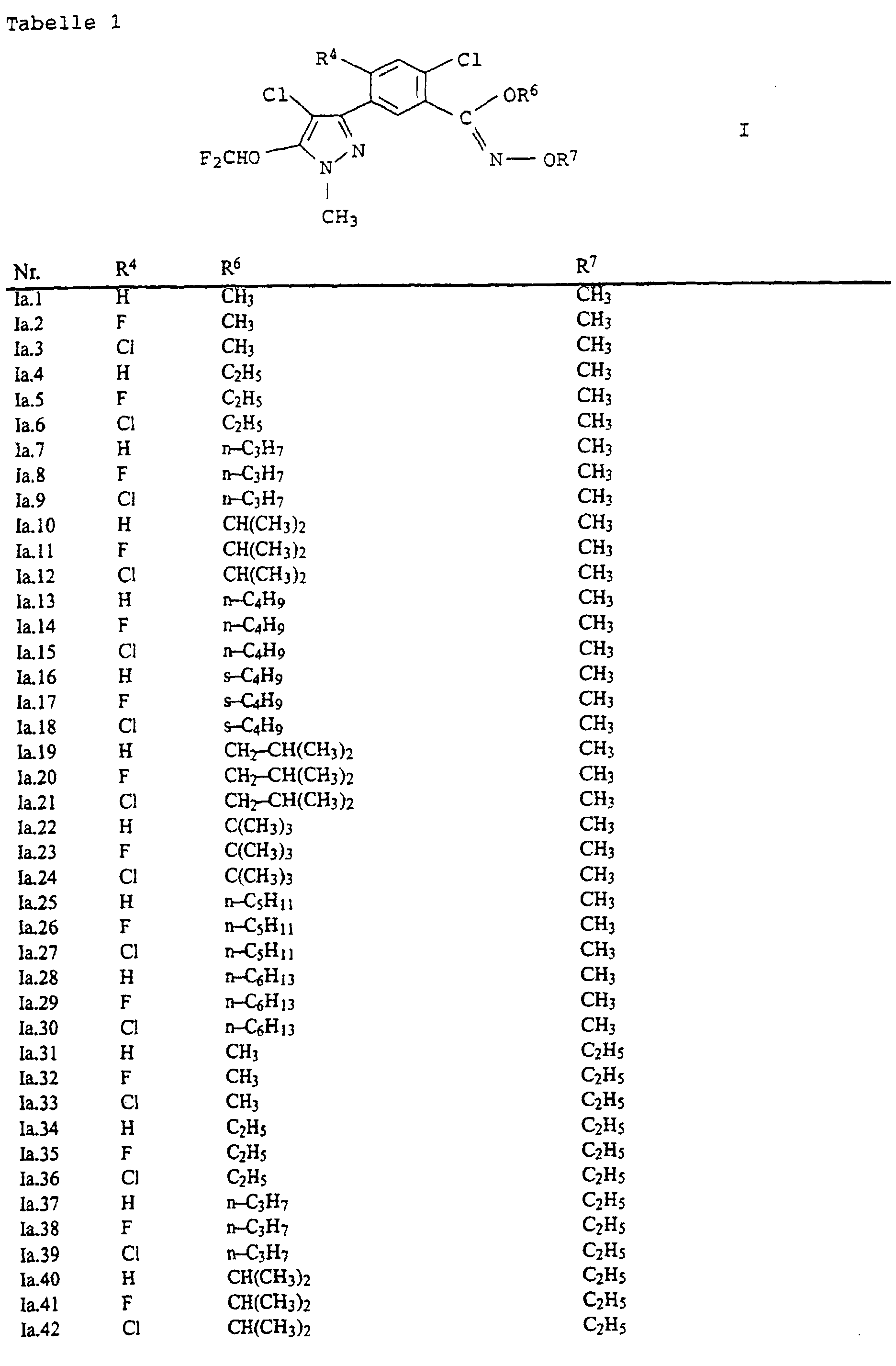

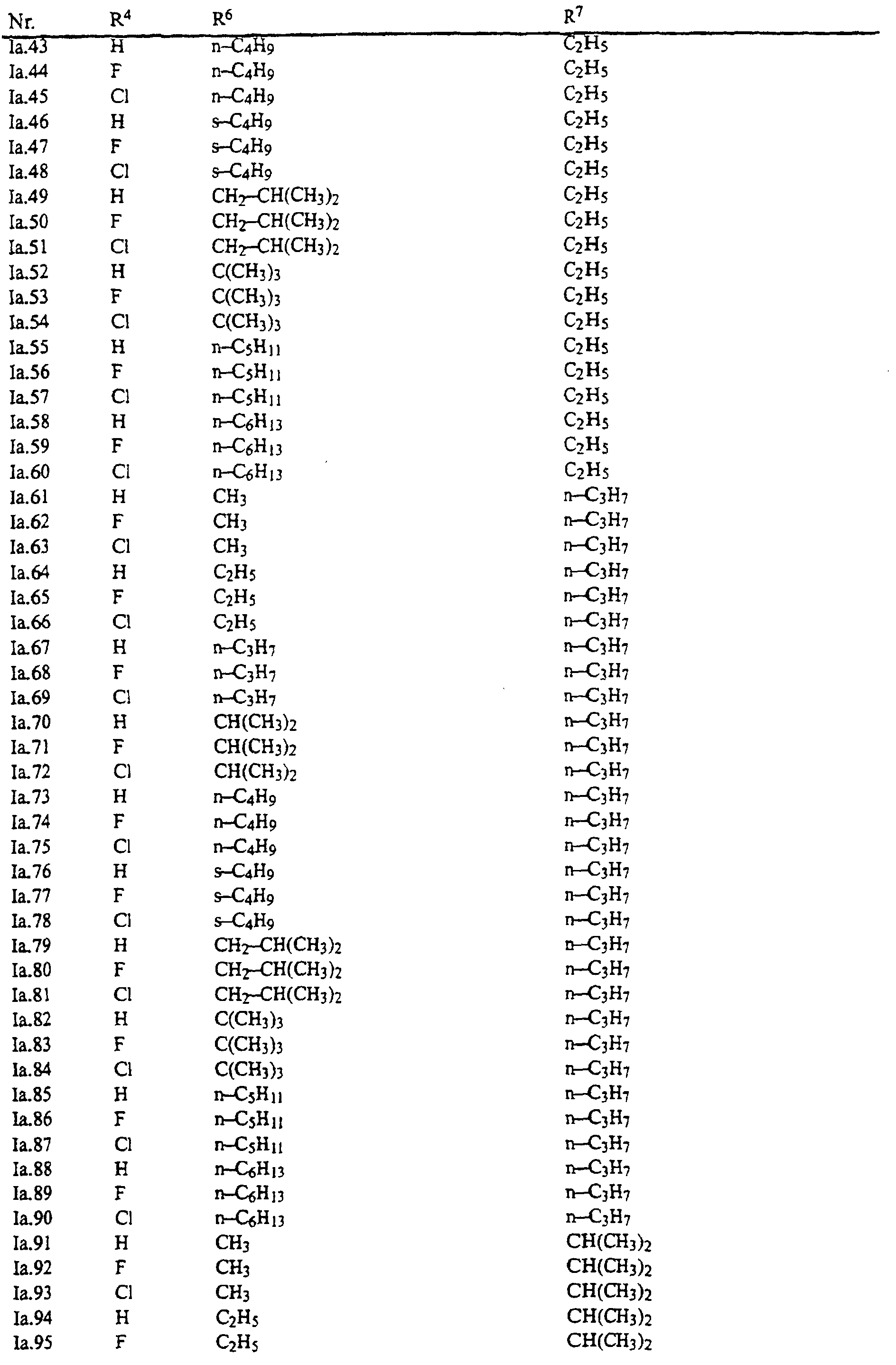

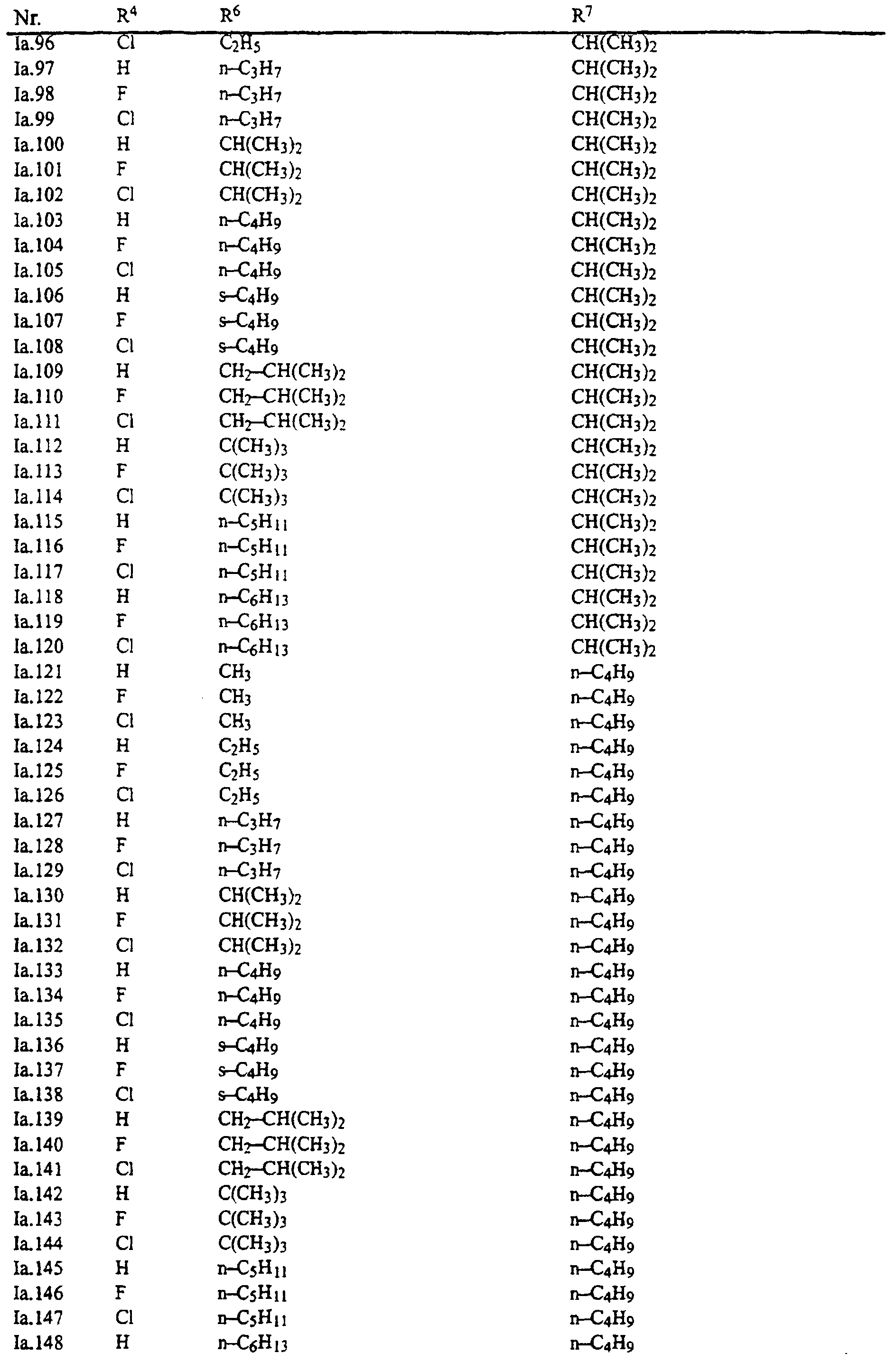

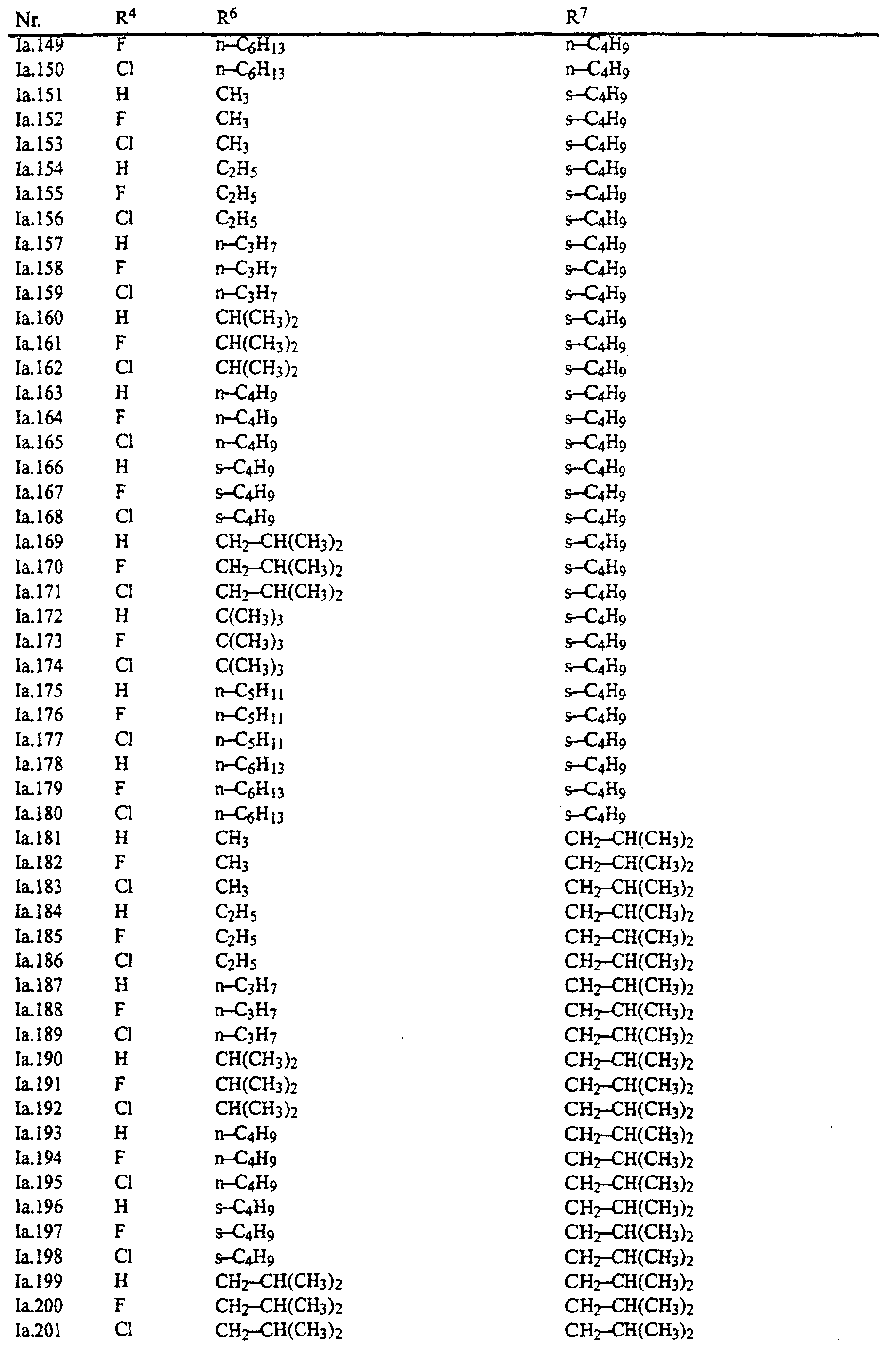

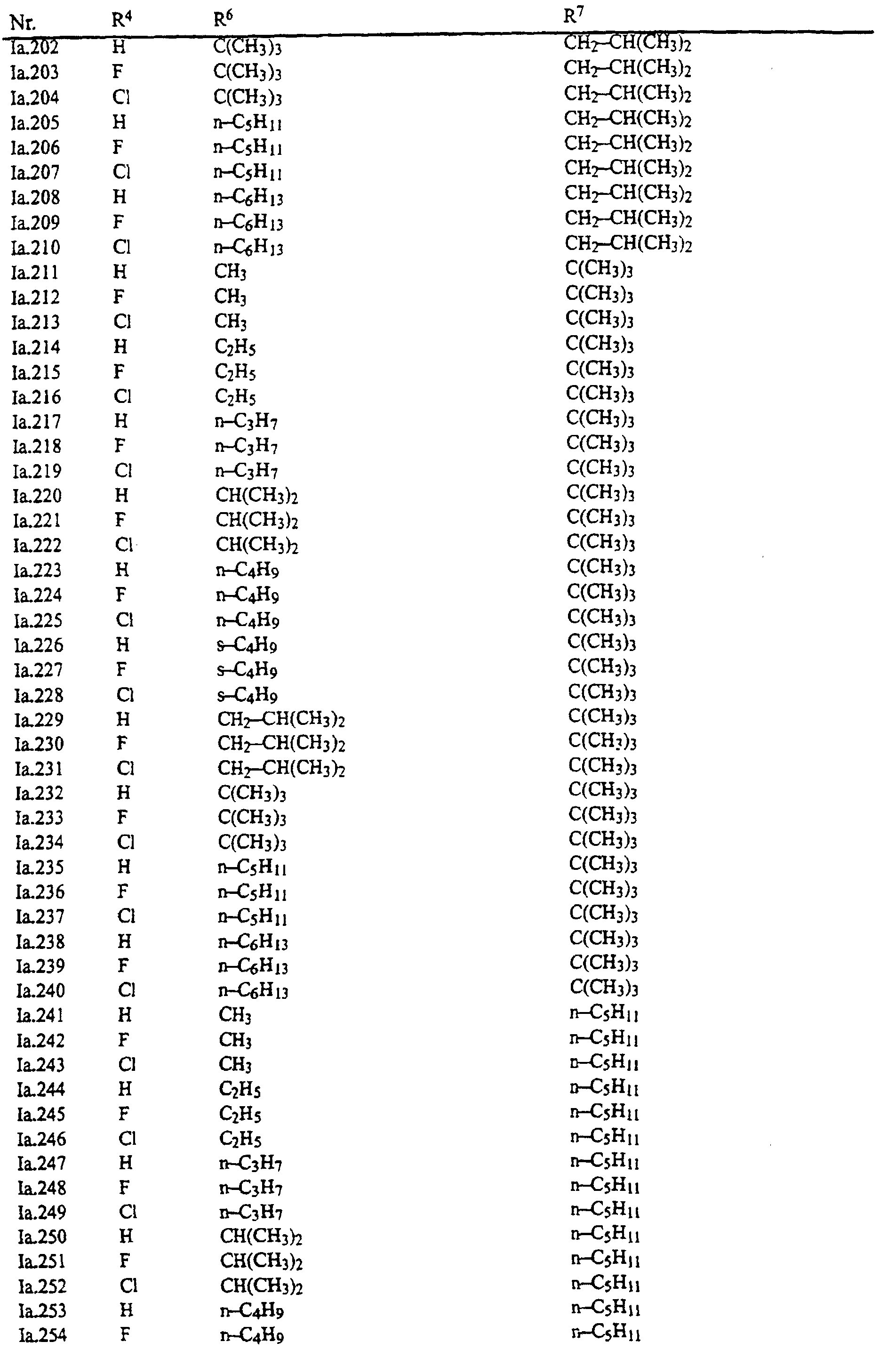

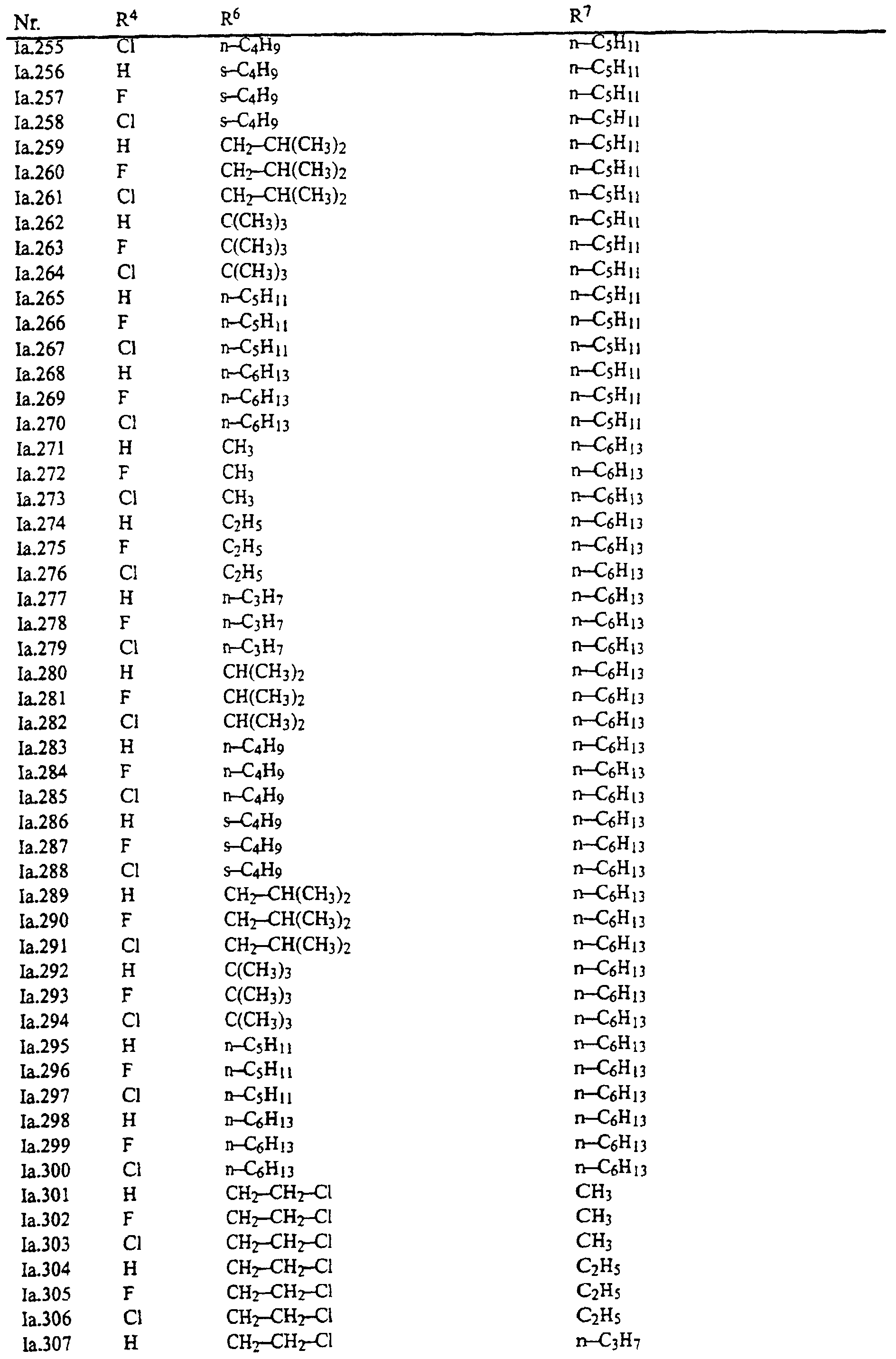

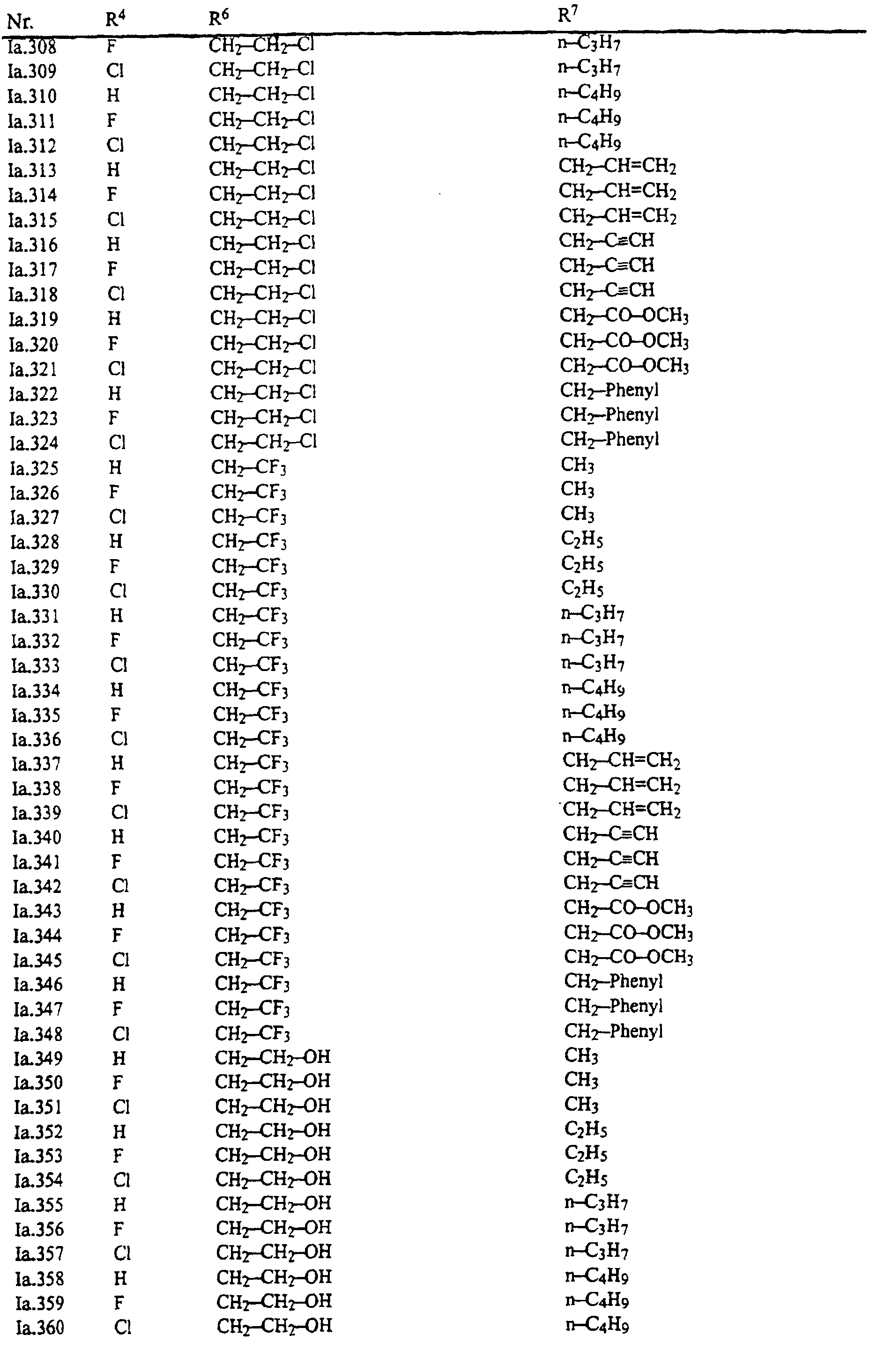

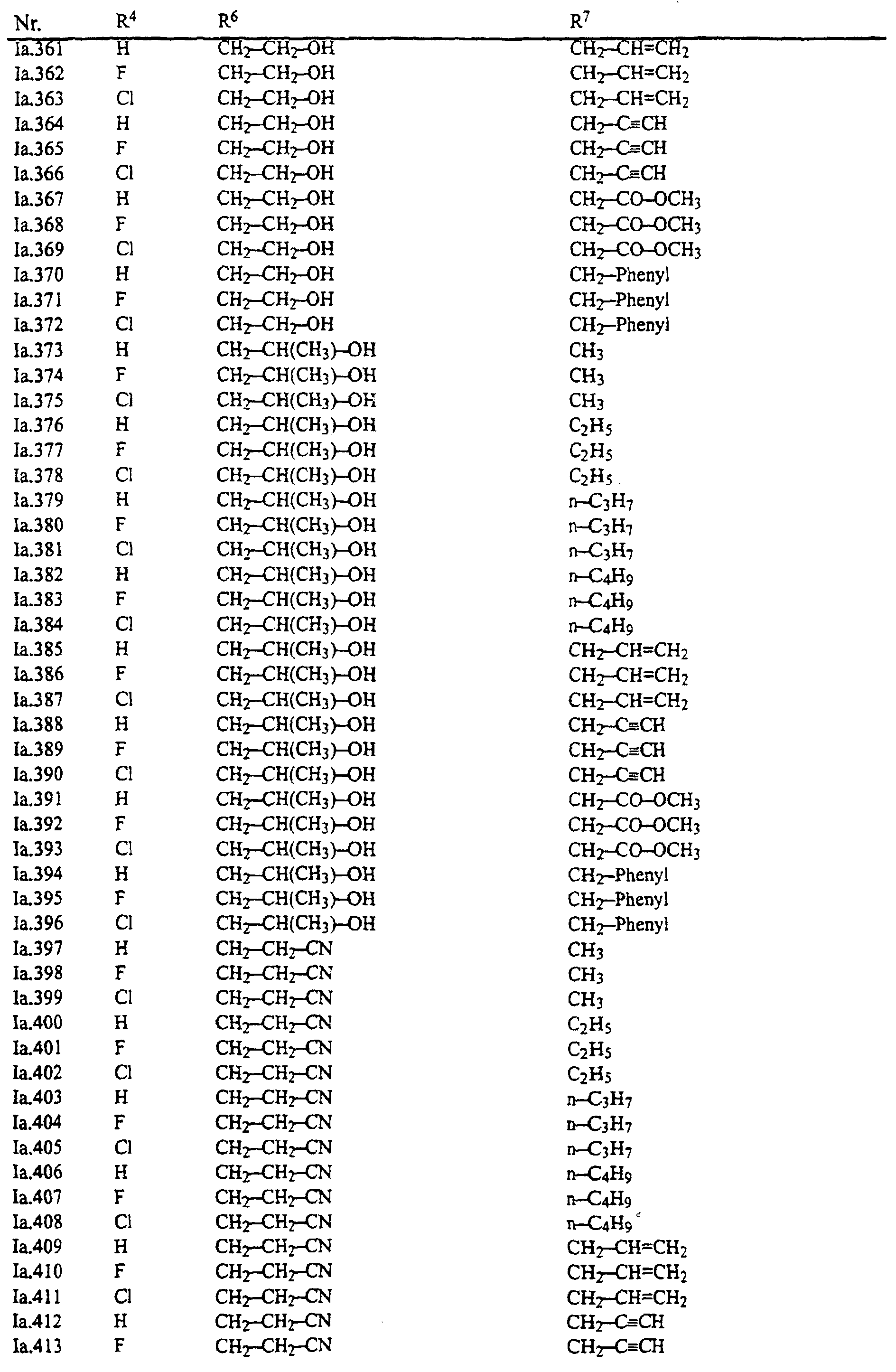

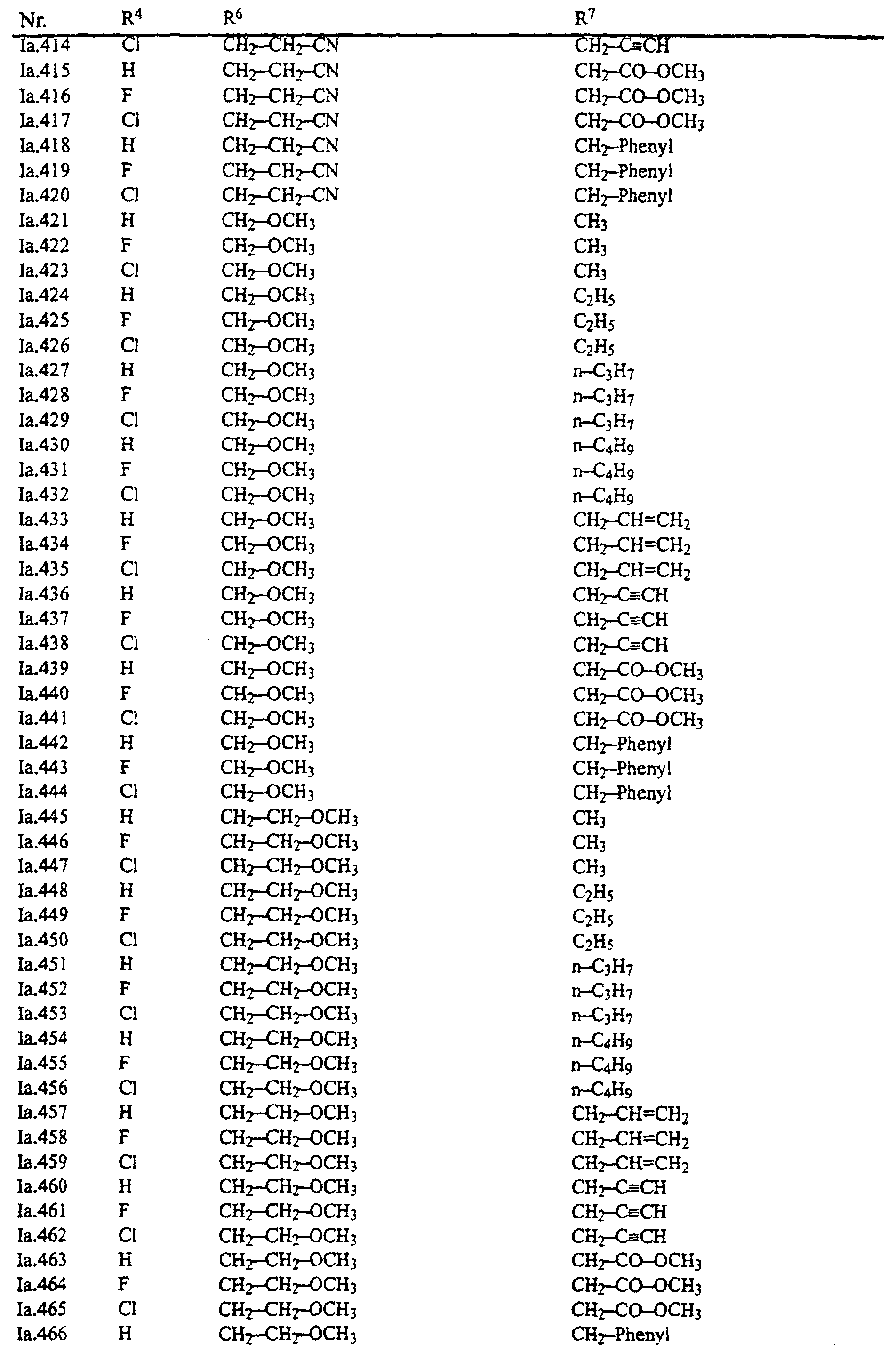

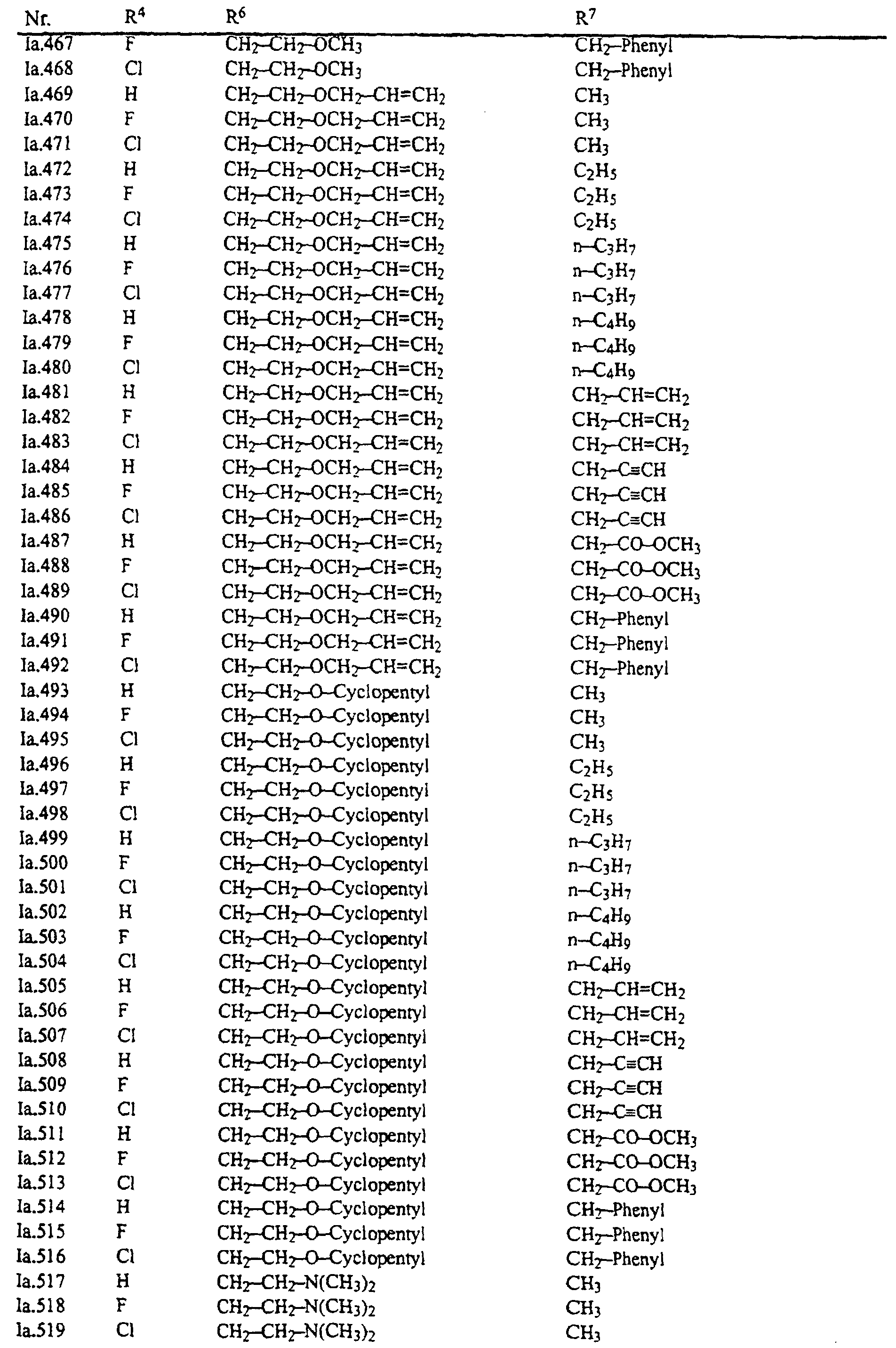

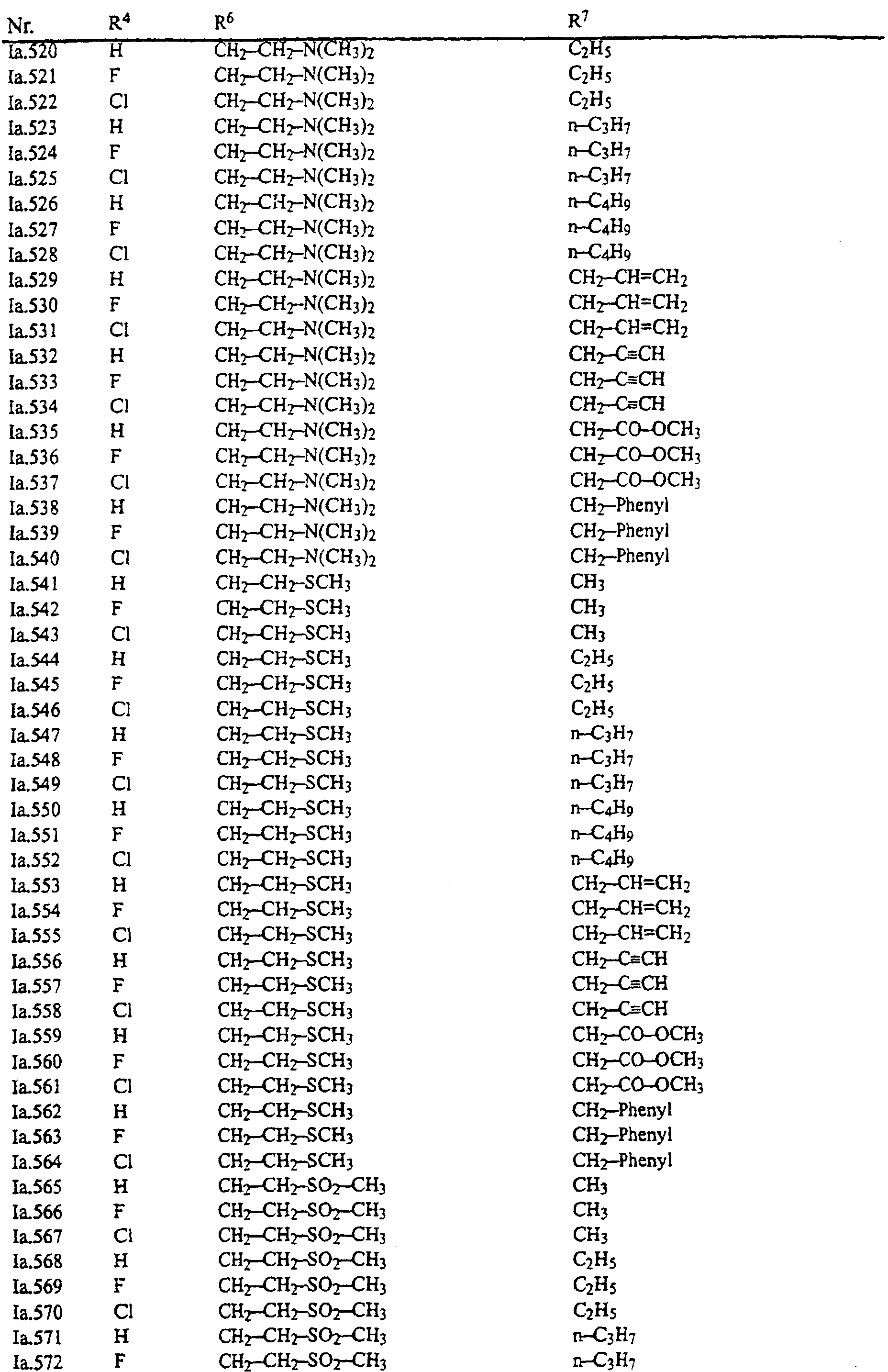

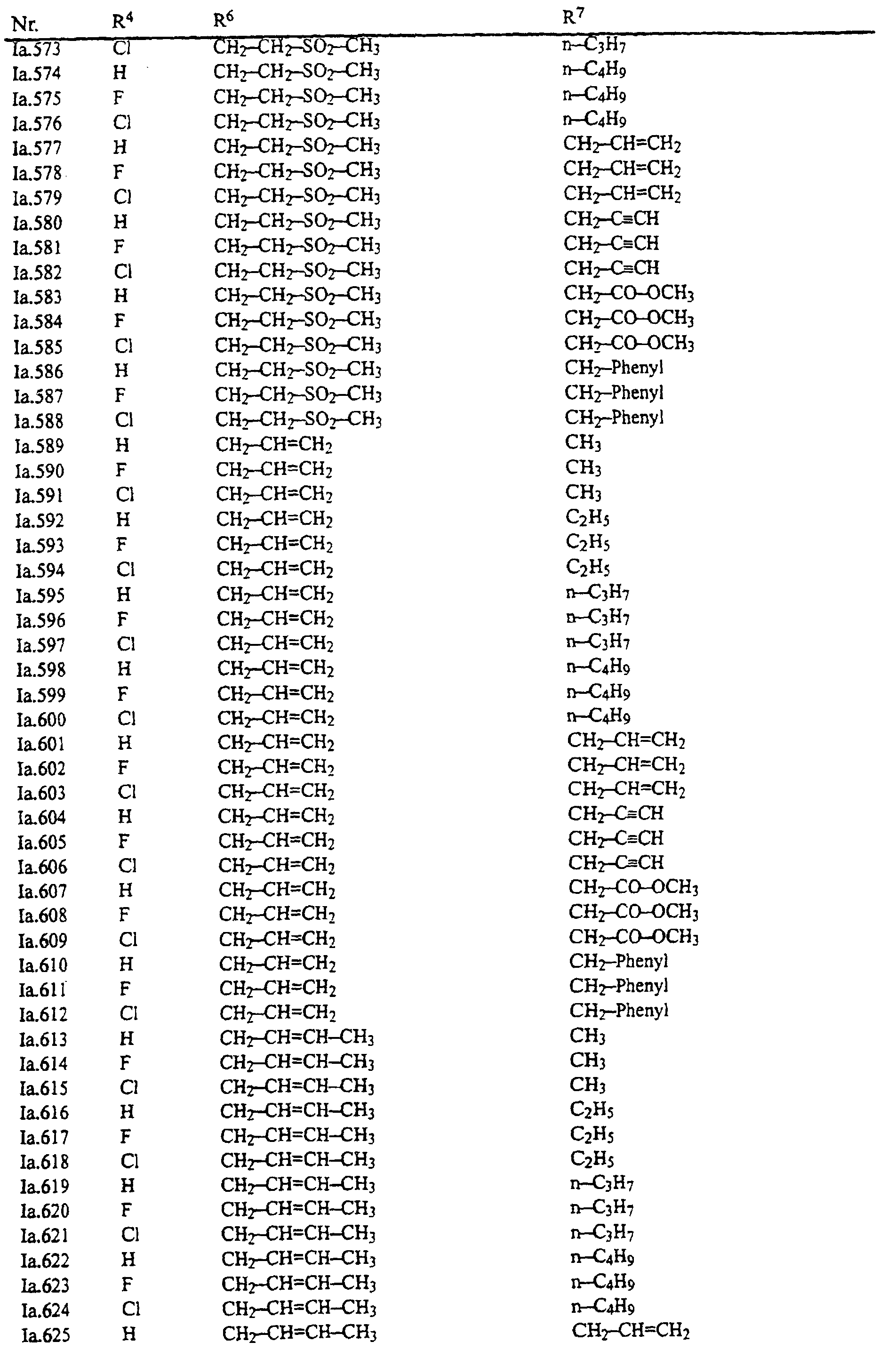

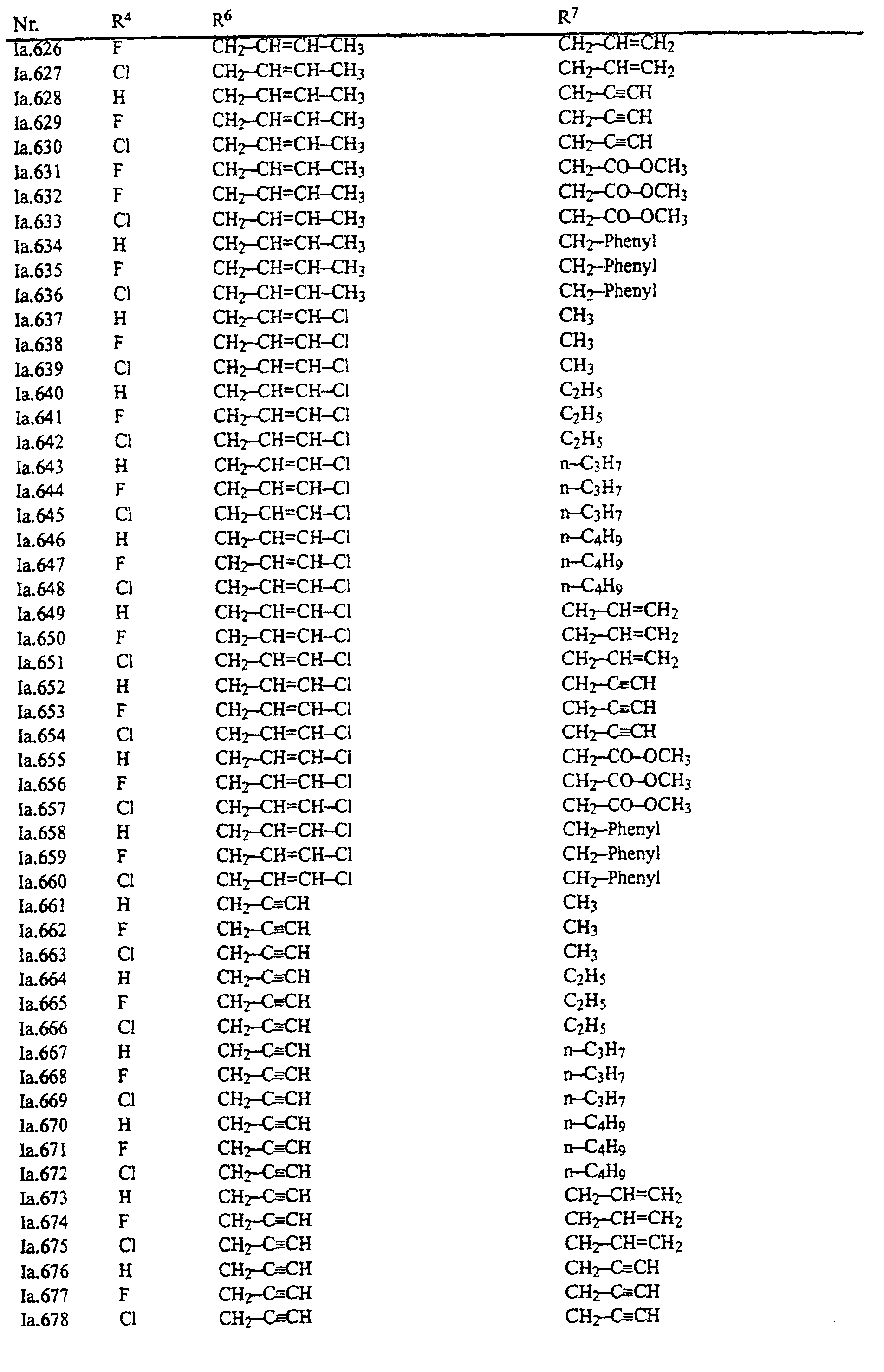

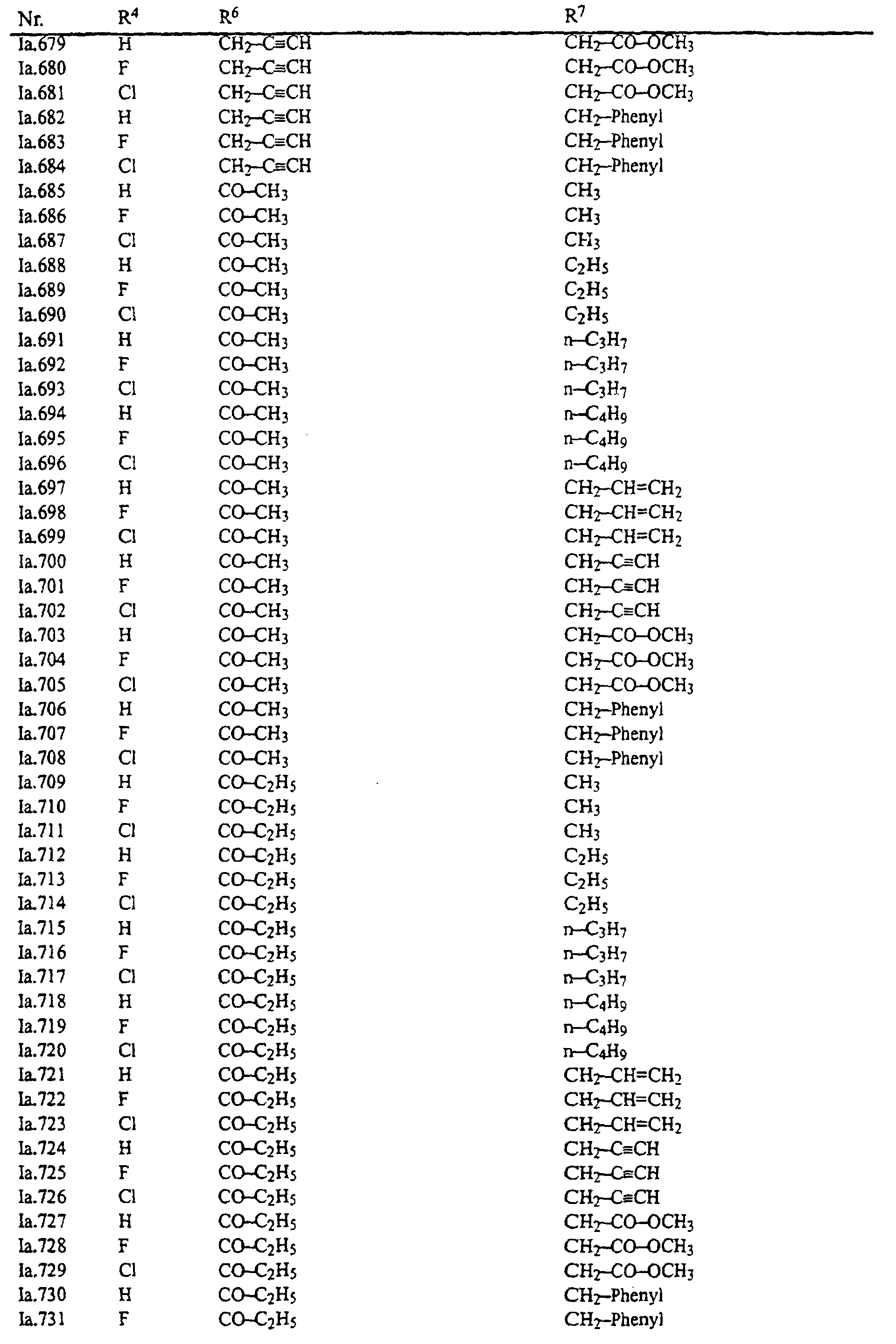

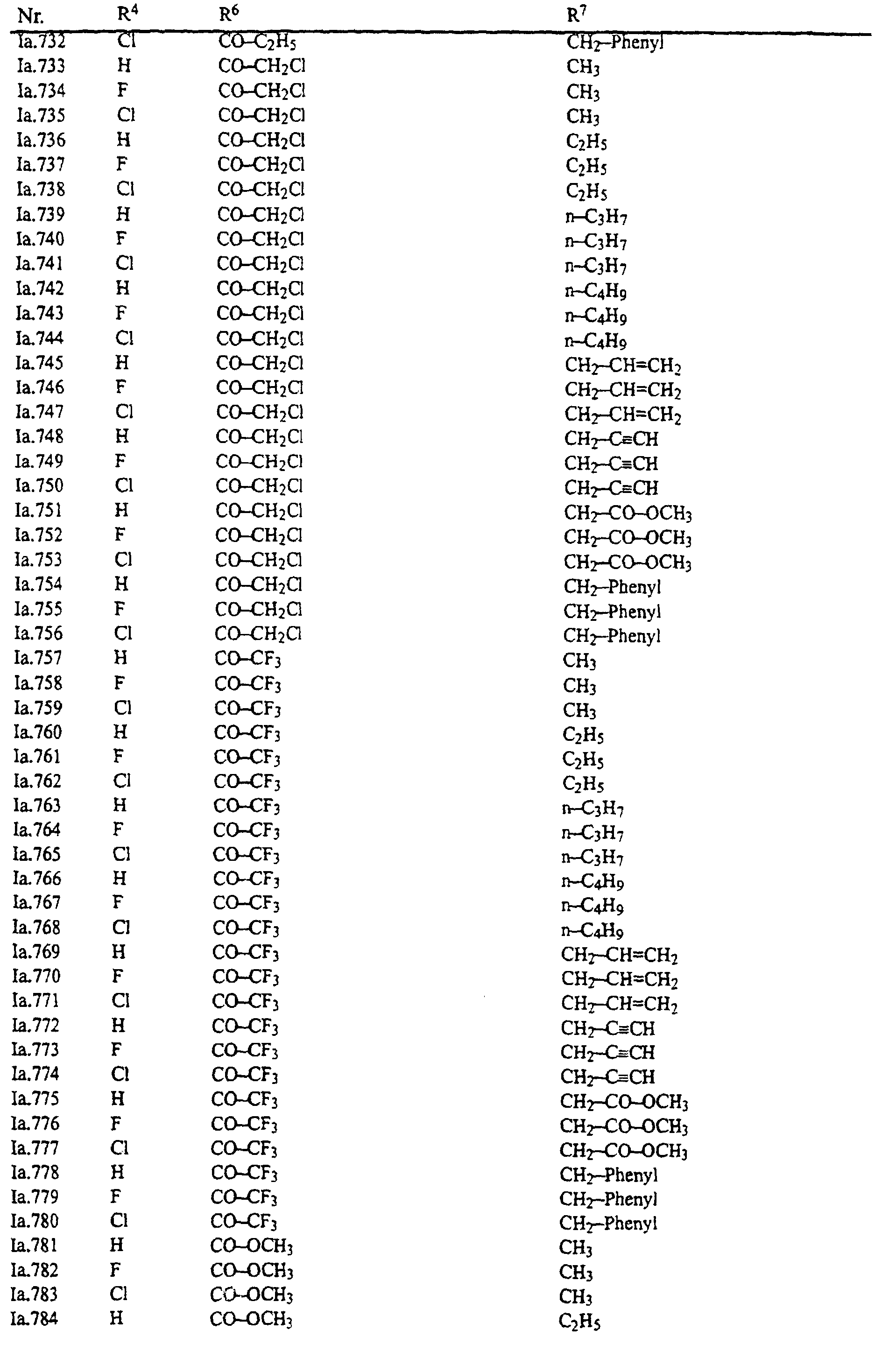

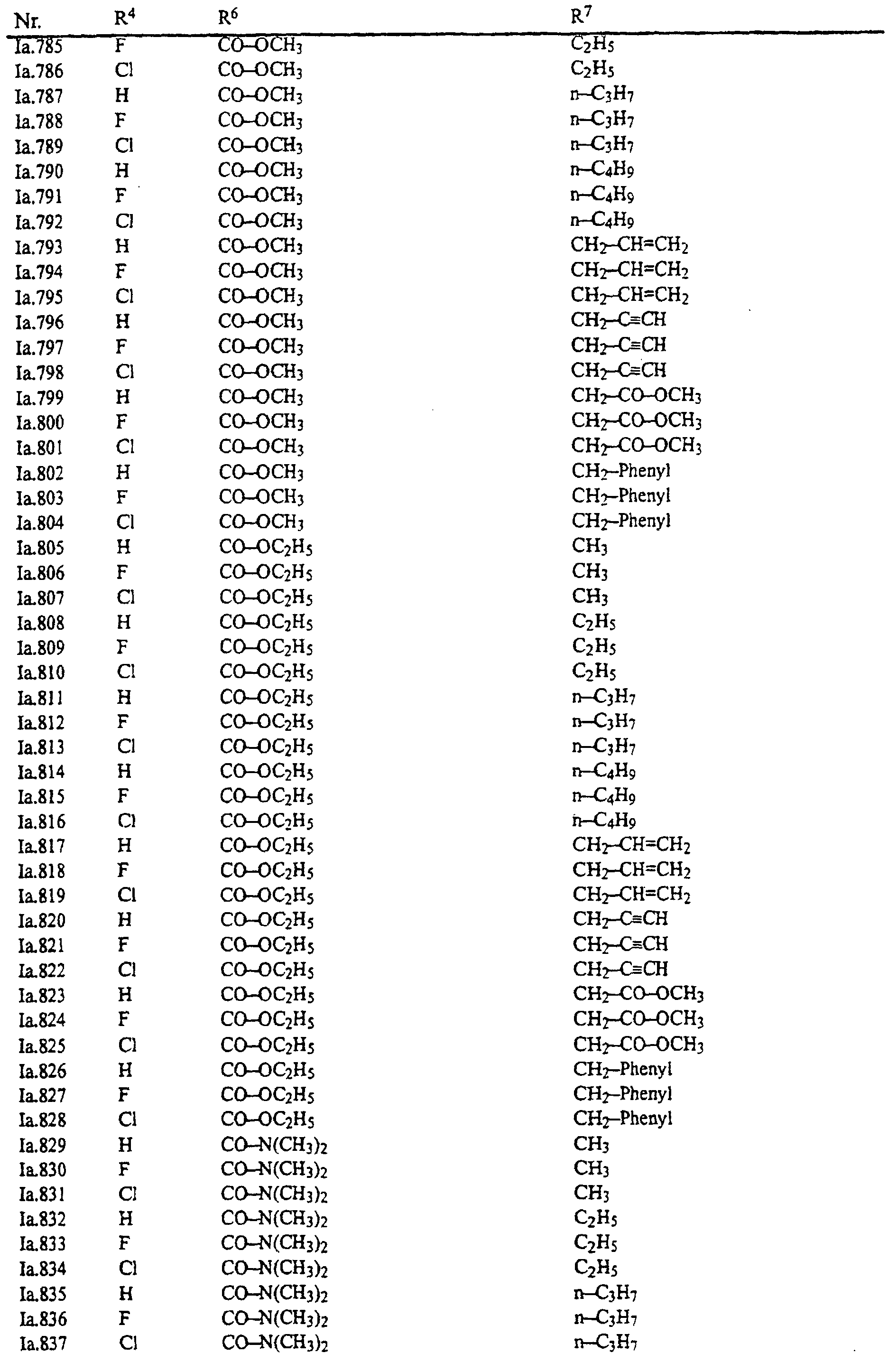

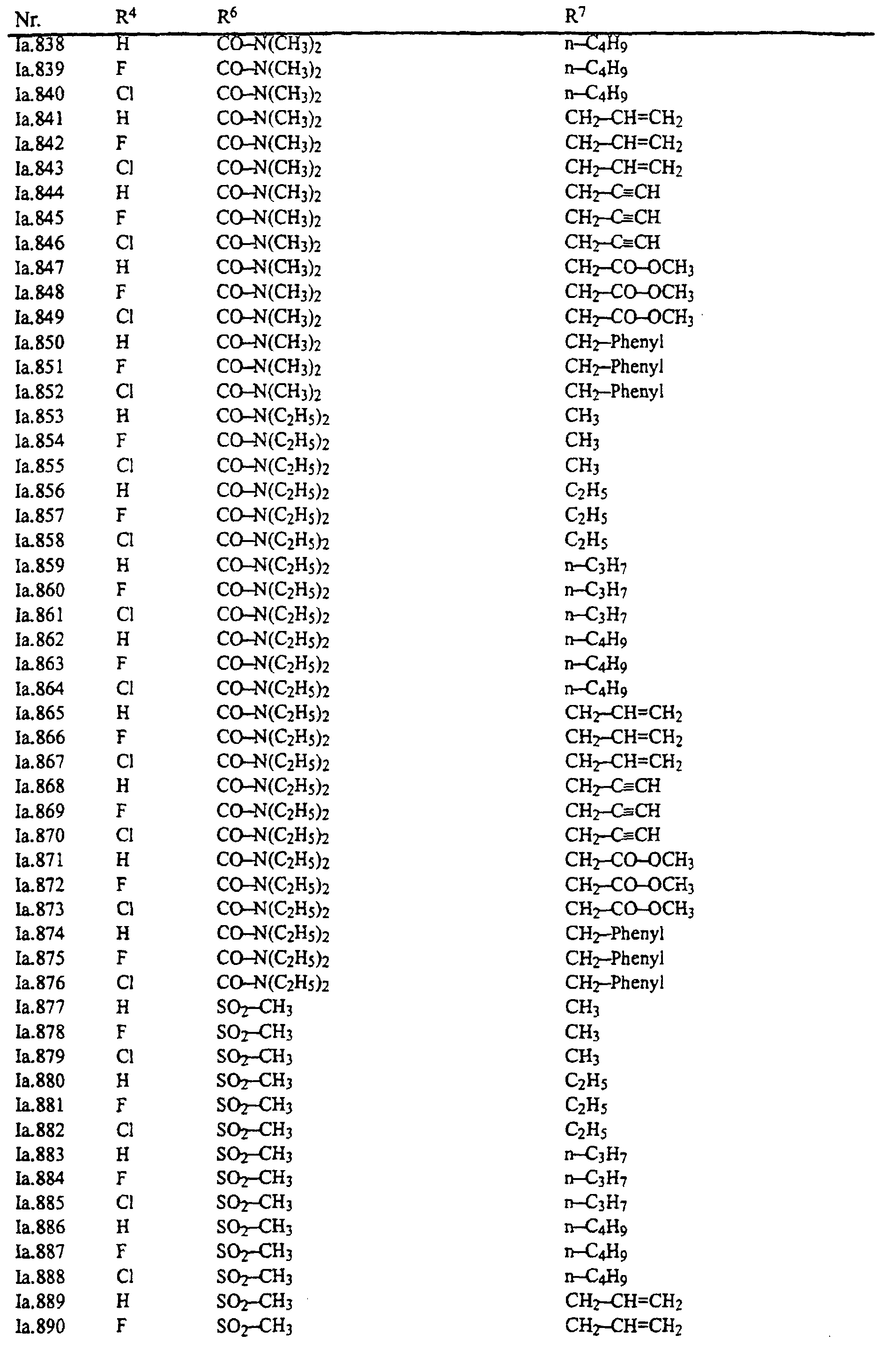

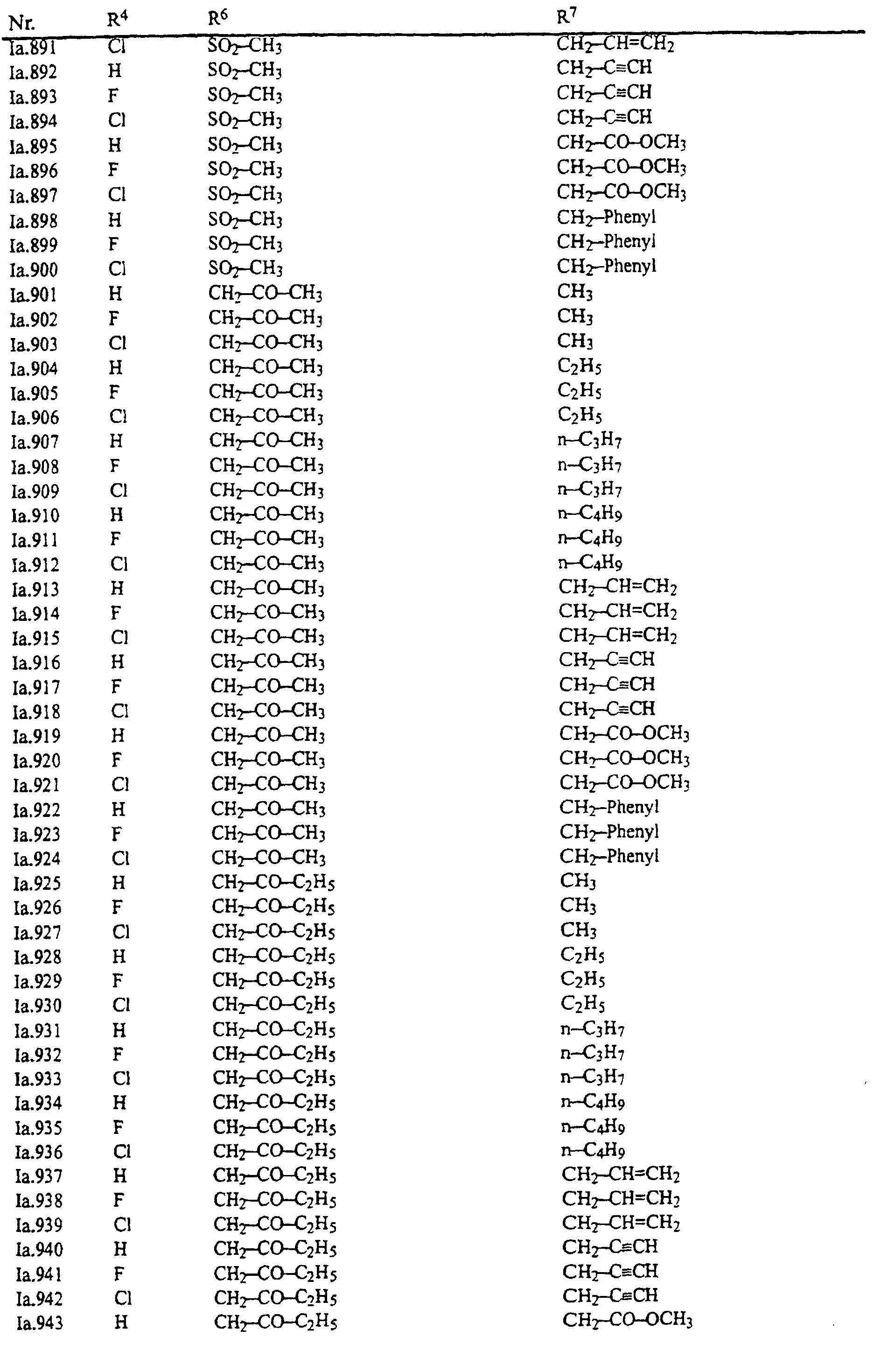

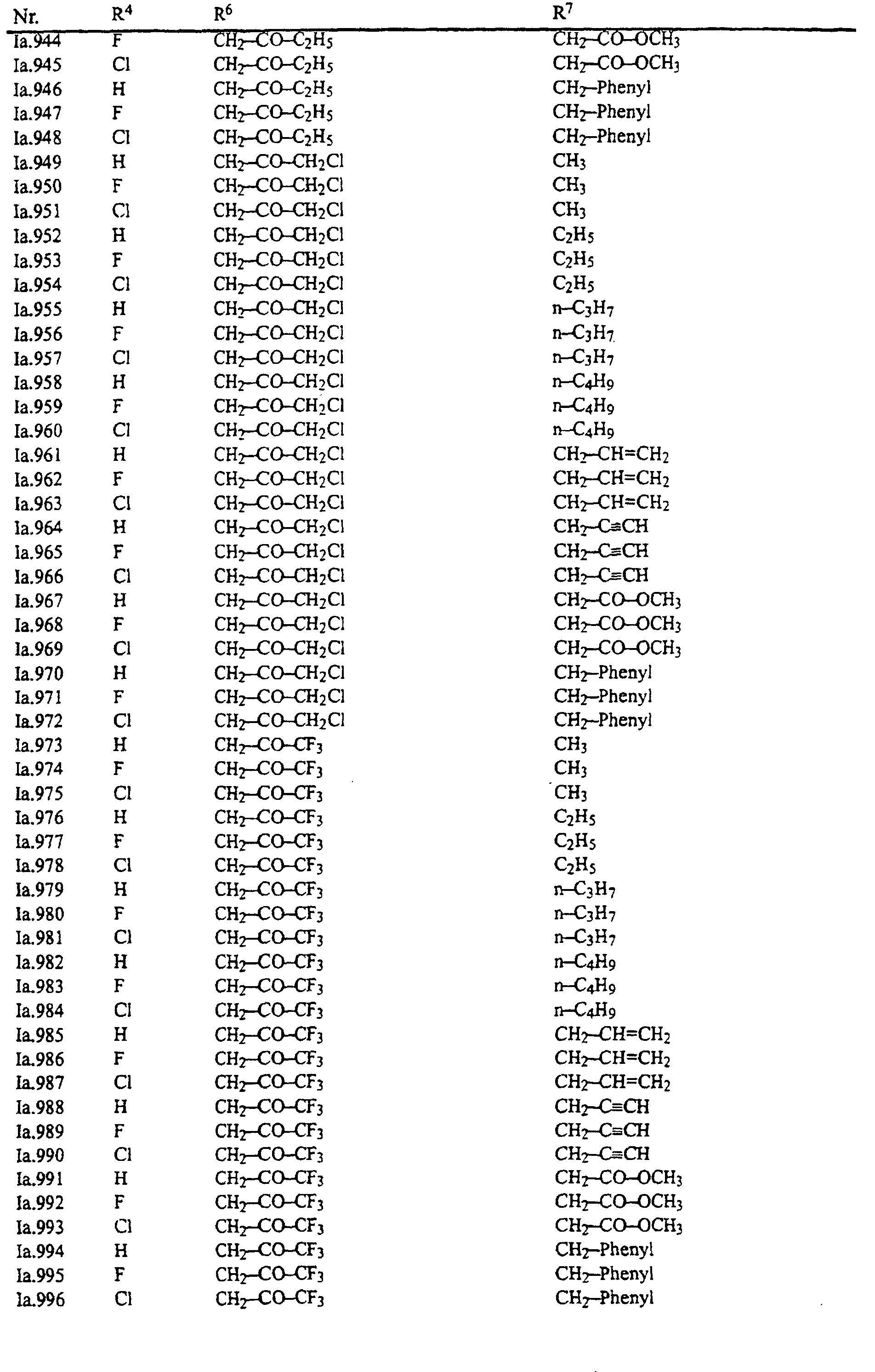

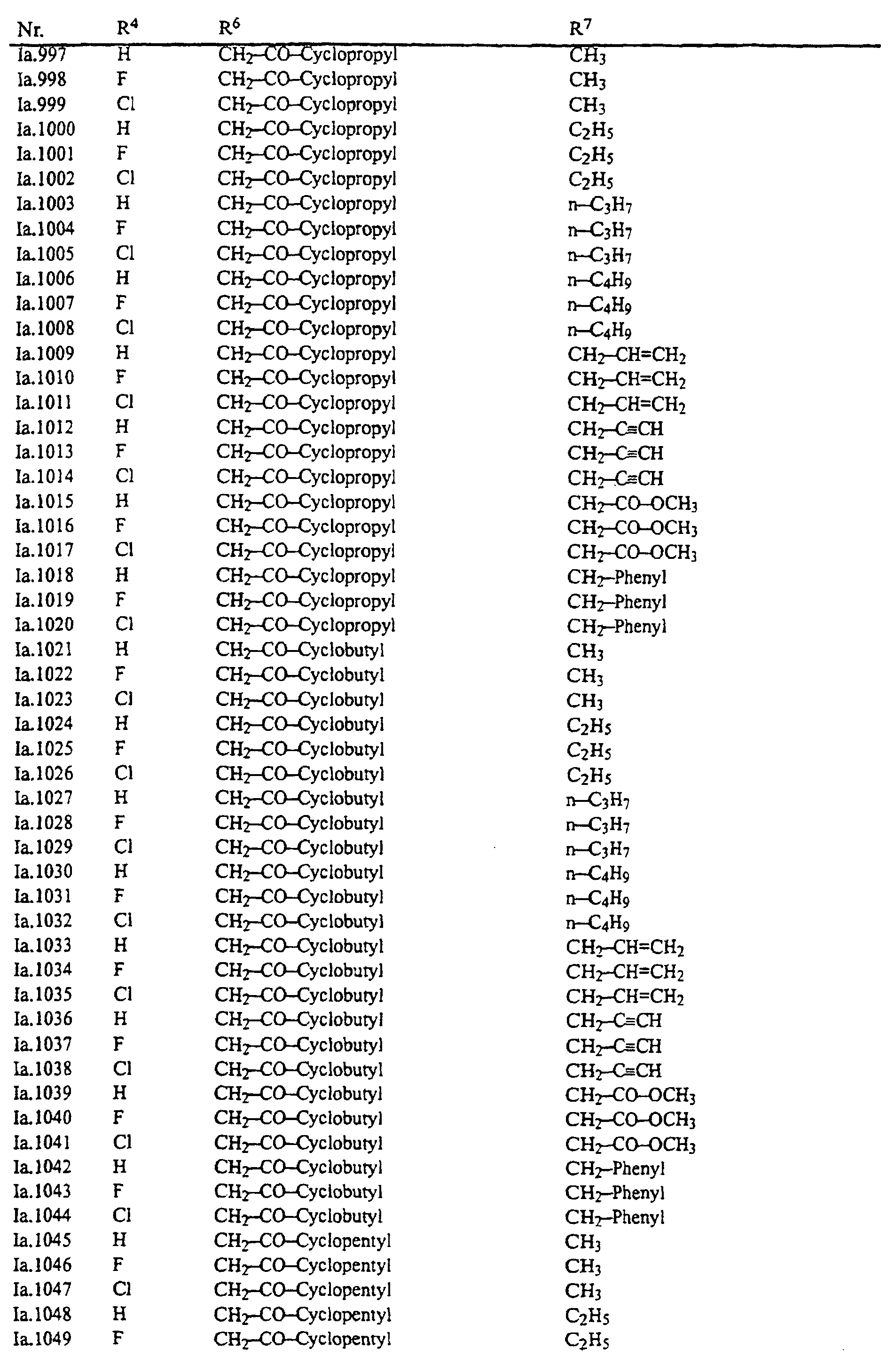

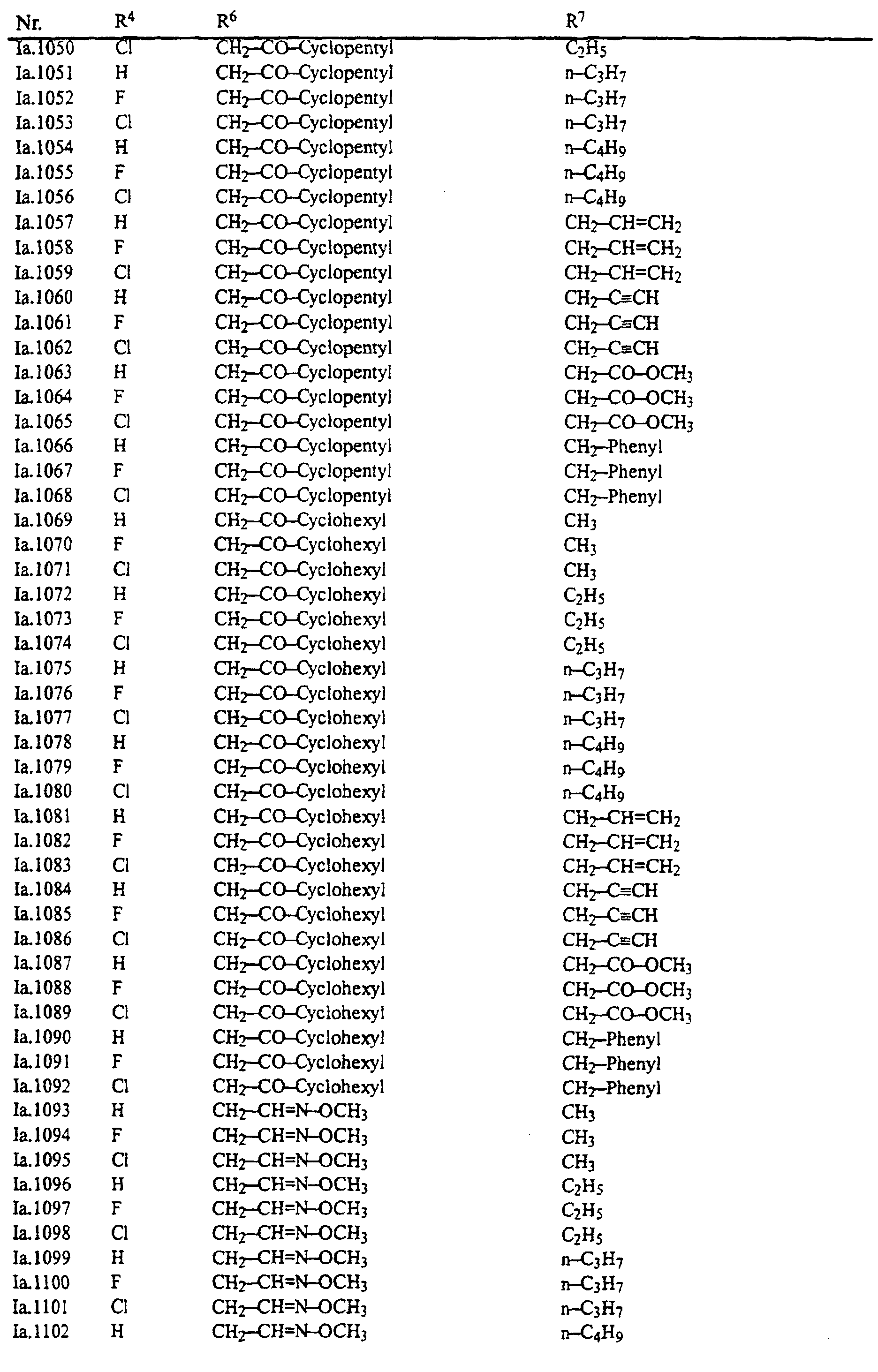

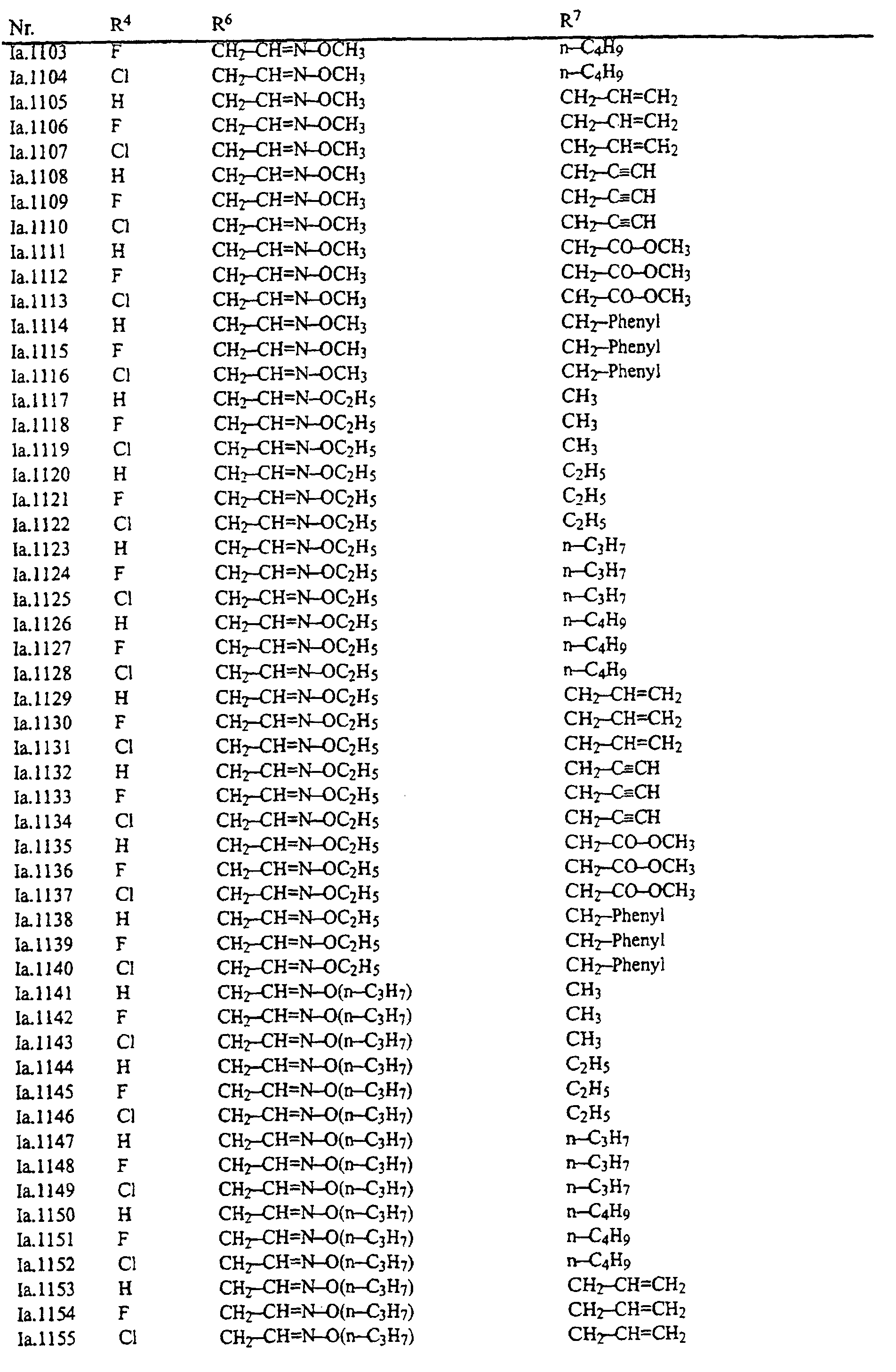

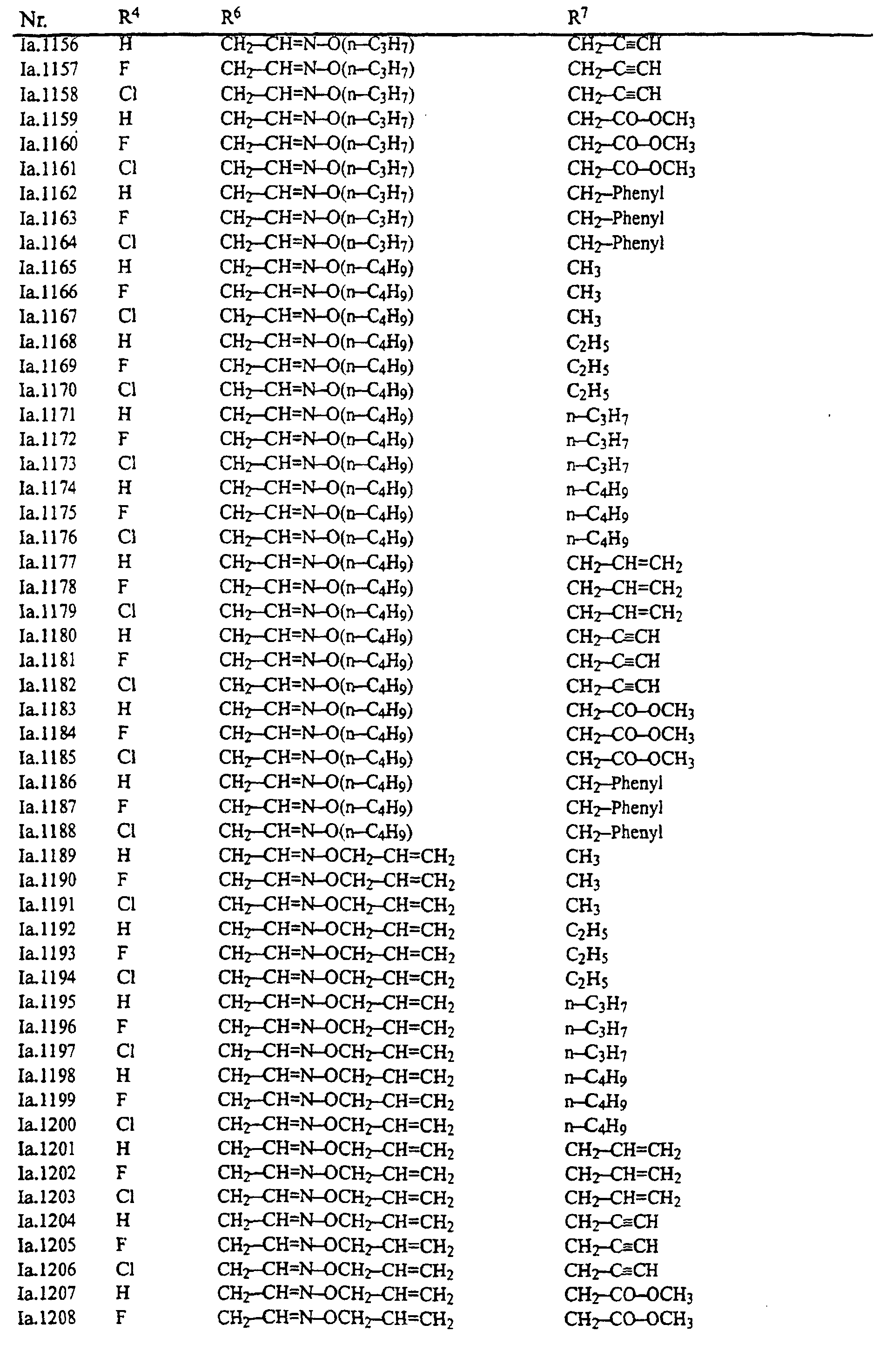

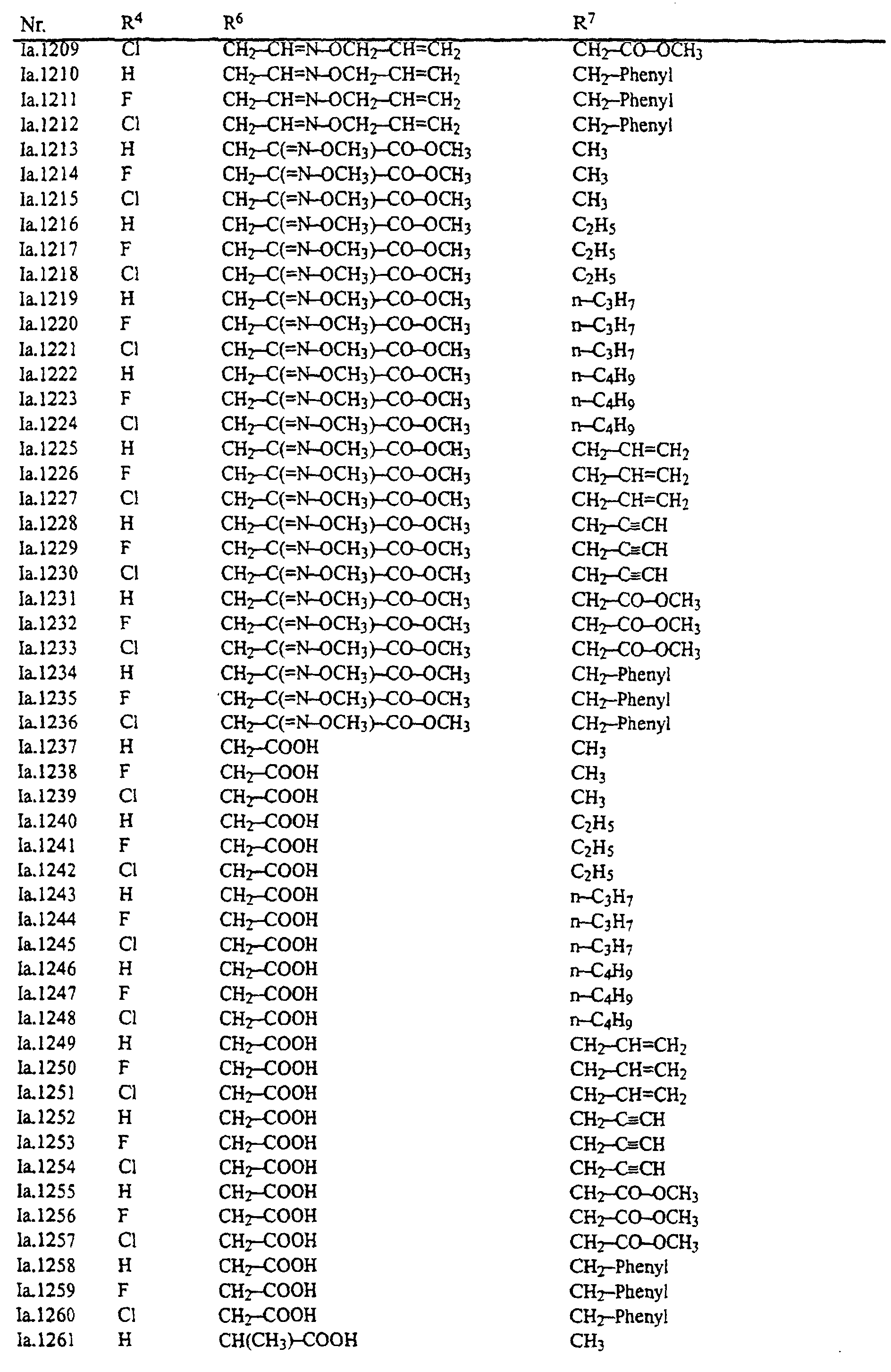

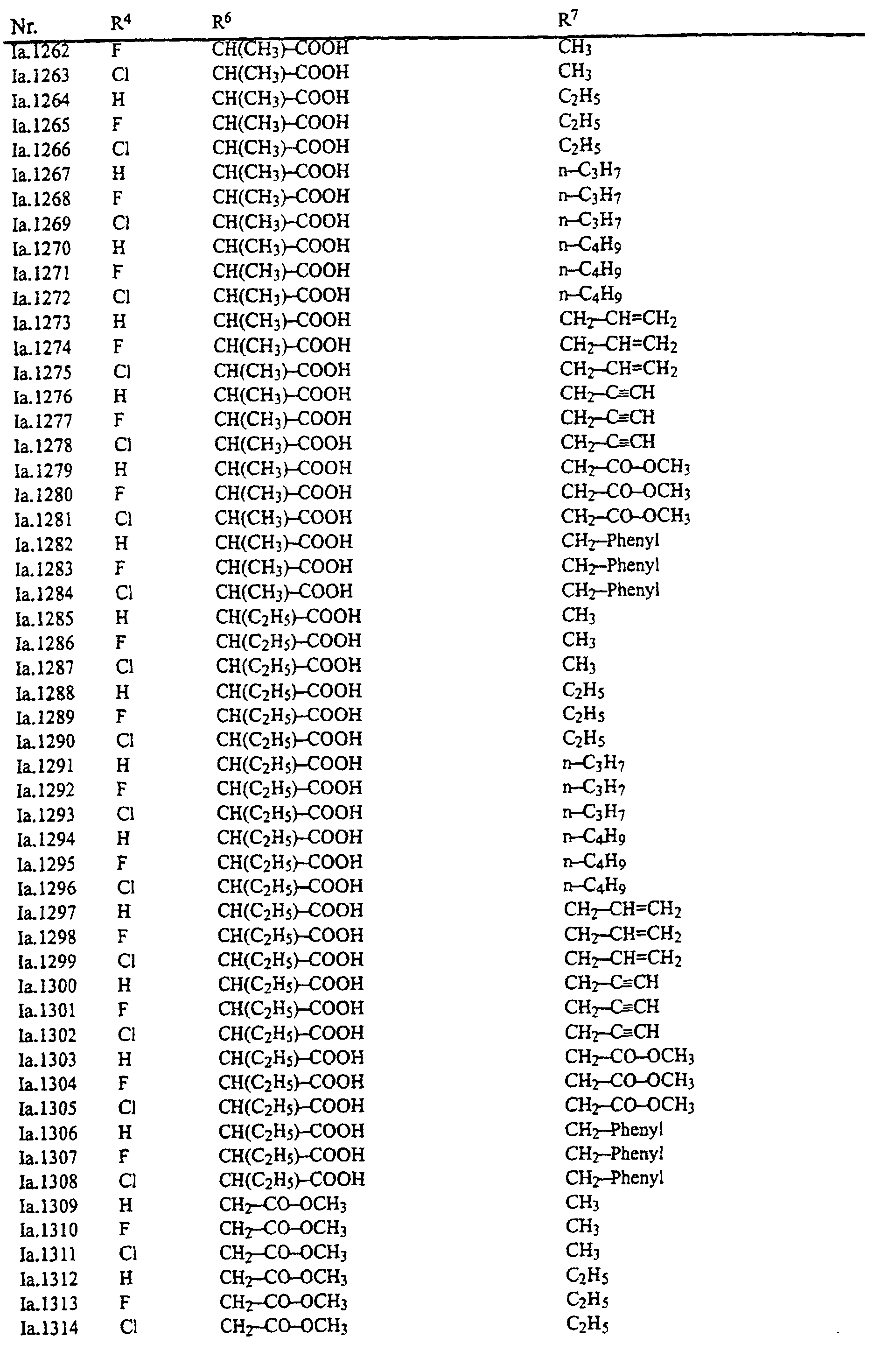

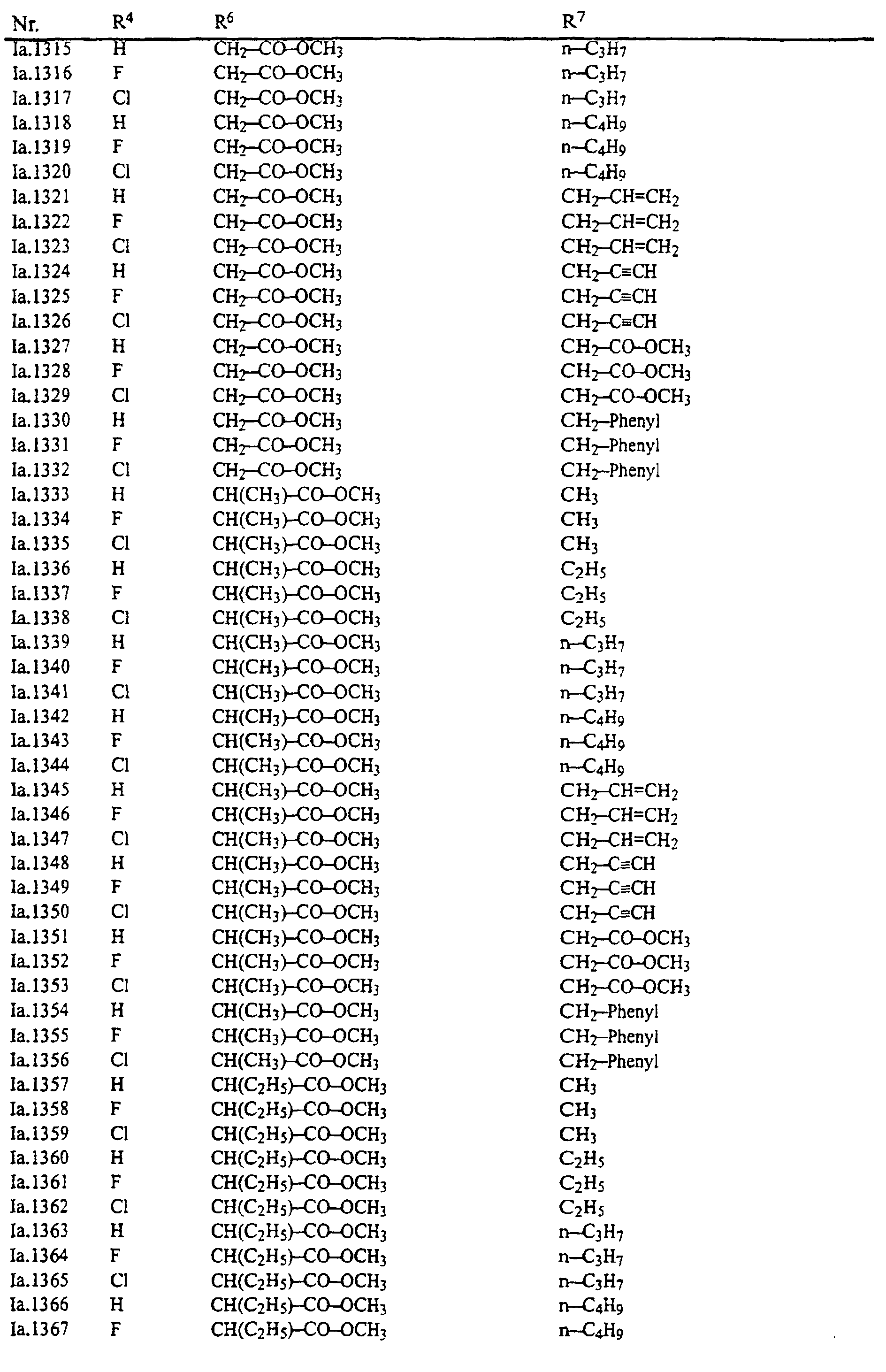

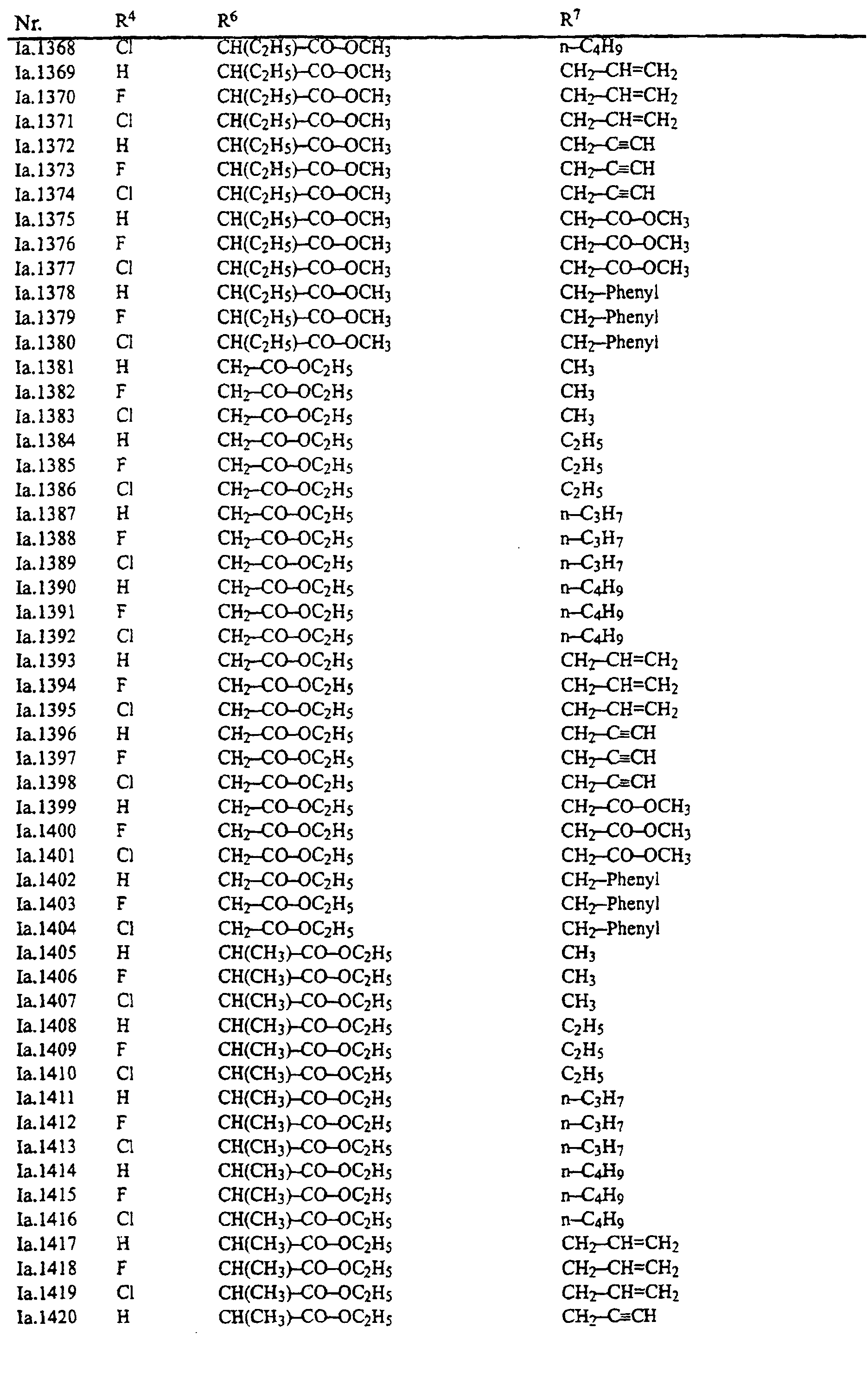

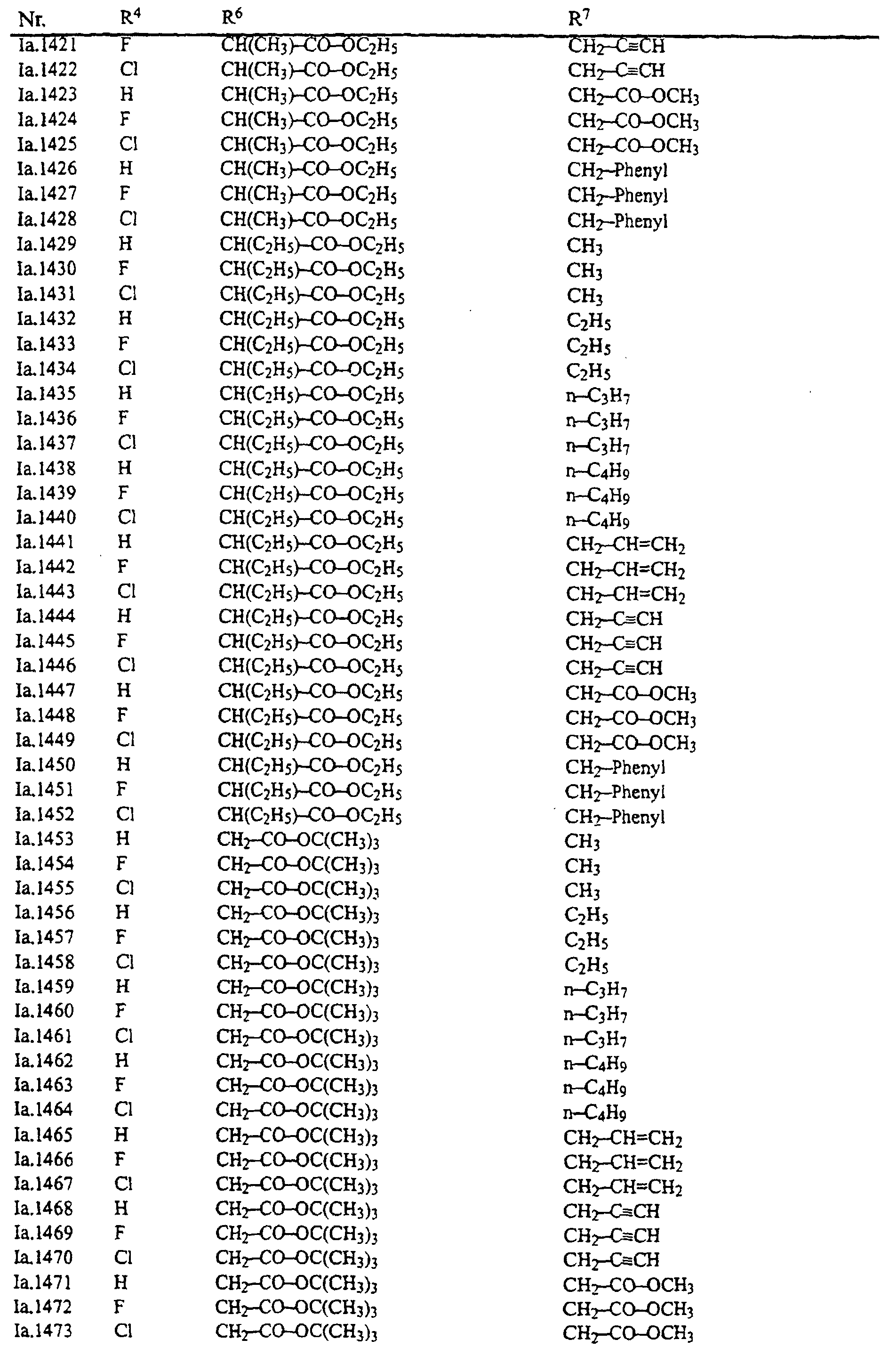

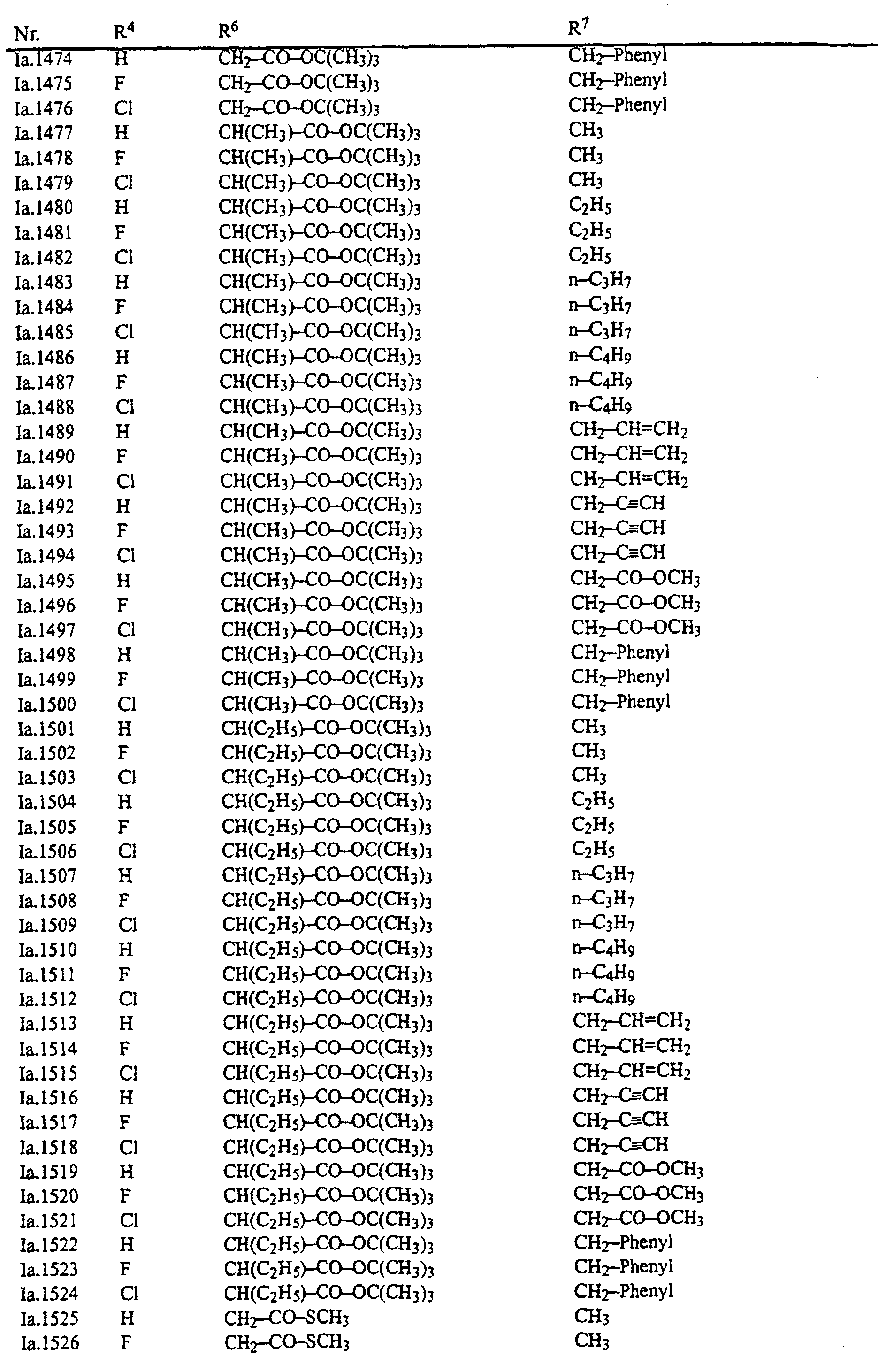

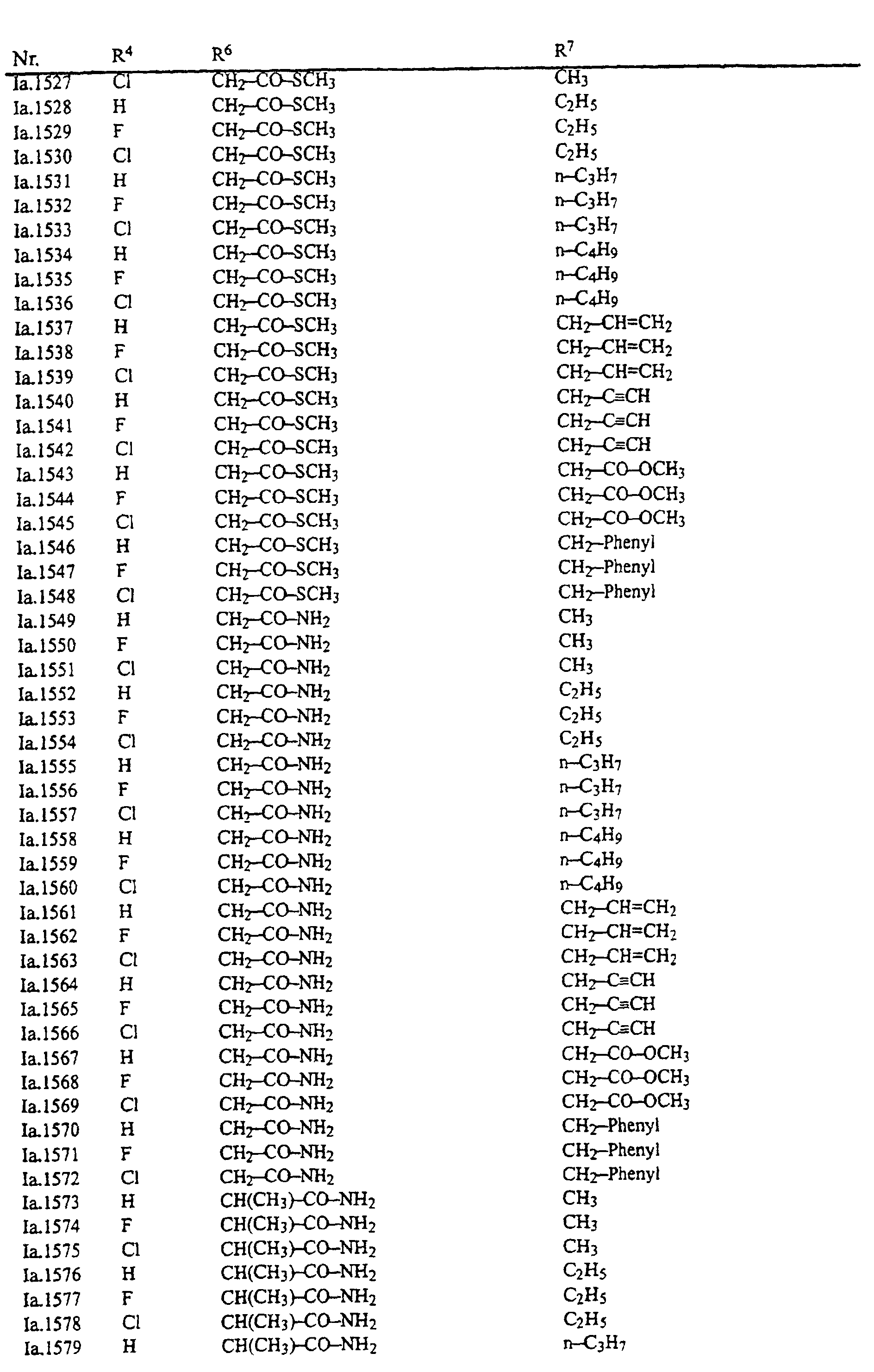

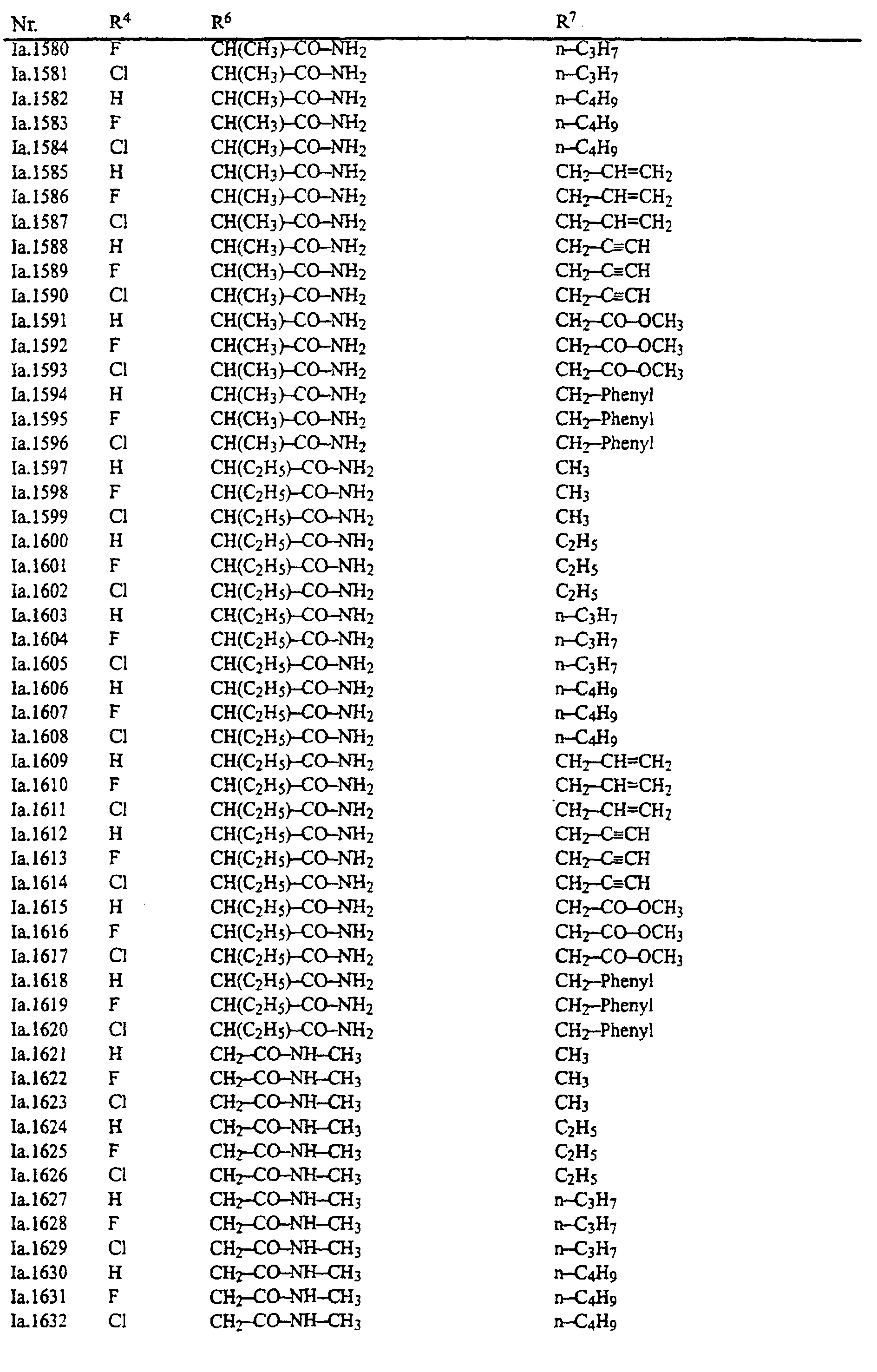

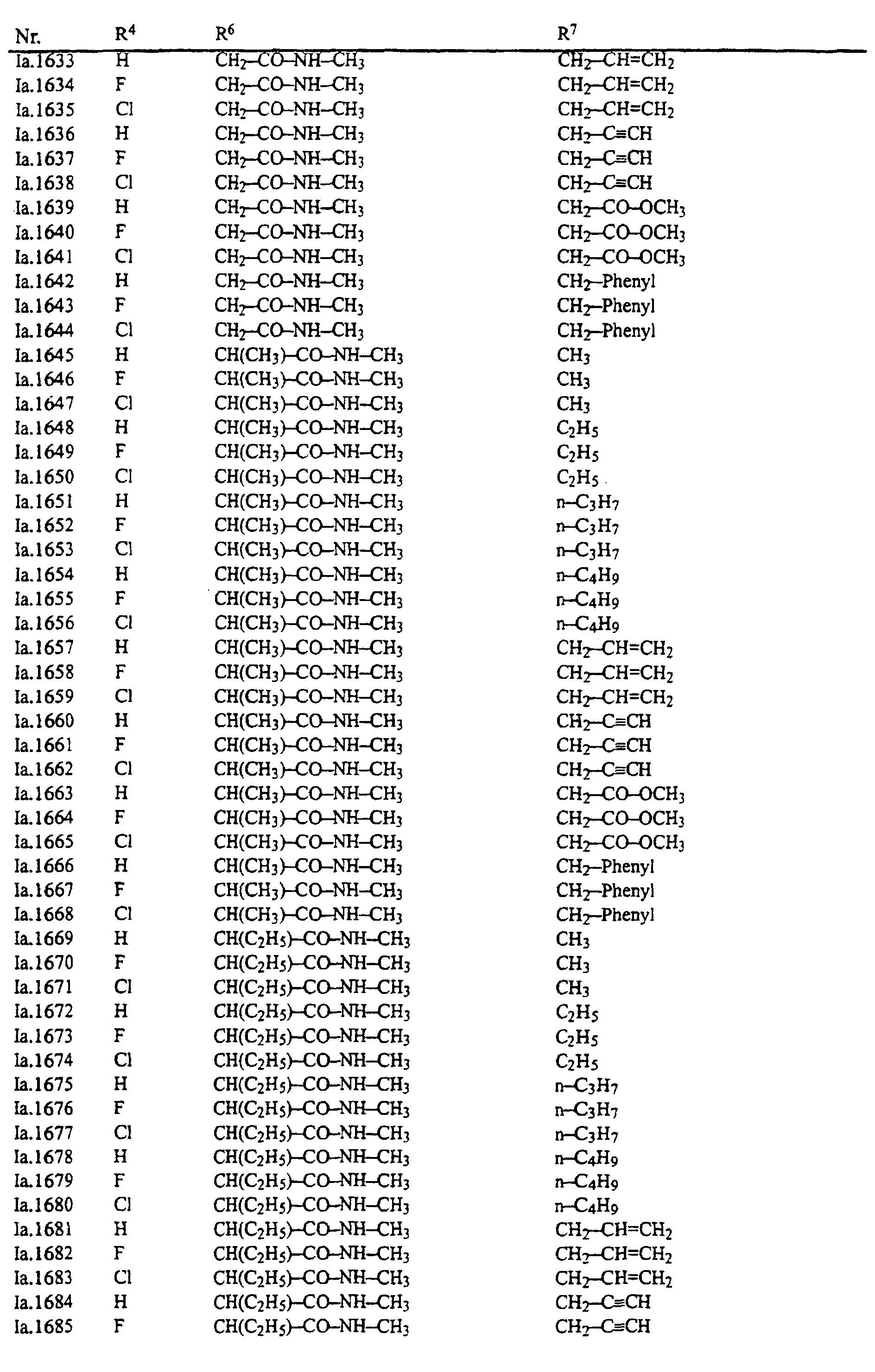

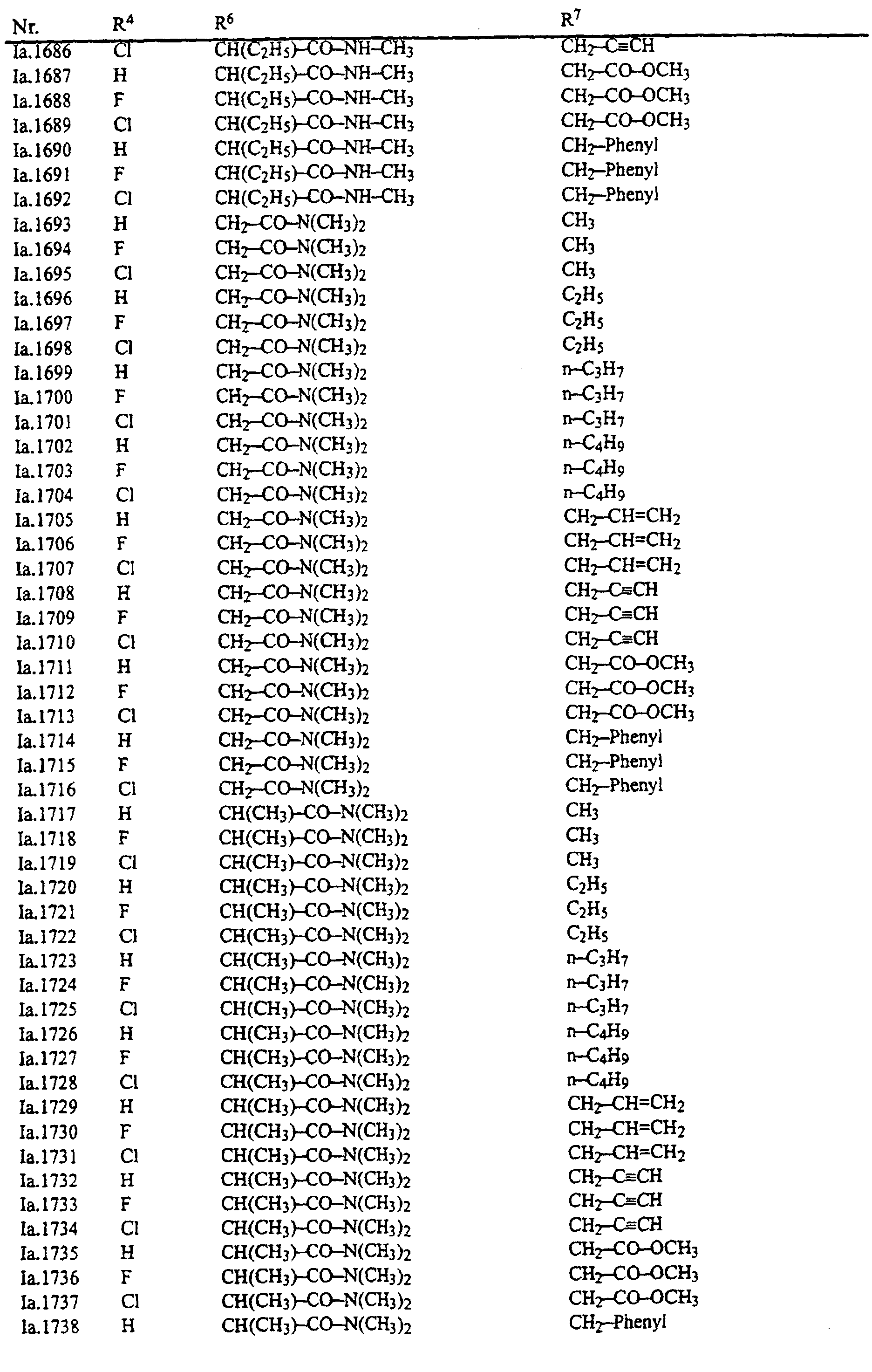

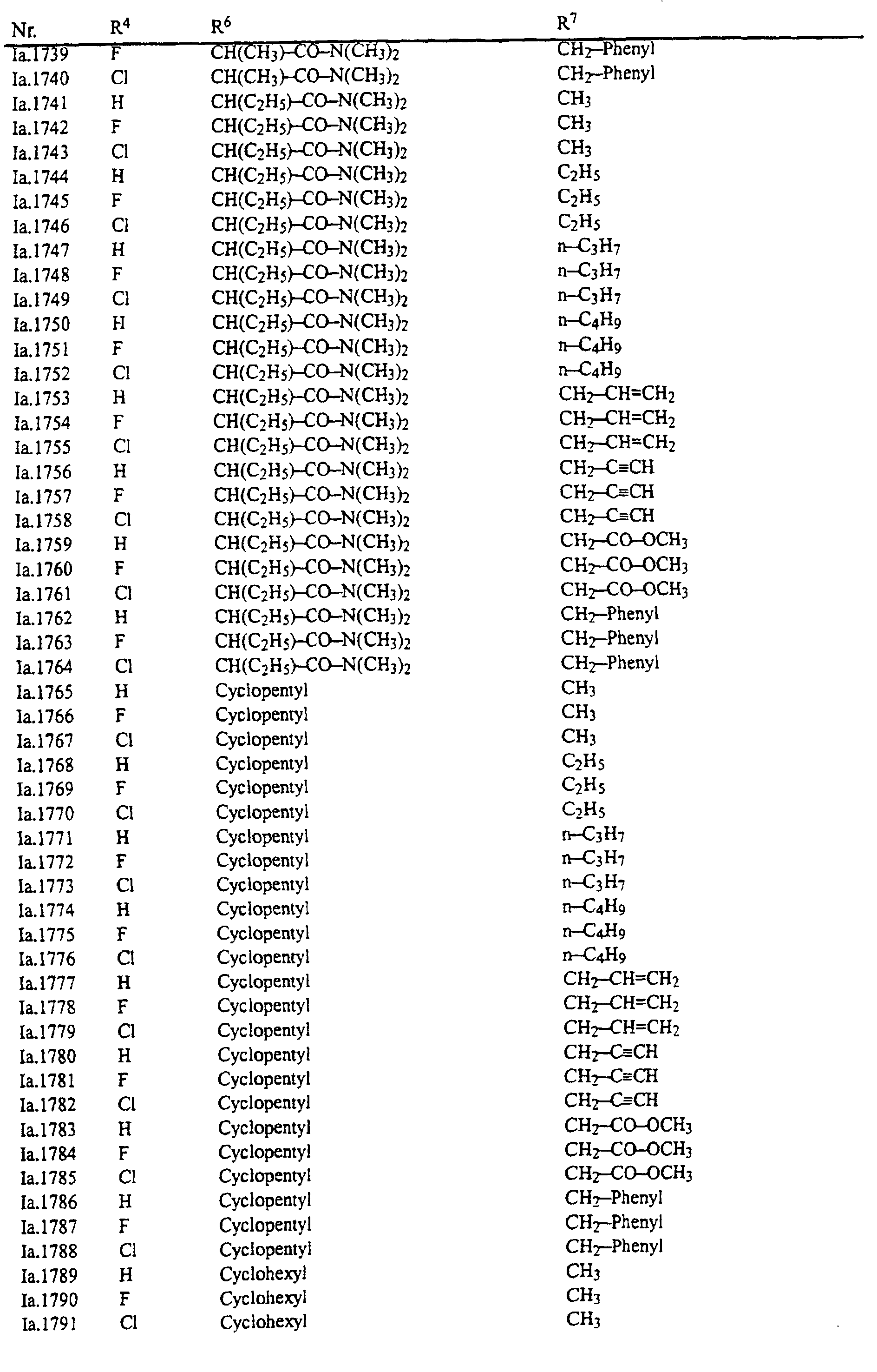

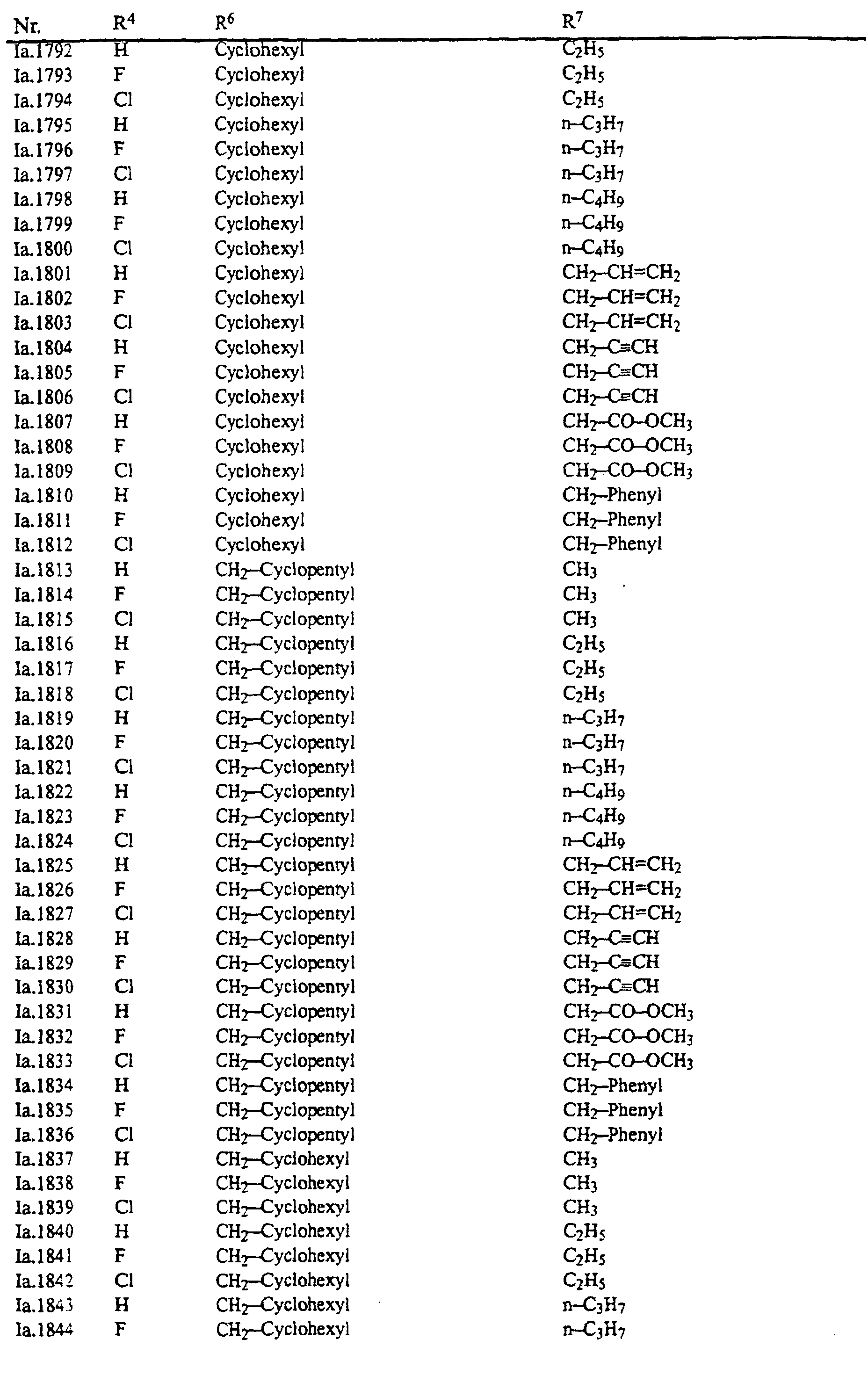

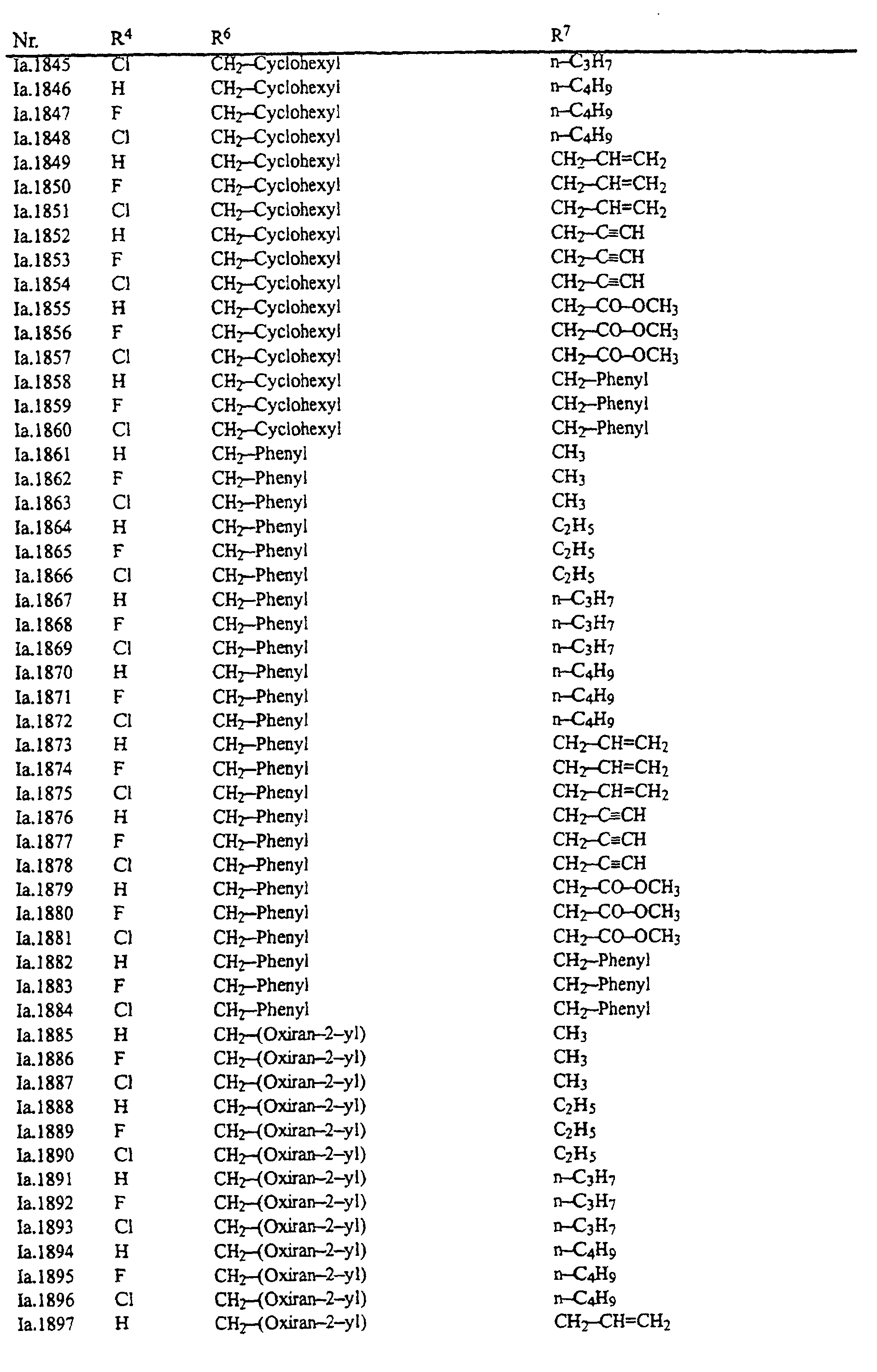

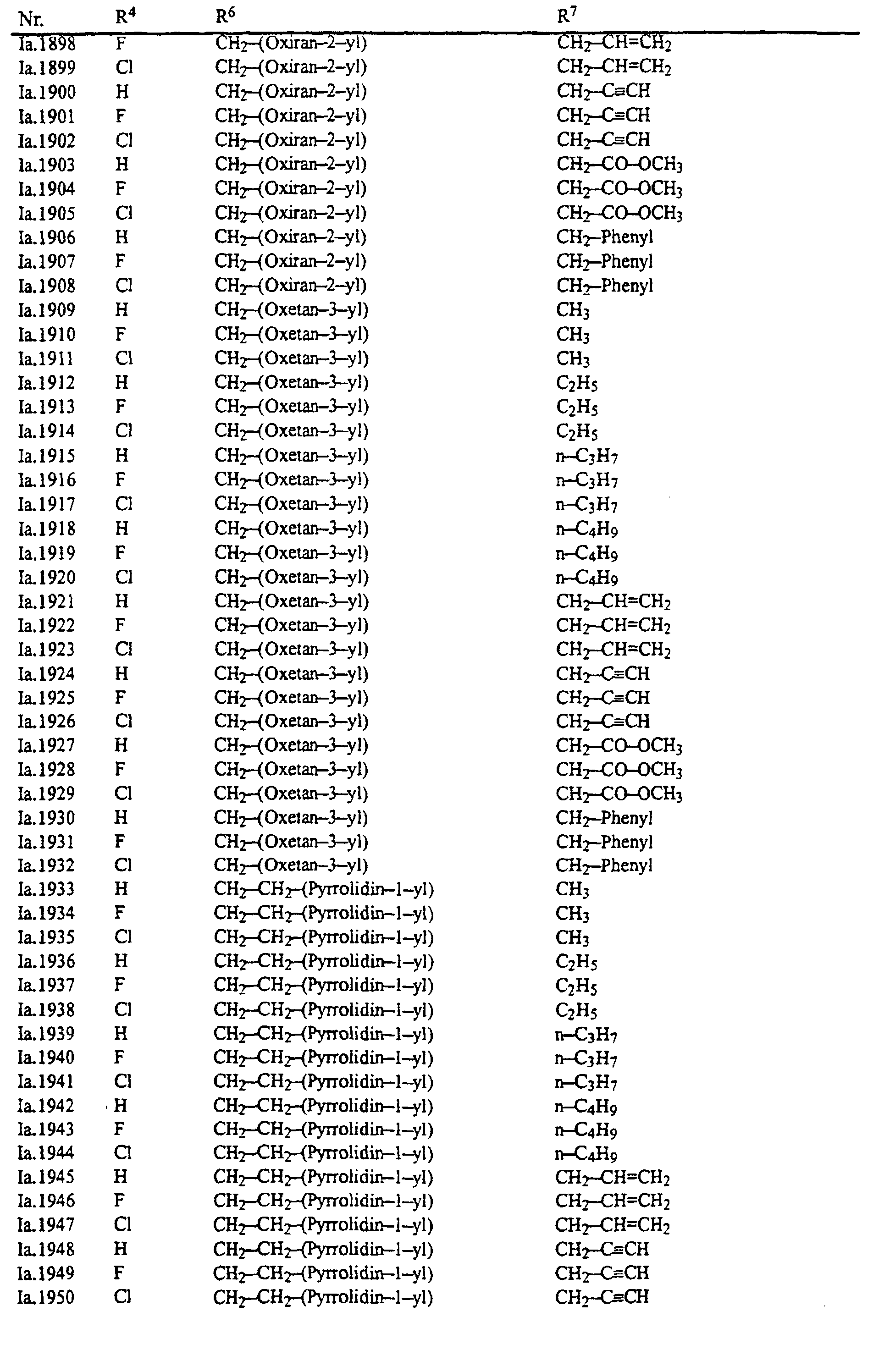

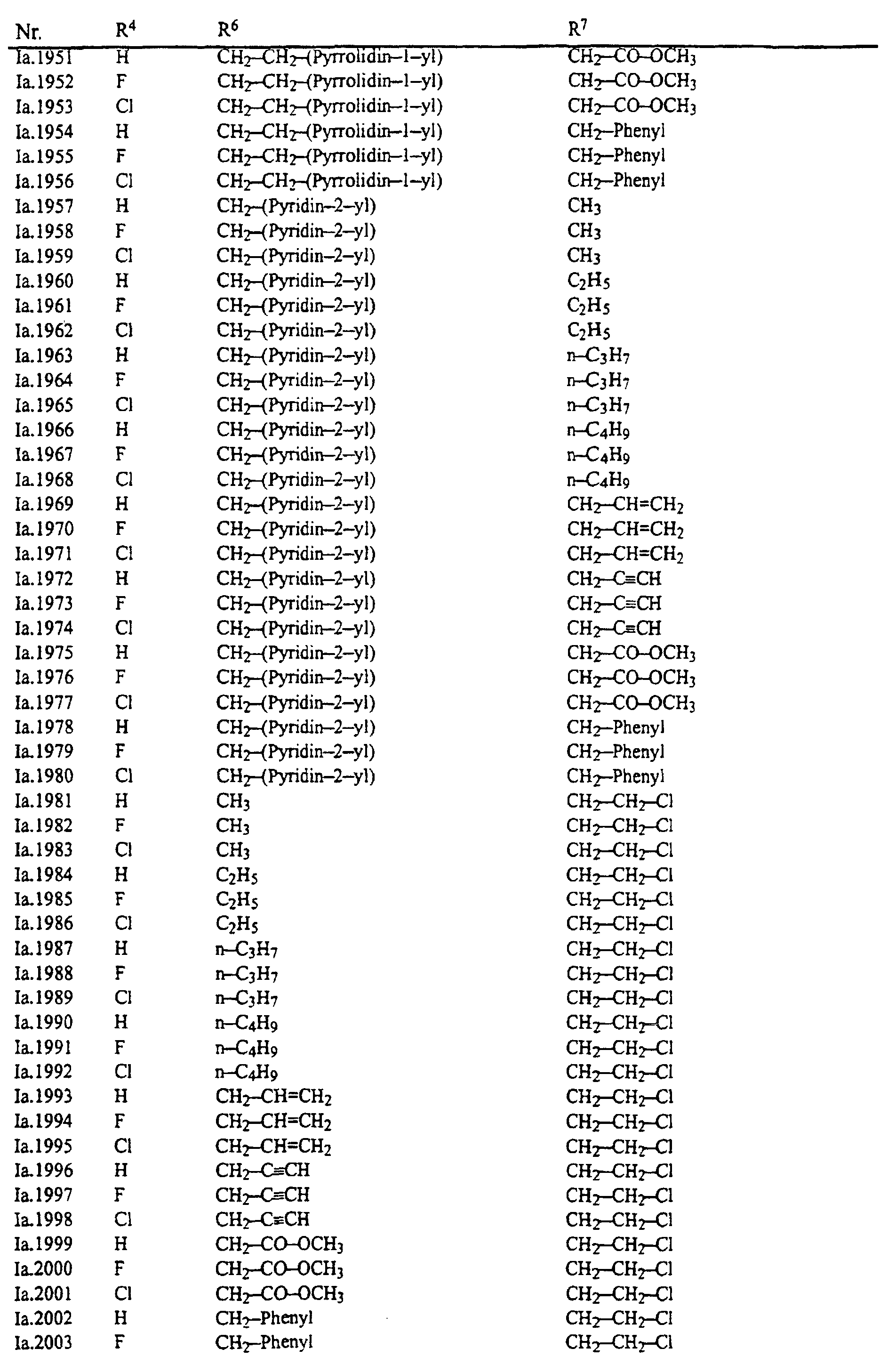

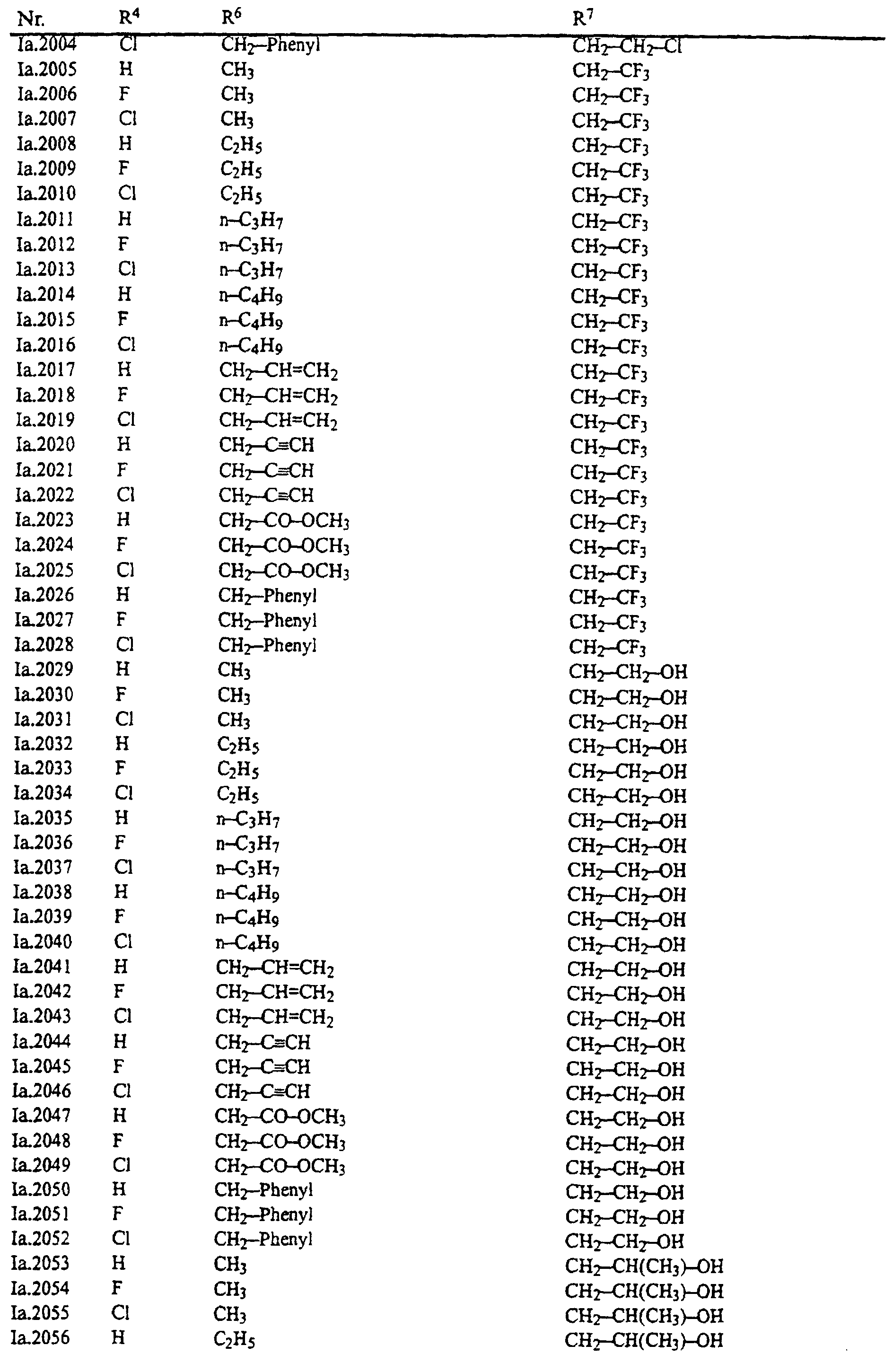

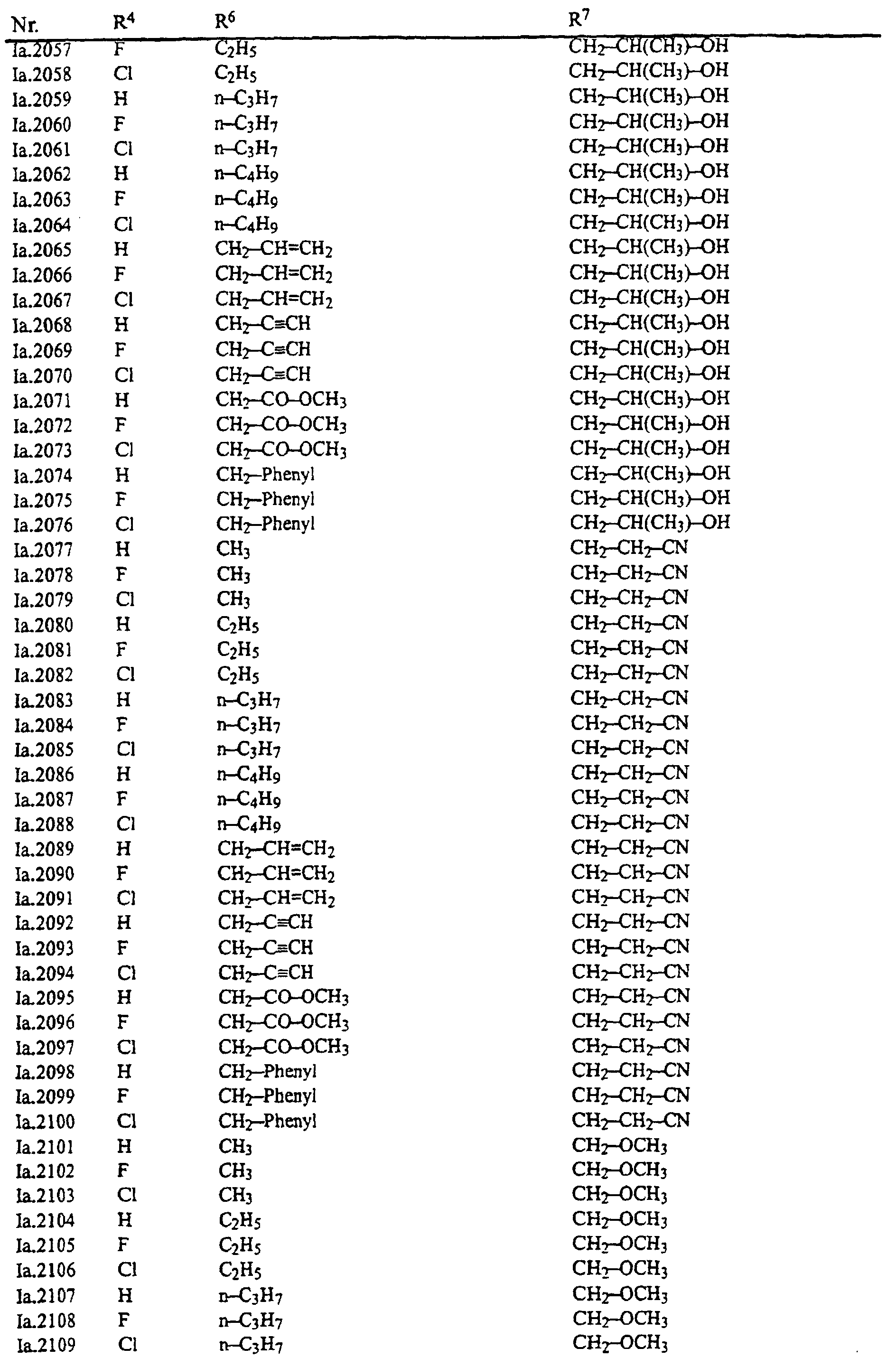

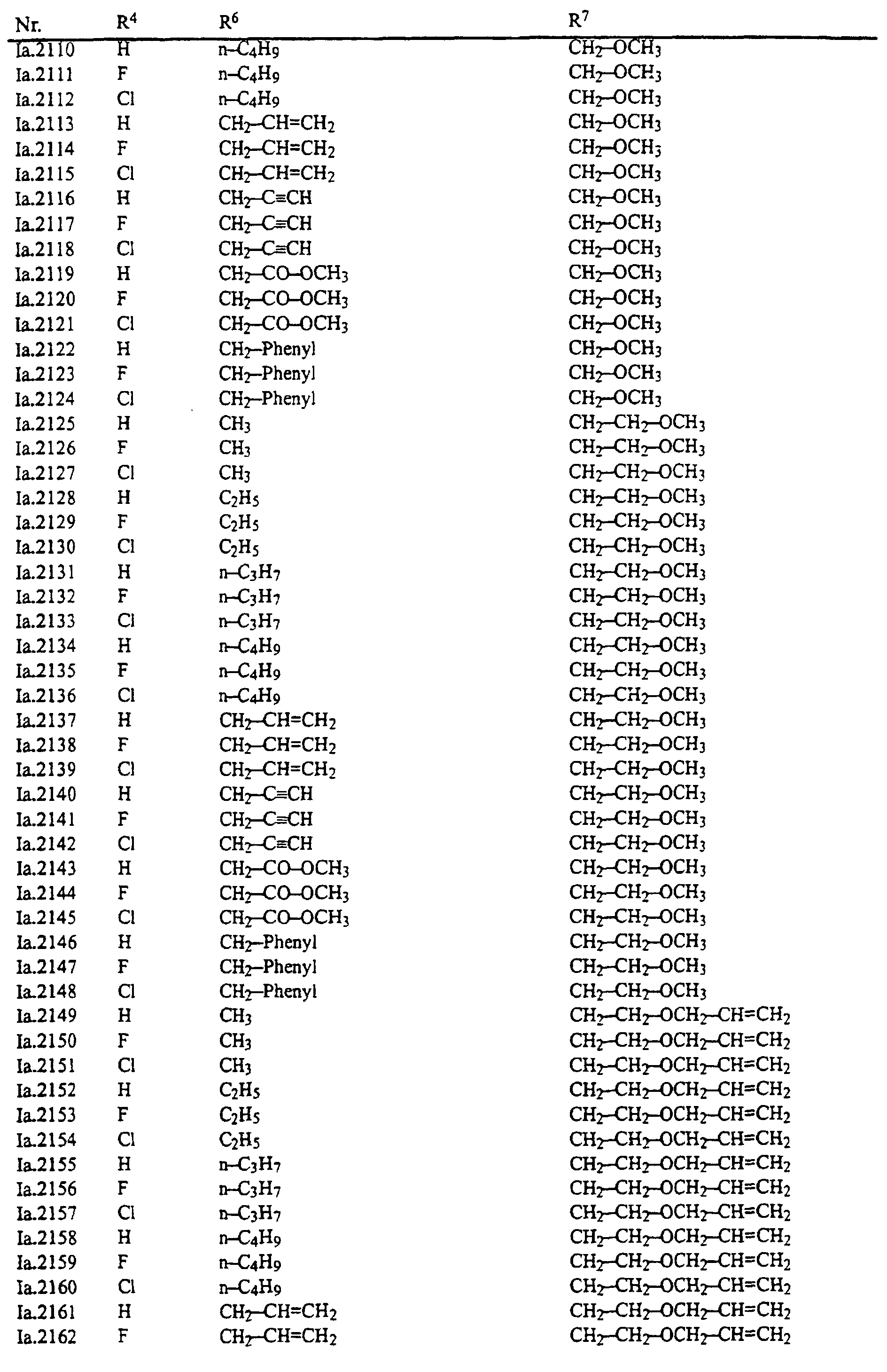

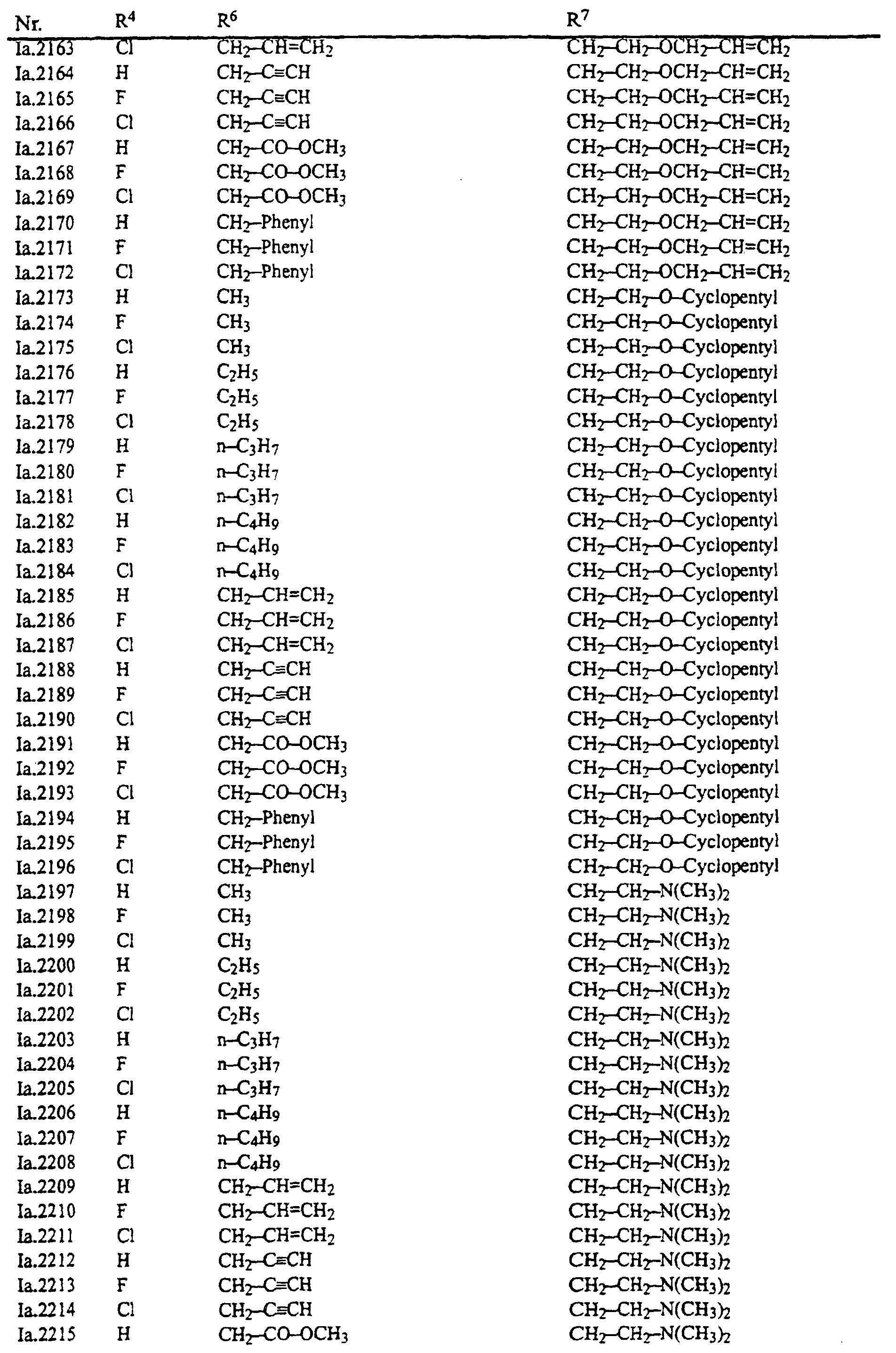

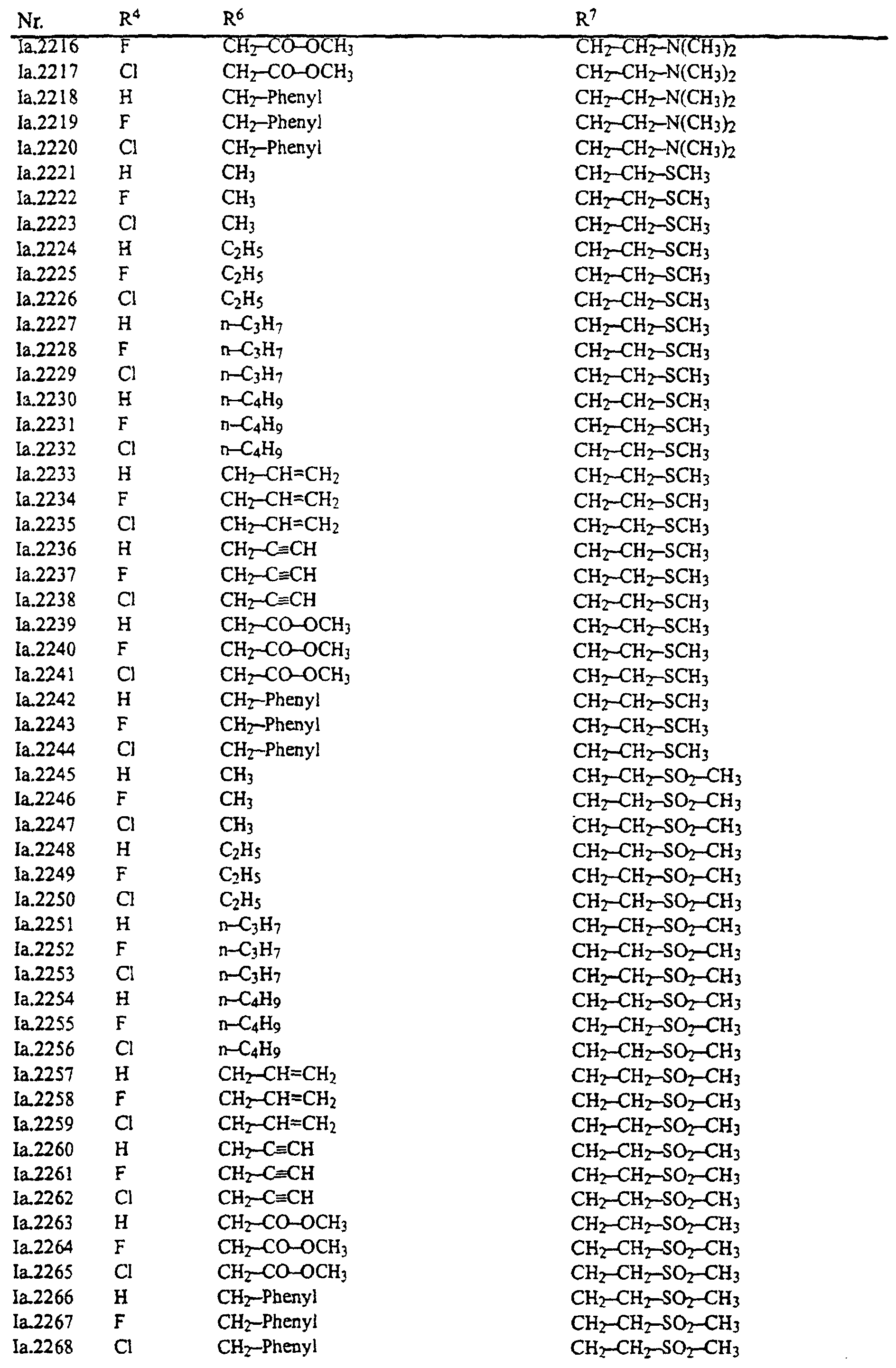

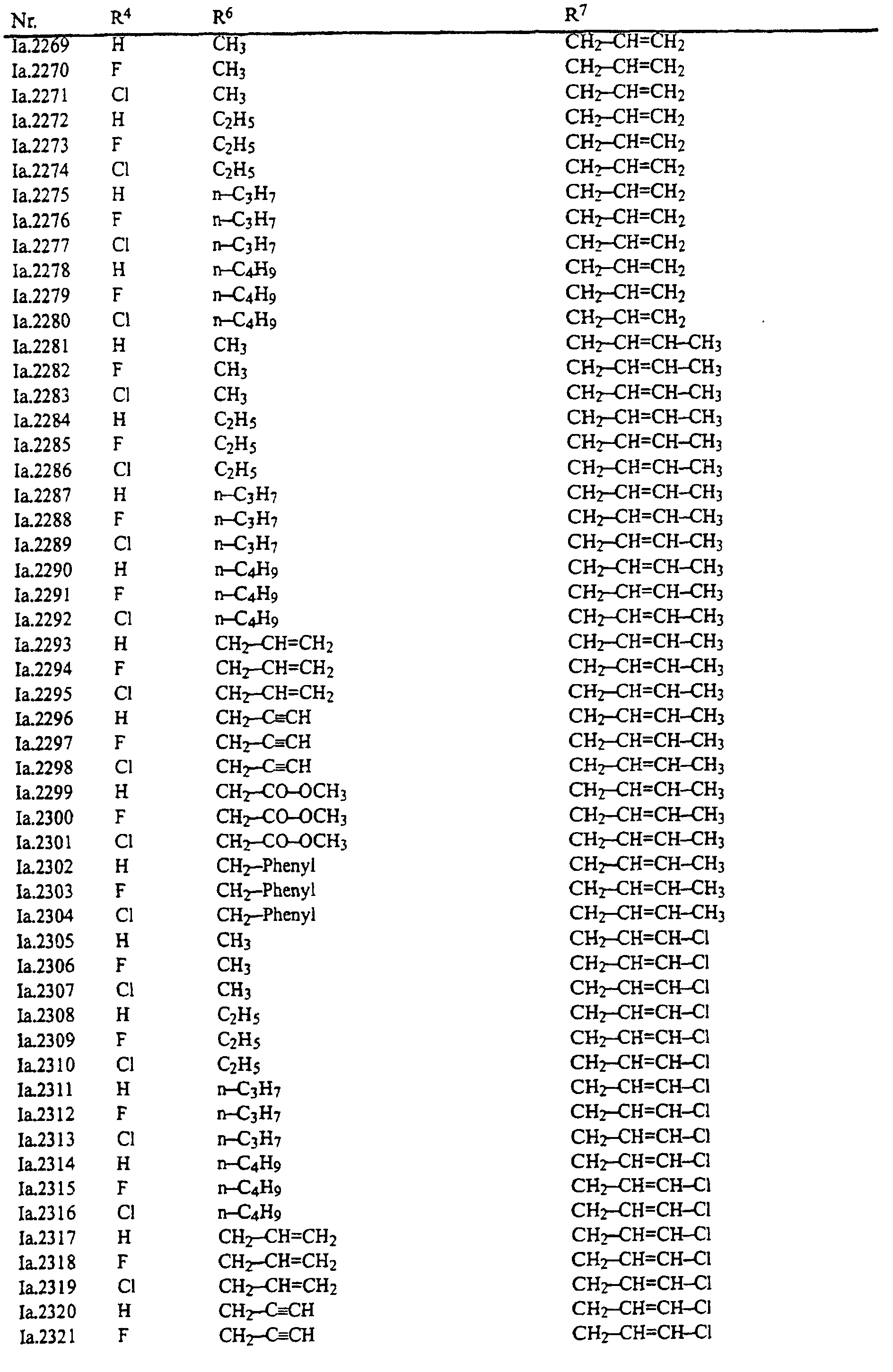

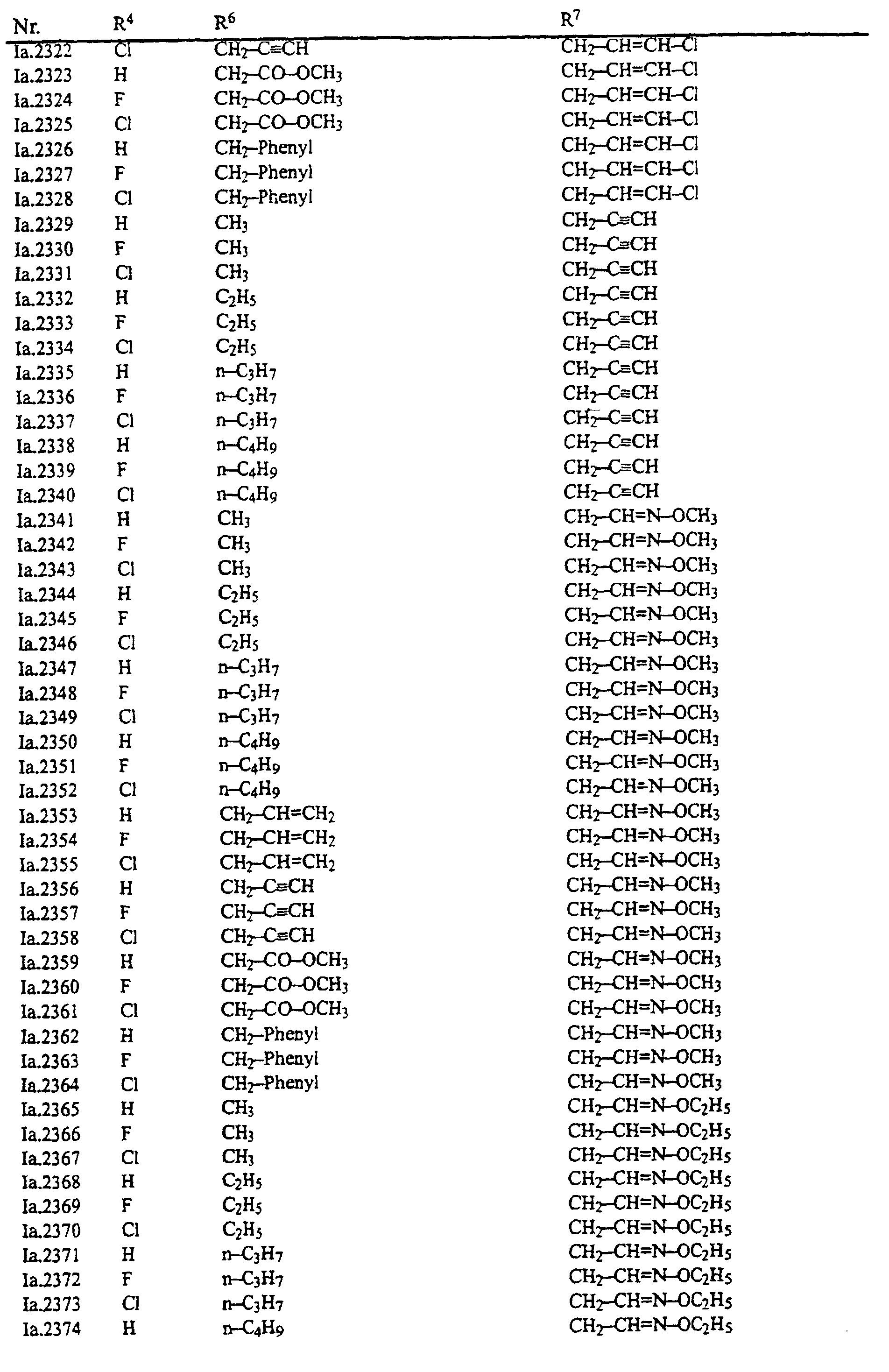

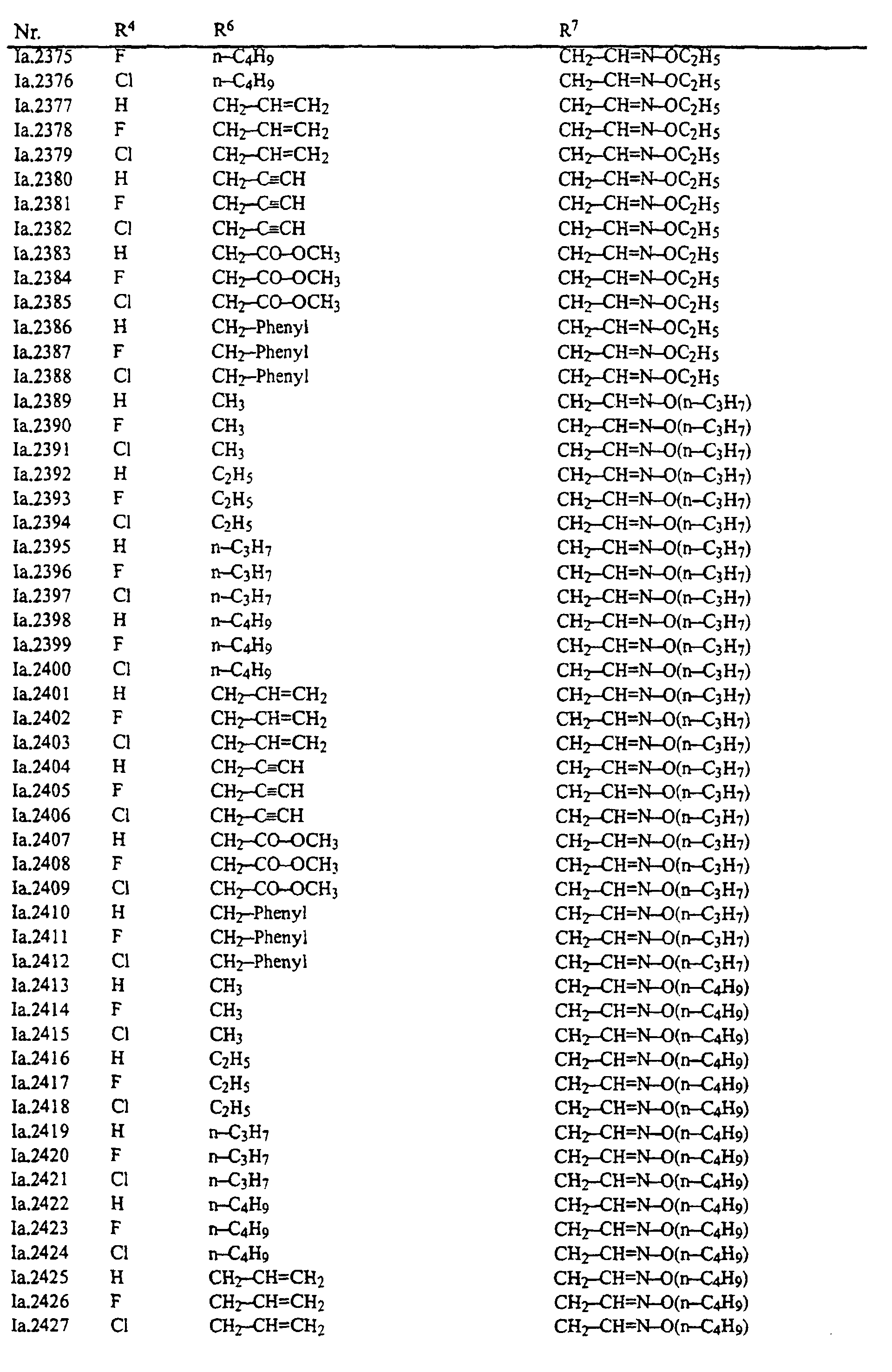

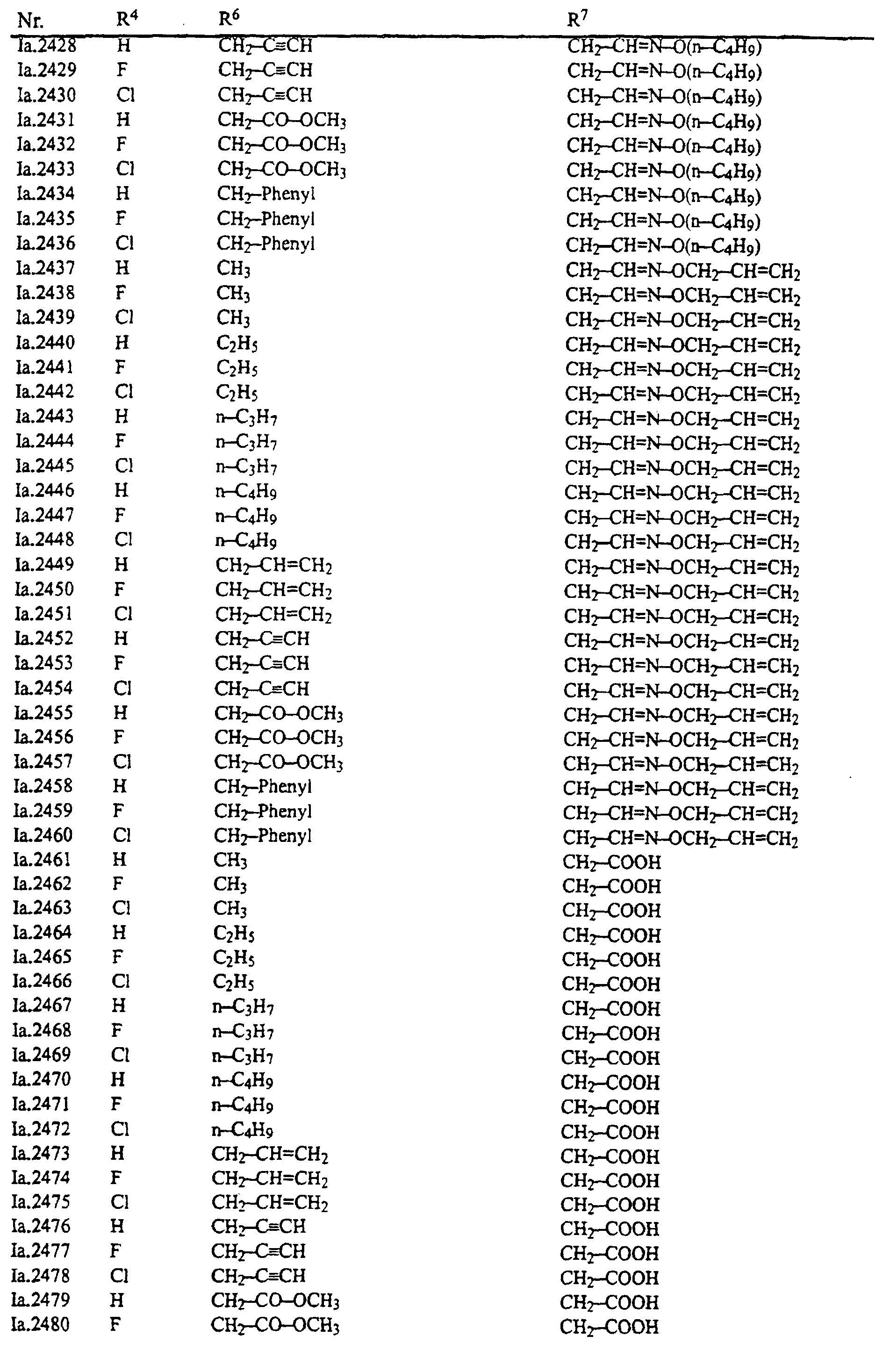

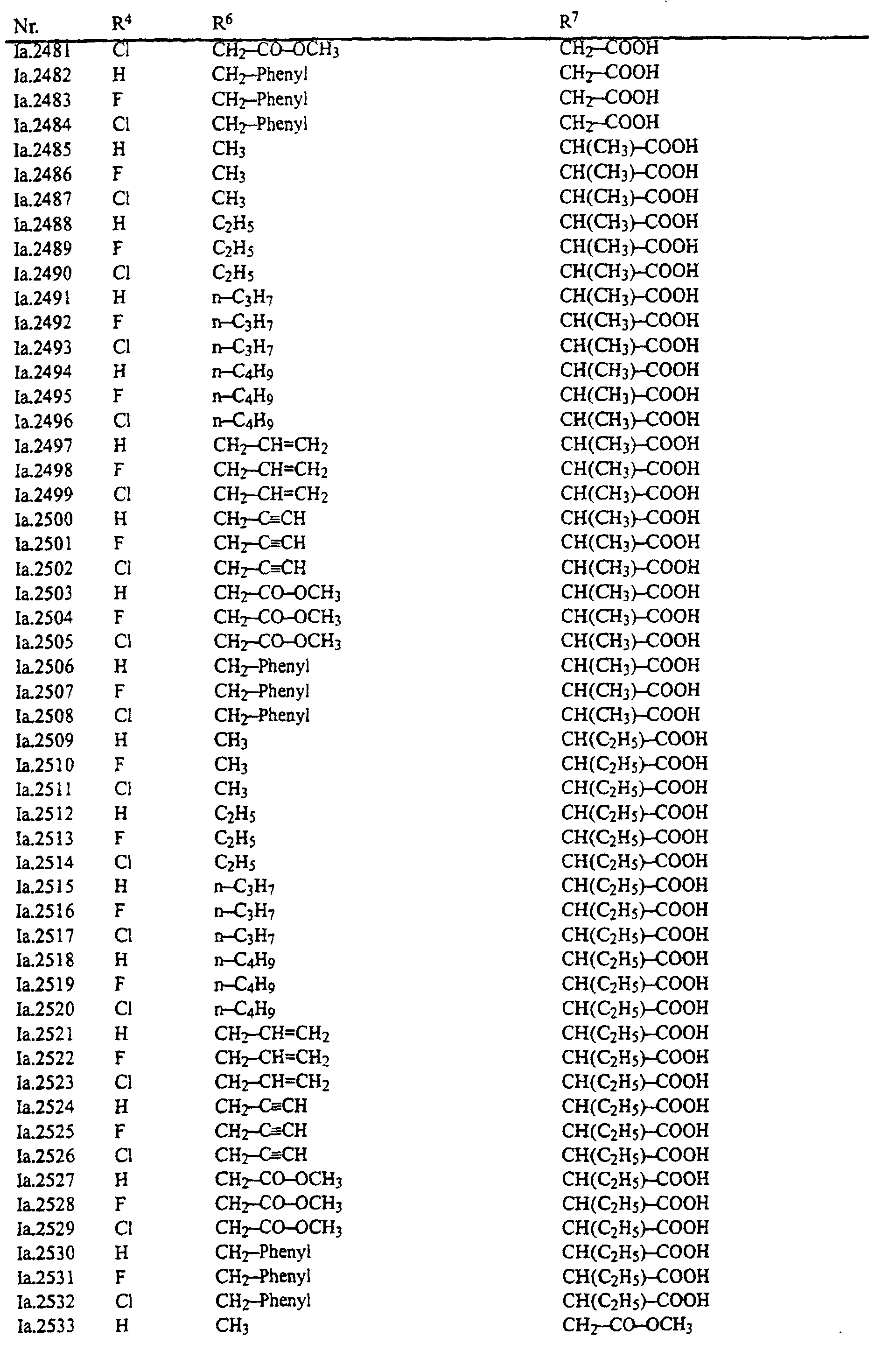

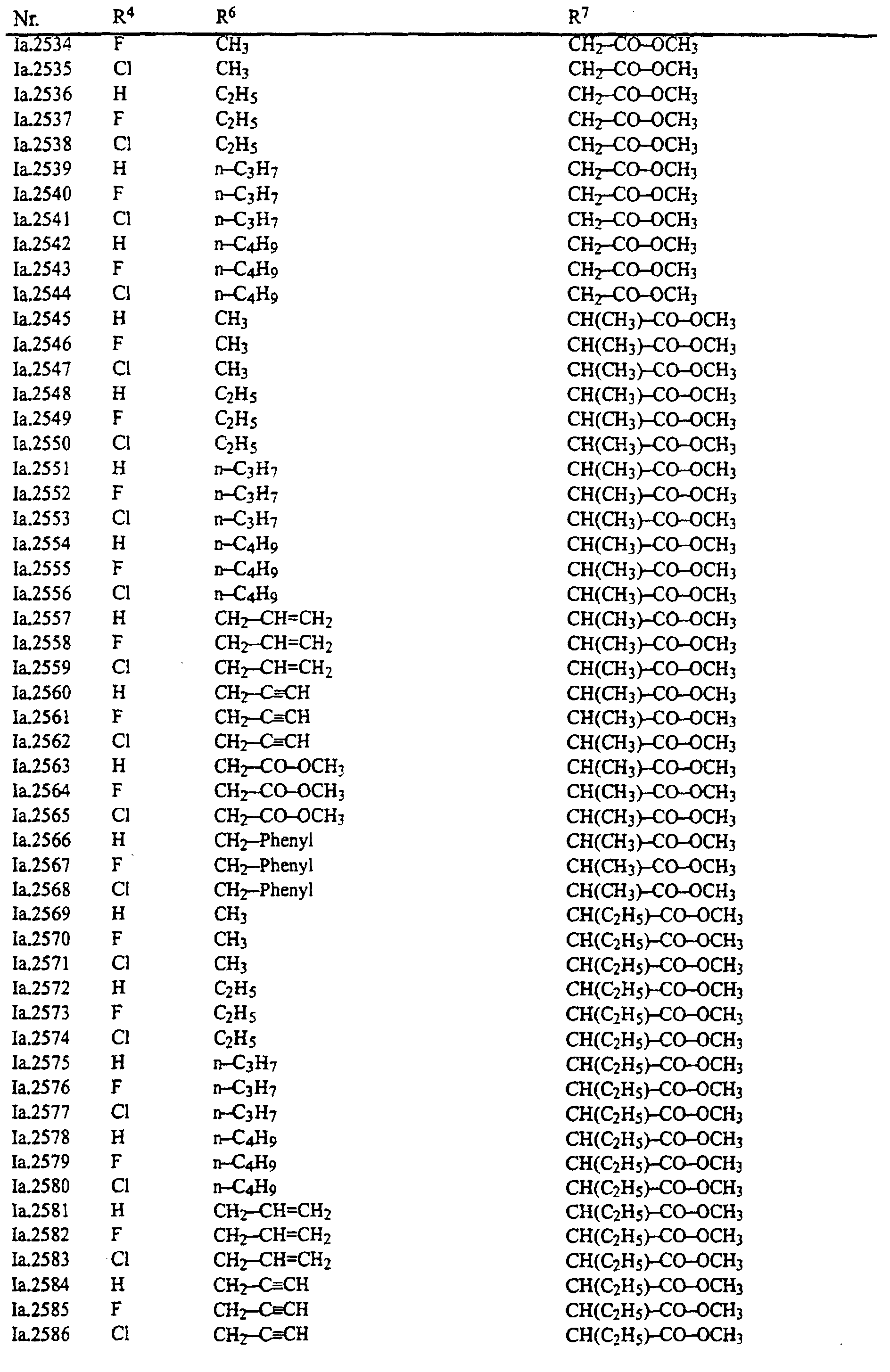

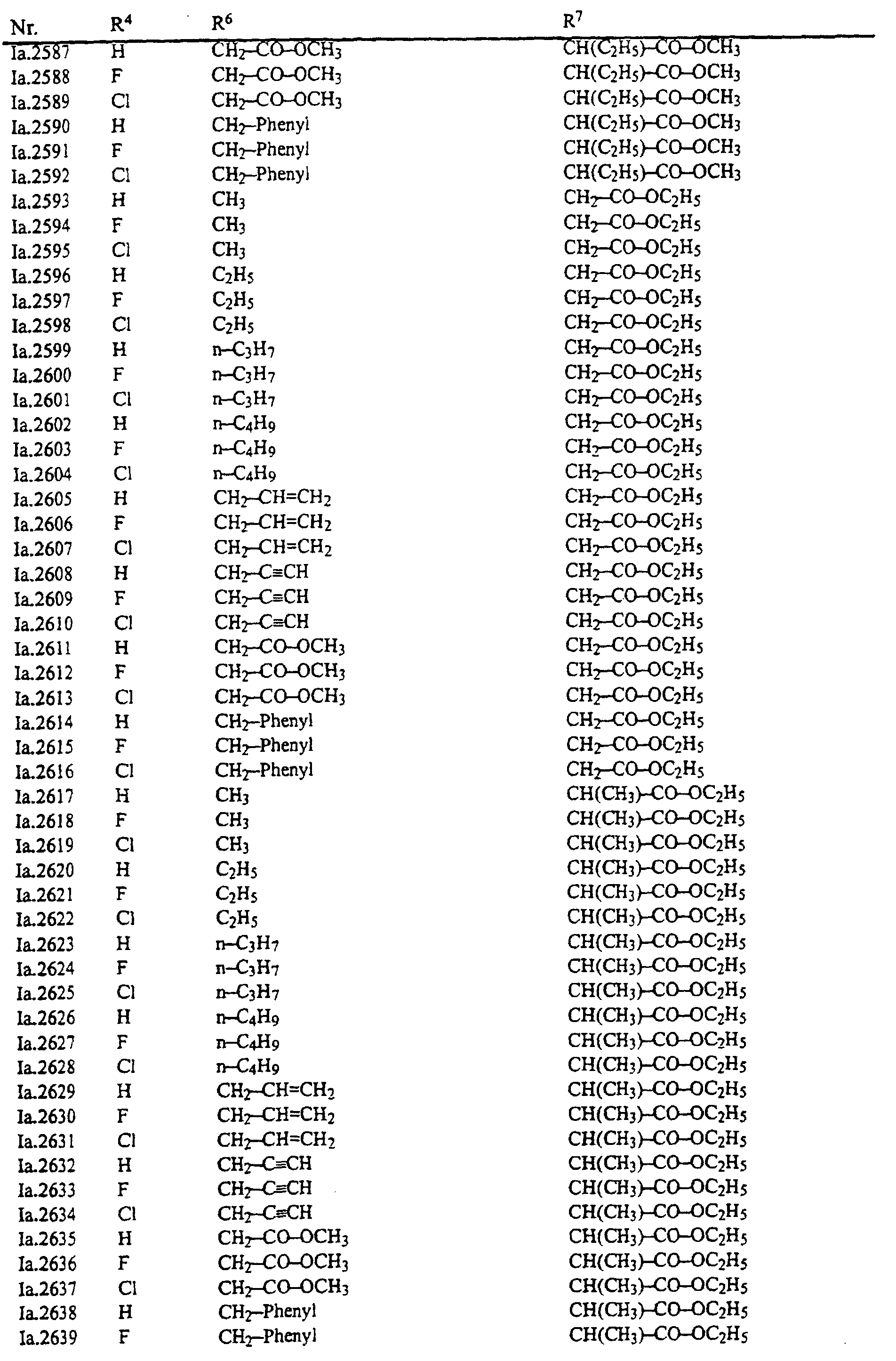

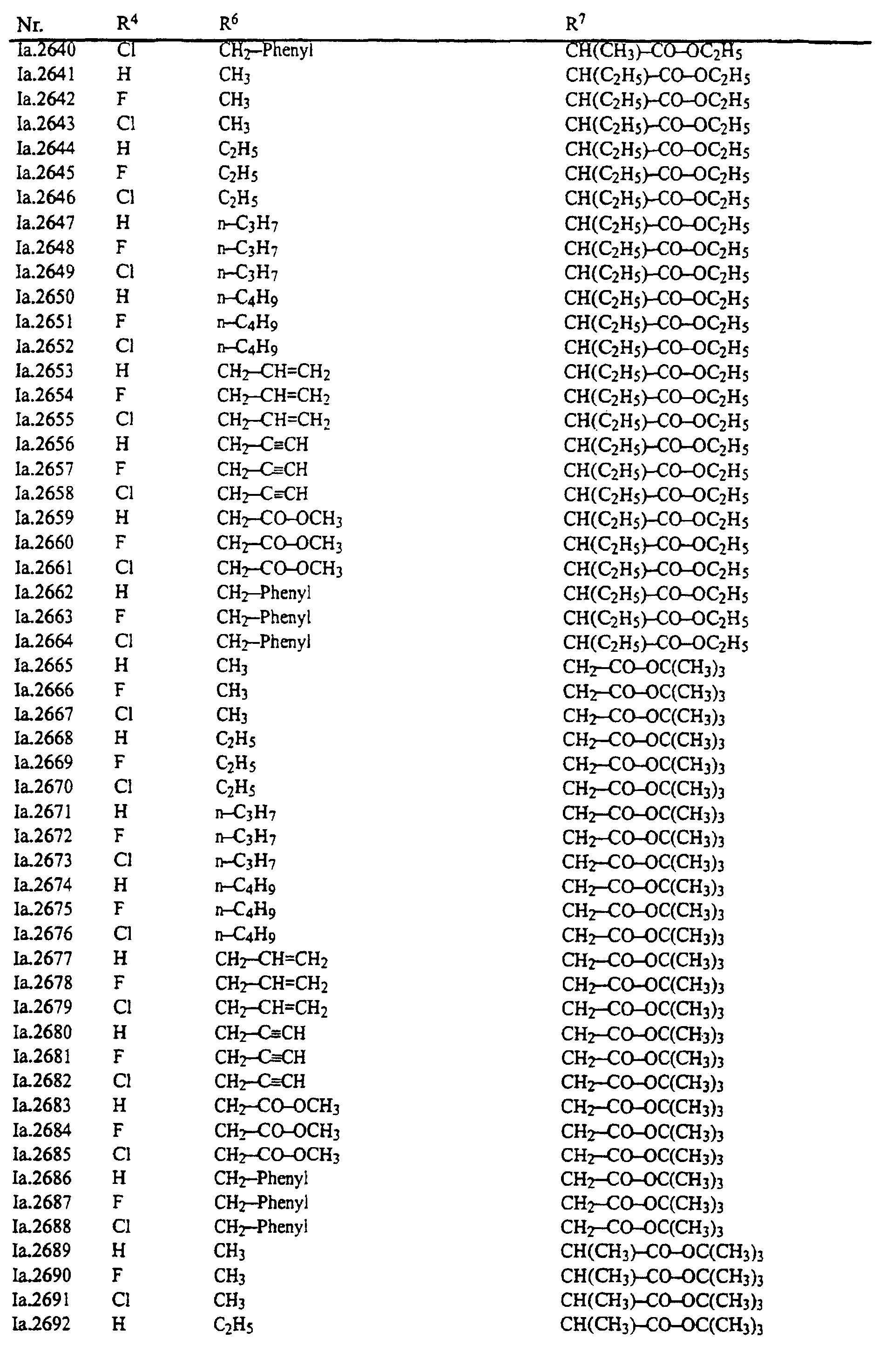

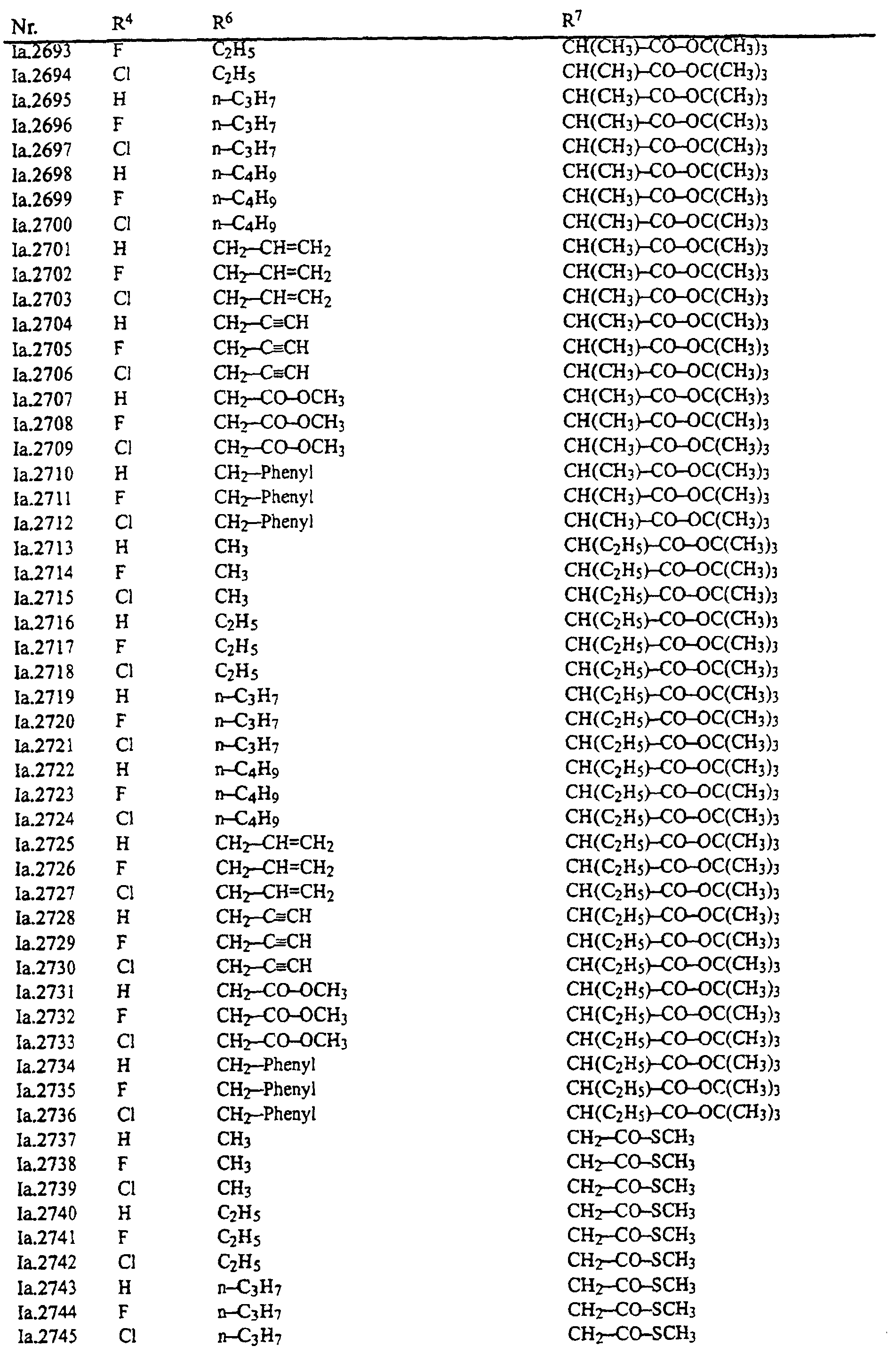

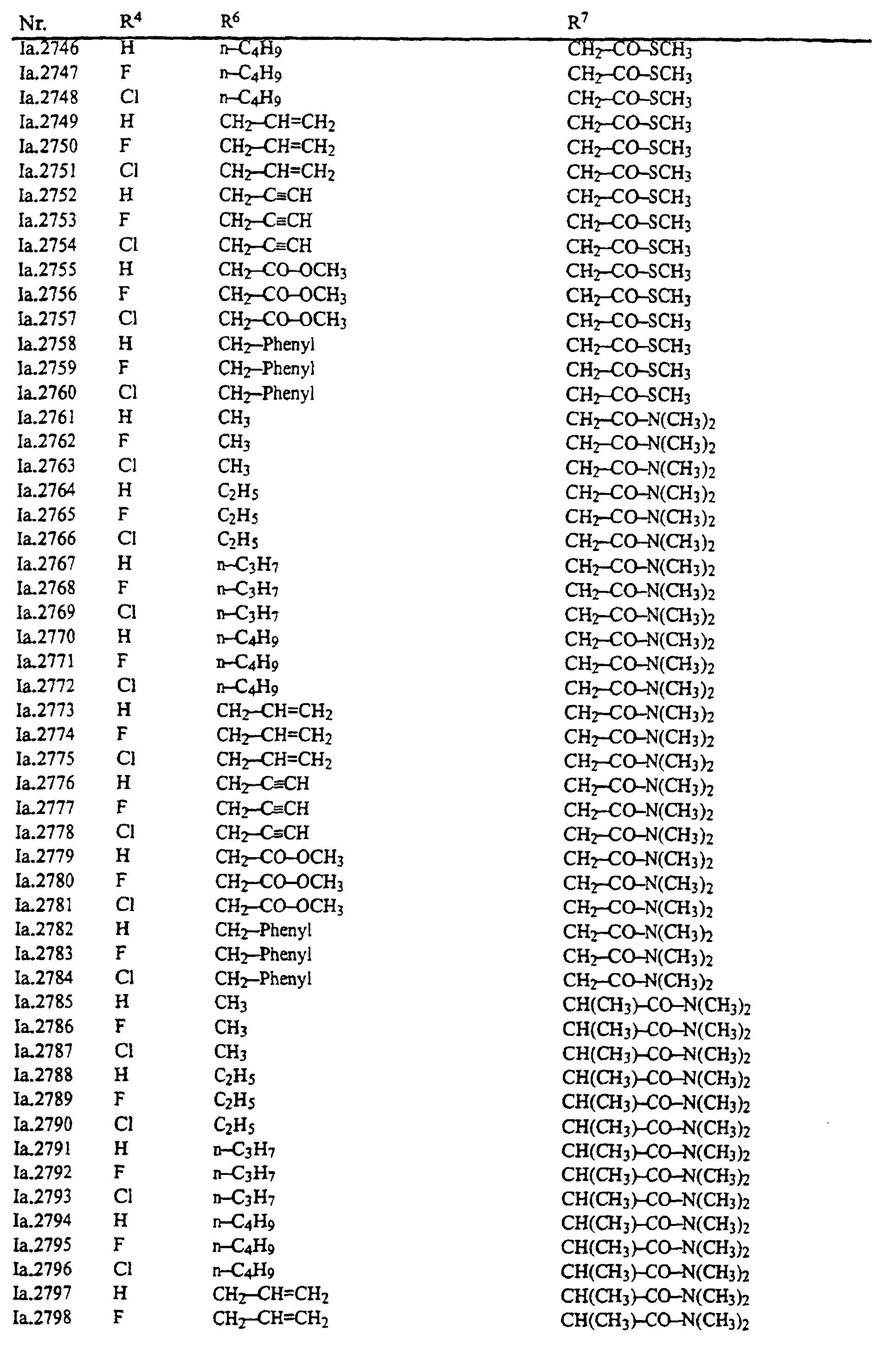

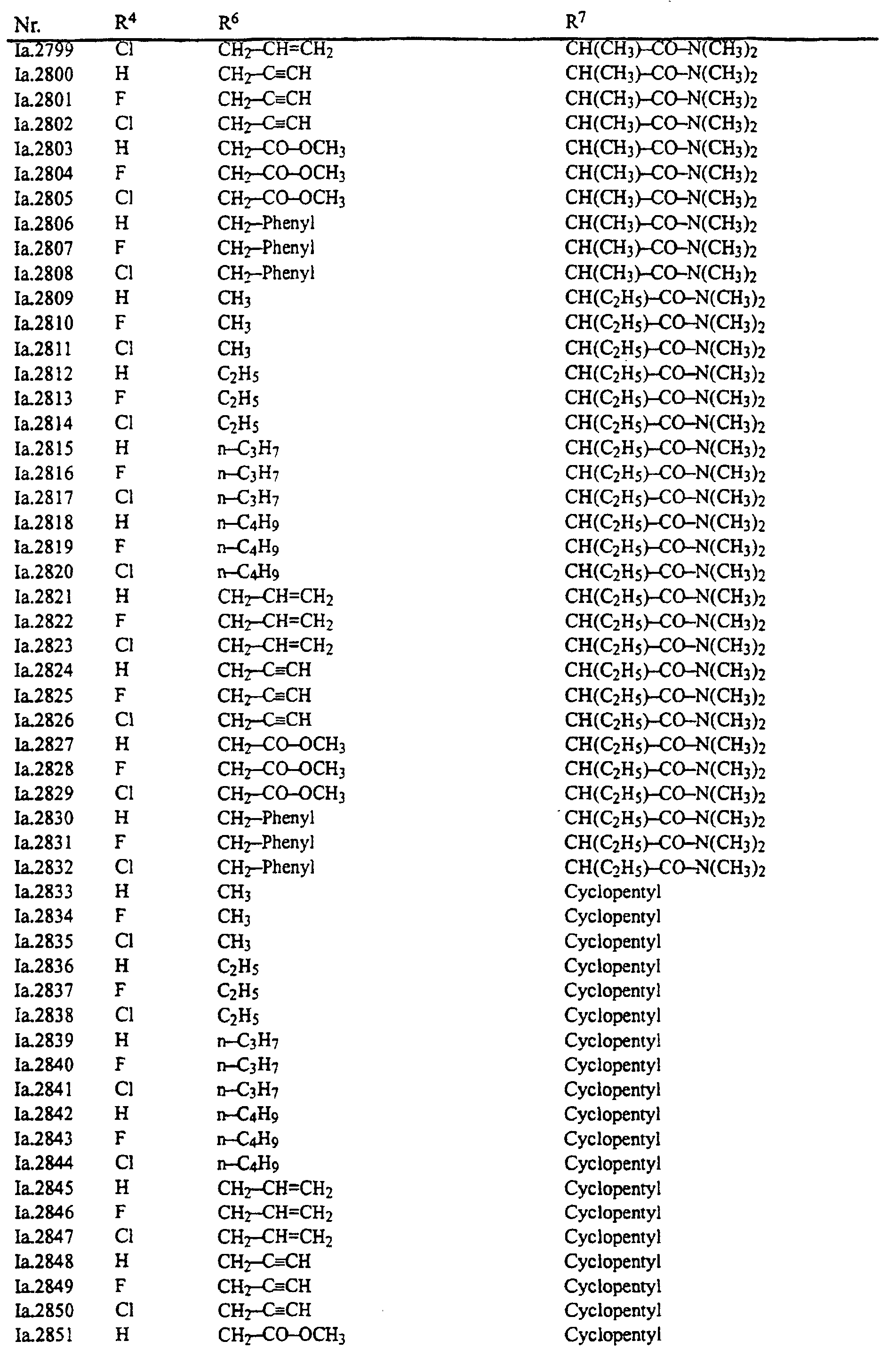

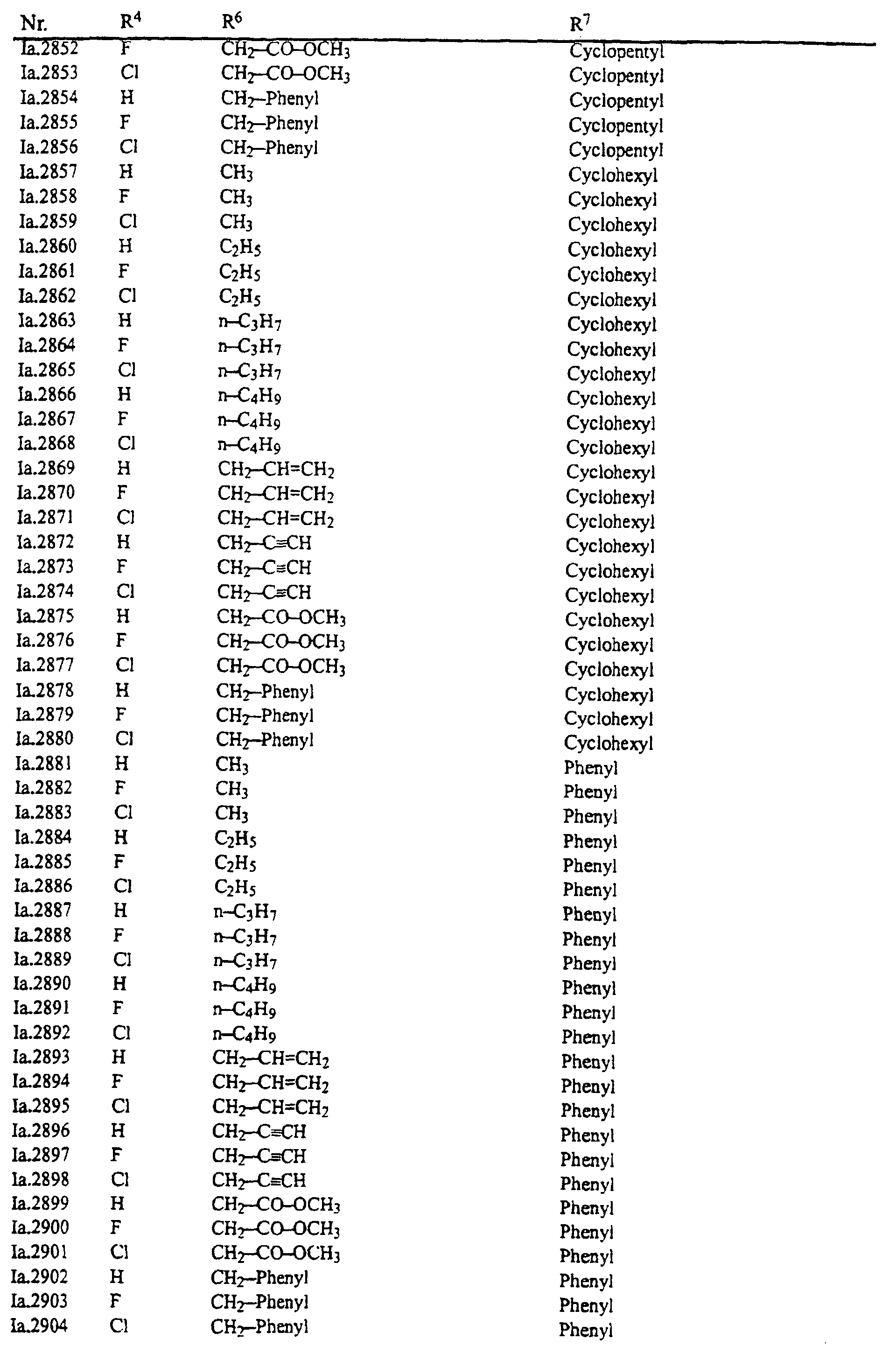

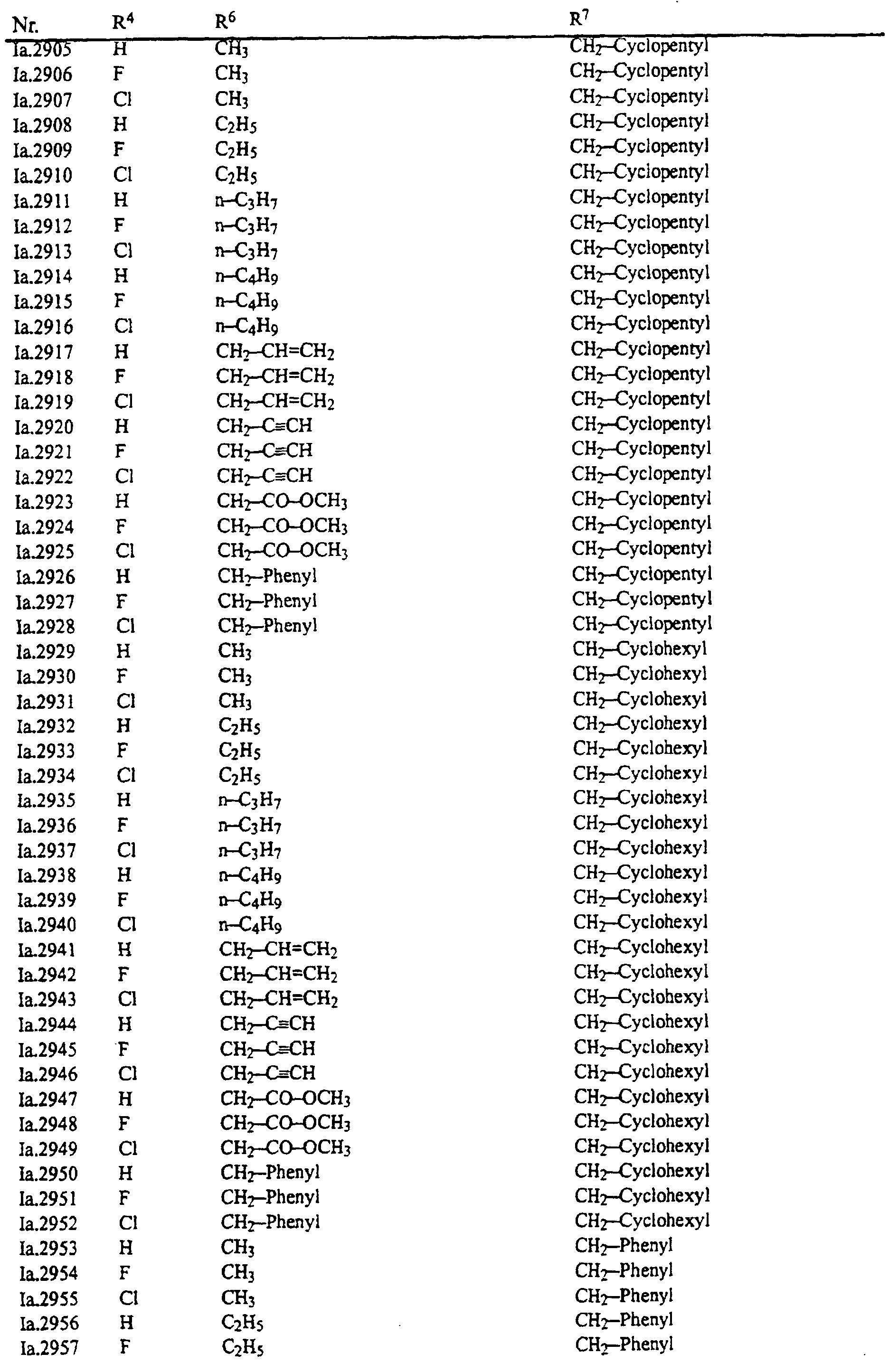

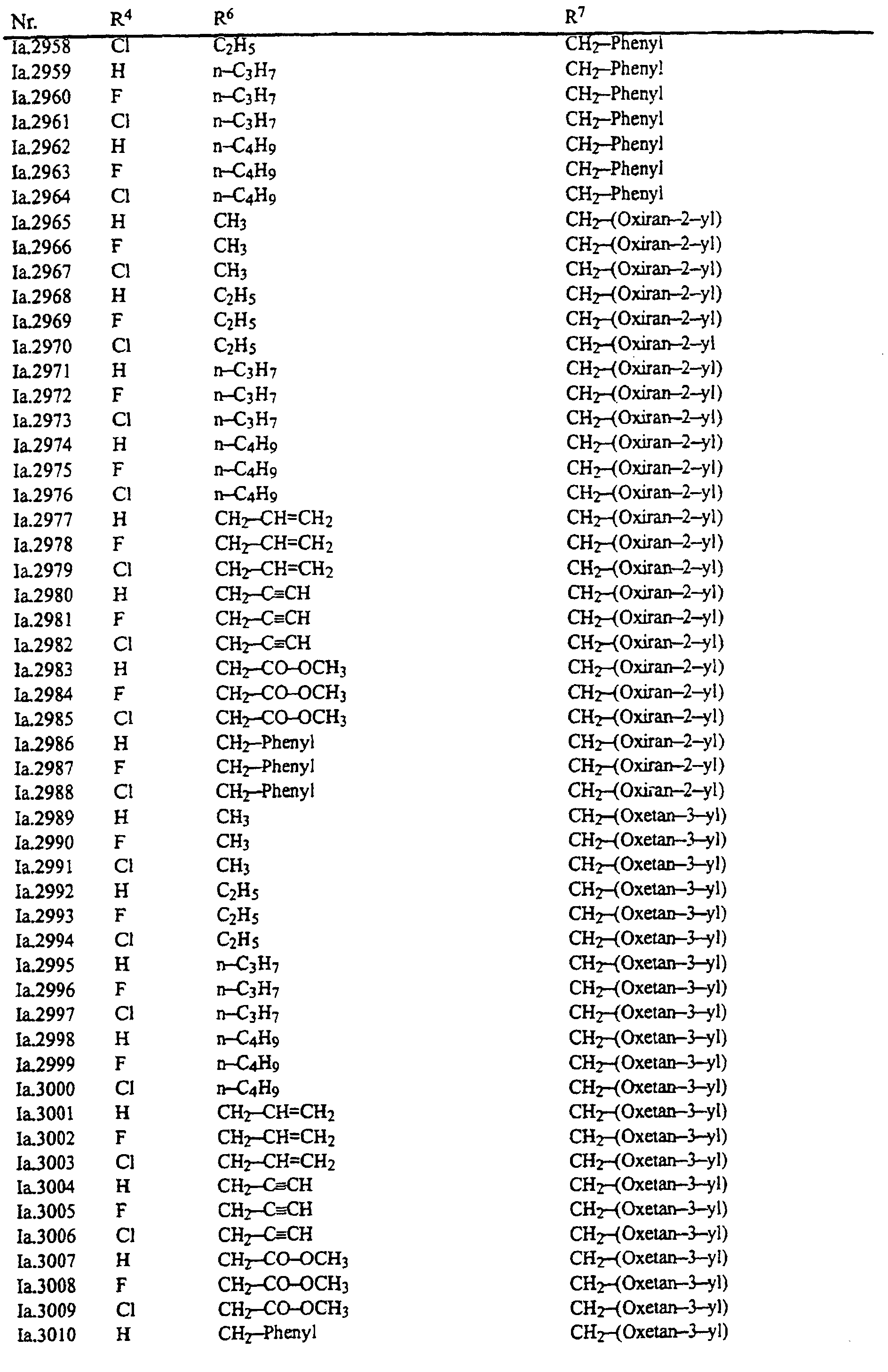

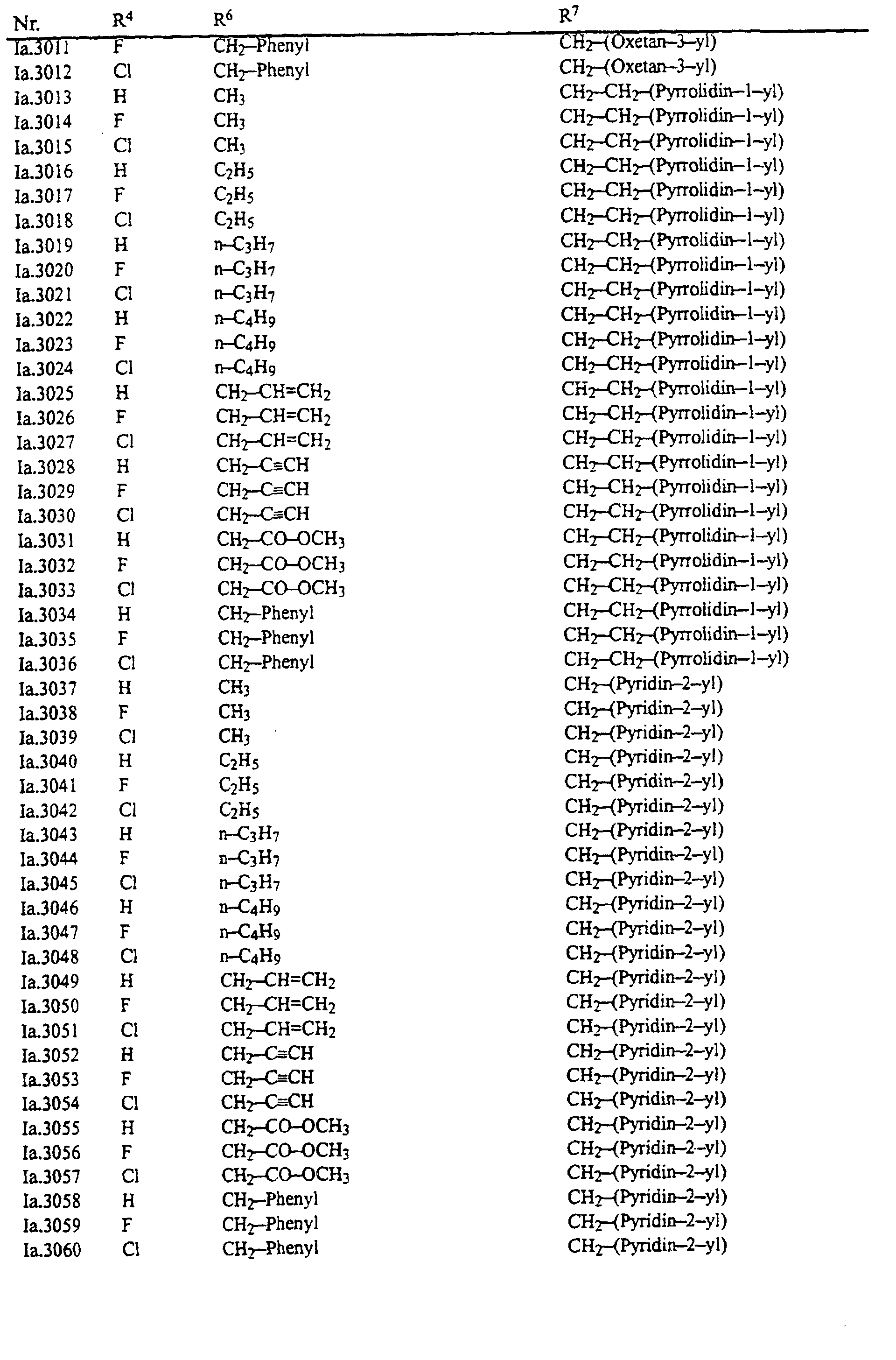

Des weiteren sind die folgenden substituierten 3-Phenylpyrazole der Formeln Ib bis Iu ganz besonders bevorzugt, insbesondere
- die Verbindungen Ib.1 - Ib.3060, die sich von den entsprechenden Verbindungen Ia.1 - Ia.3060 lediglich dadurch unterscheiden, daß R³ für Fluor steht: 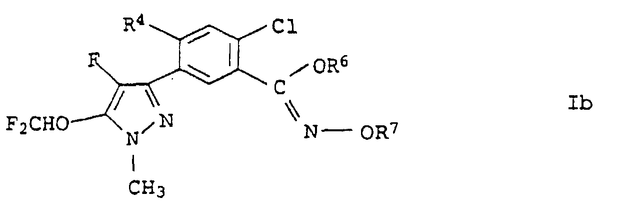
- die Verbindungen Ic.1 - Ic.3060, die sich von den entsprechenden Verbindungen Ia.1 - Ia.3060 lediglich dadurch unterscheiden, daß R³ für Trifluormethyl steht: 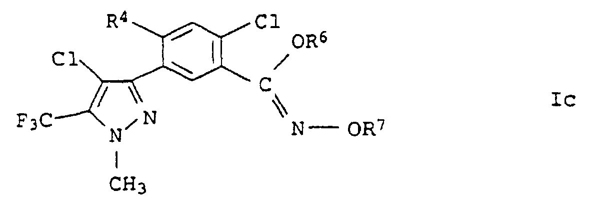
- die Verbindungen Id.1 - Id.3060, die sich von den entsprechenden Verbindungen Ia.1 - Ia.3060 lediglich dadurch unterscheiden, daß R² für Trifluormethyl und R³ für Fluor stehen: 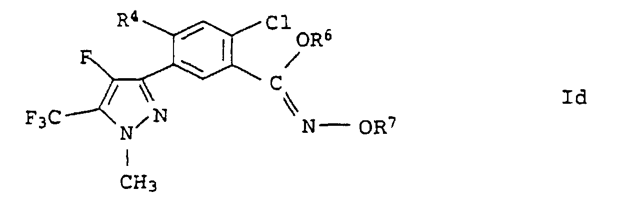
- die Verbindungen Ie.1 - Ie.3060, die sich von den entsprechenden Verbindungen Ia.1 - Ia.3060 lediglich dadurch unterscheiden, daß R² für Methylsulfonyl steht: 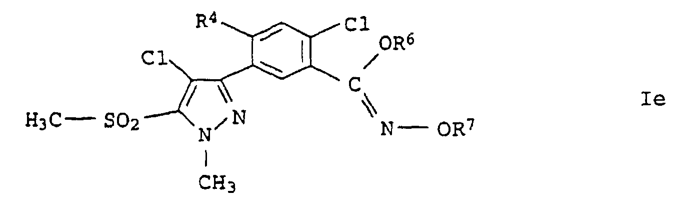
- die Verbindungen If.1 - If.3060, die sich von den entsprechenden Verbindungen Ia.1 - Ia.3060 lediglich dadurch unterscheiden, daß R² für Methylsulfonyl und R³ für Fluor stehen: 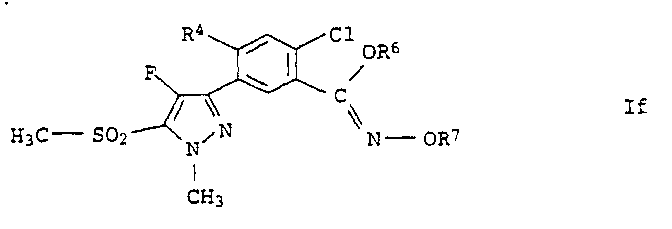
- die Verbindungen Ig.1 - Ig.3060, die sich von den entsprechenden Verbindungen Ia.1 - Ia.3060 lediglich dadurch unterscheiden, daß X für die Gruppe -CH=C(Cl)- steht: 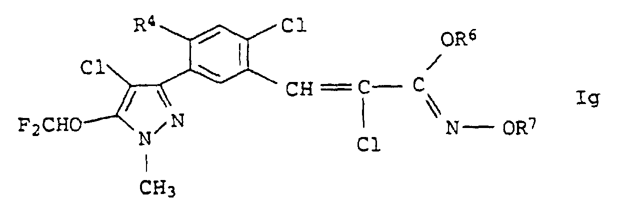
- die Verbindungen Ih.1 - Ih.3060, die sich von den entsprechenden Verbindungen Ia.1 - Ia.3060 lediglich dadurch unterscheiden, daß X für die Gruppe -CH=C(Cl)- und R³ für Fluor stehen: 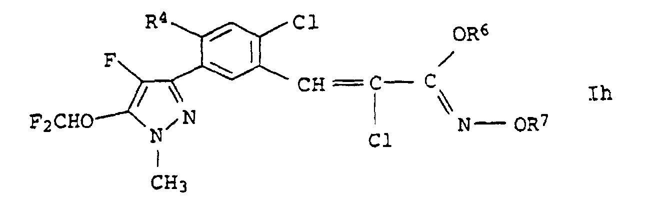
- die Verbindungen Ik.1 - Ik.3060, die sich von den entsprechenden Verbindungen Ia.1 - Ia.3060 lediglich dadurch unterscheiden, daß X für die Gruppe -CH=C(Cl)- und R² für Trifluormethyl stehen: 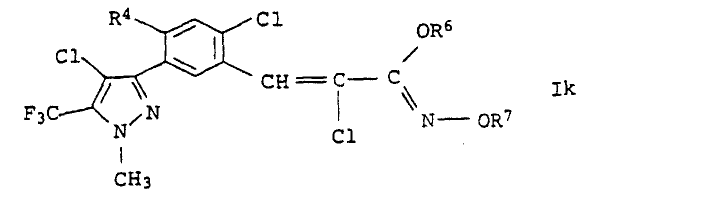
- die Verbindungen Im.1 - Im.3060, die sich von den entsprechenden Verbindungen Ia.1 - Ia.3060 lediglich dadurch unterscheiden, daß X für die Gruppe -CH=C(Cl)-, R² für Trifluormethyl und R³ für Fluor stehen: 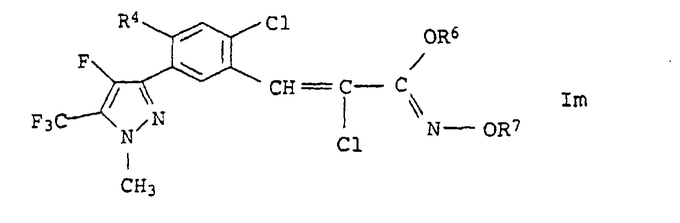
- die Verbindungen In.1 - In.3060, die sich von den entsprechenden Verbindungen Ia.1 - Ia.3060 lediglich dadurch unterscheiden, daß X für die Gruppe -CH=C(Cl)- und R² für Methylsulfonyl stehen: 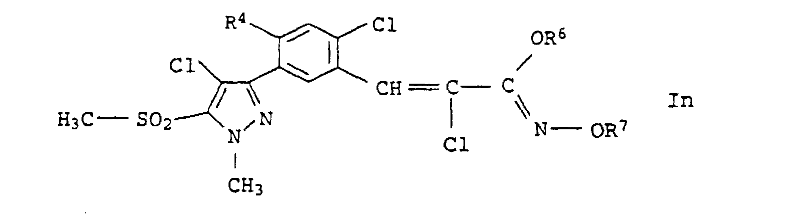
- die Verbindungen Io.1 - Io.3060, die sich von den entsprechenden Verbindungen Ia.1 - Ia.3060 lediglich dadurch unterscheiden, daß X für die Gruppe -CH=C(Cl)-, R² für Methylsulfonyl und R³ für Fluor stehen: 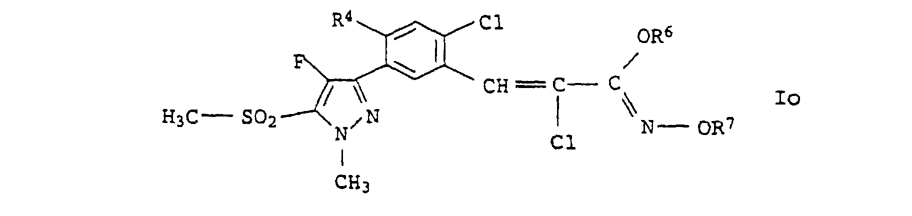
- die Verbindungen Ip.1 - Ip.3060, die sich von den entsprechenden Verbindungen Ia.1 - Ia.3060 lediglich dadurch unterscheiden, daß X für die 1,2-Ethindiyl-Gruppe steht: 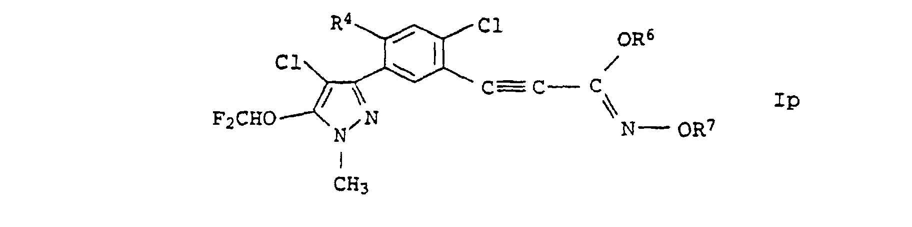
- die Verbindungen Iq.1 - Iq.3060, die sich von den entsprechenden Verbindungen Ia.1 - Ia.3060 lediglich dadurch unterscheiden, daß X für die 1,2-Ethindiyl-Gruppe und R³ für Fluor stehen: 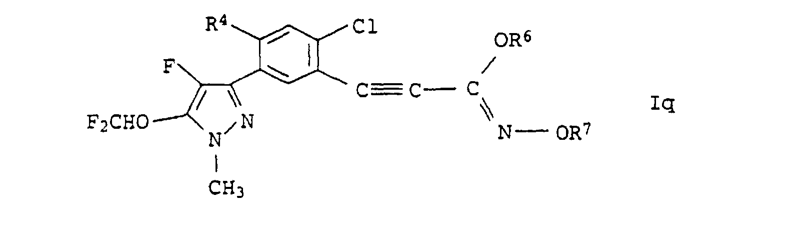
- die Verbindungen Ir.1 - Ir.3060, die sich von den entsprechenden Verbindungen Ia.1 - Ia.3060 lediglich dadurch unterscheiden, daß X für die 1,2-Ethindiyl-Gruppe und R² für Trifluormethyl stehen: 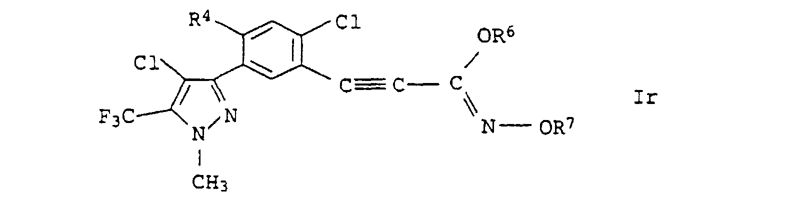
- die Verbindungen Is.1 - Is.3060, die sich von den entsprechenden Verbindungen Ia.1 - Ia.3060 lediglich dadurch unterscheiden, daß X für die 1,2-Ethindiyl-Gruppe, R² für Trifluormethyl und R³ für Fluor stehen: 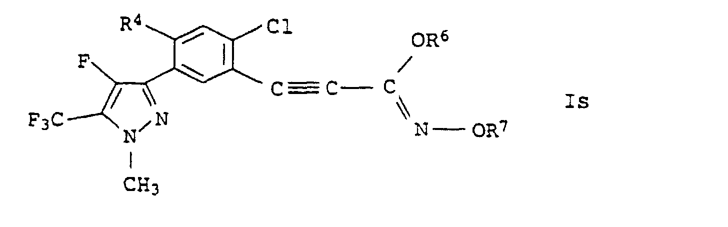
- die Verbindungen It.1 - It.3060, die sich von den entsprechenden Verbindungen Ia.1 - Ia.3060 lediglich dadurch unterscheiden, daß X für die 1,2-Ethindiyl-Gruppe und R² für Methylsulfonyl stehen: 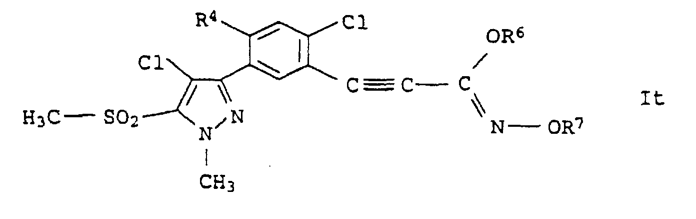
- die Verhindungen Iu.1 - Iu.3060, die sich von den entsprechenden Verbindungen Ia.1 - Ia.3060 lediglich dadurch unterscheiden, daß X für die 1,2-Ethindiyl-Gruppe, R² für Methylsulfonyl und R³ für Fluor stehen:

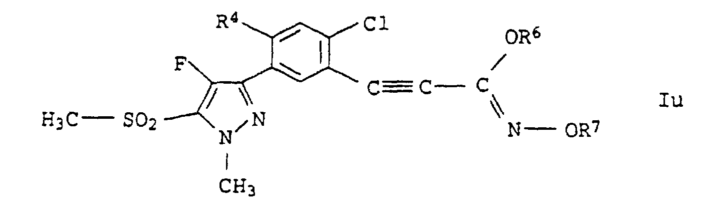

Die substituierten 3-Phenylpyrazole der Formel I sind auf verschiedene Weise erhältlich, insbesondere nach einem der folgenden Verfahren:
A) überführung einer 3-Pyrazolylphenylcarbonsäure II in das entsprechende Alkoxyamid III mit anschließender Alkylierung oder Acylierung auf an sich bekannte Weise {siehe z.B. Houben-Weyl, Methoden der organischen Chemie, Bd. E5, Georg Thieme Verlag, Stuttgart 1985, S. 587 ff., 826 ff. und 1141 ff.}: 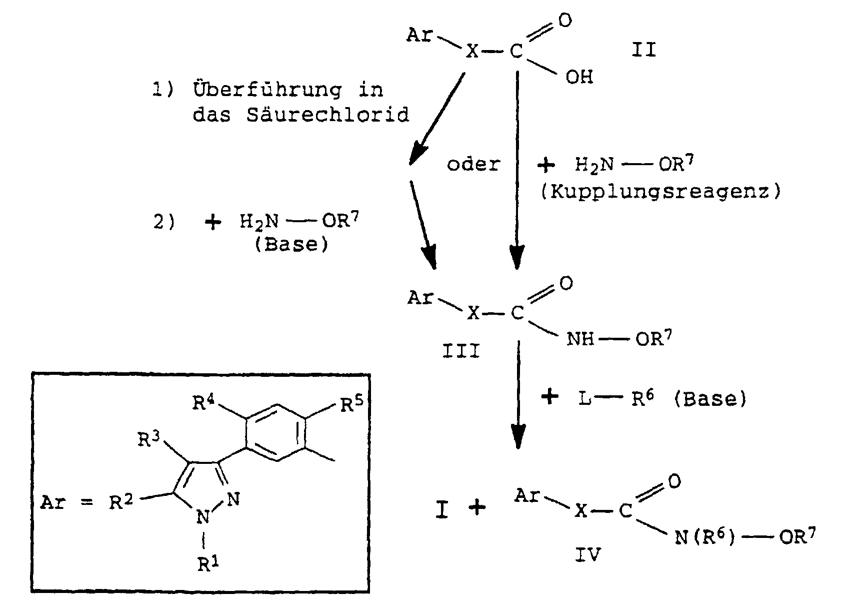 L steht für eine übliche Abgangsgruppe wie Halogen, Methylsulfonyloxy, Trifluormethylsulfonyloxy oder p-Tolylsulfonyloxy.
   Die Überführung von II in III kann auf zweierlei Arten erfolgen:
   Eine Ausführungsform besteht darin, aus II zuerst das entprechende Säurechlorid herzustellen und dieses anschließend in Gegenwart einer Base - z.B. einem Amin wie Triethylamin oder Pyridin, einem Alkalimetallcarbonat wie Kaliumcarbonat, einem Alkalimetallhydrid wie Natriumhydrid oder einem Alkalimetallhydroxid wie Natriumhydroxid und Kaliumhydroxid - mit einem Alkoxyamin H₂N-OR⁷ umzusetzen.

   Geeignete Chlorierungsmittel sind beispielsweise Phosgen, Oxalylchlorid, Phosphoroxychlorid und Thionylchlorid.
   Die Chlorierung erfolgt in der Regel in einem inerten organischen Lösungsmittel, wobei z.B. aliphatische oder aromatische Kohlenwasserstoffe wie n-Hexan und Toluol, Halogenkohlenwasserstoffe wie Dichlormethan, Ether wie Diethylether und Tetrahydrofuran, sowie aprotische Lösungsmittel wie Dimethylformamid und Acetonitril in Betracht kommen.
   Für die Umsetzung des Säurechlorids mit H₂N-OR⁷ sind die genannten Solventien im allgemeinen ebenfalls geeignet, sowie deren Mischungen mit Wasser.
   Eine zweite Ausführungsform besteht darin, II direkt mit dem Alkoxyamin H₂N-OR⁷ in einem der vorstehend genannten organischen Lösungsmitteln umzusetzen. Vorzugsweise arbeitet man hierbei in Gegenwart eines Kupplungsreagenzes wie Carbonyldiimidazol und Dicyclohexylcarbodiimid.
   Das Alkoxyamid III wird anschließend mit einem Alkylierungsoder Acylierungsmittel L-R⁶ alkyliert oder acyliert. Normalerweise arbeitet man dabei in Gegenwart einer Base, z.B. einem Amin wie Triethylamin und Pyridin, einem Alkalimetallcarbonat wie Kaliumcarbonat, einem Alkalimetallhydrid wie Natriumhydrid oder einem Alkalimetallhydroxid wie Natriumhydroxid und Kaliumhydroxid.
   Als Lösungsmittel eignen sich beispielsweise aliphatische oder aromatische Kohlenwasserstoffe wie n-Hexan und Toluol, Halogenkohlenwasserstoffe wie Dichlormethan, Ether wie Diethylether und Tetrahydrofuran, aprotische Lösungsmittel wie Dimethylformamid und Acetonitril sowie Mischungen der genannten Solventien.
   Bei der Reaktion kann als Nebenprodukt das Isomer IV entstehen; es ist auf übliche Weise, z.B. mittels Extraktion, Kristallisation, Destillation oder Chromatographie, abtrennbar.
   Alle genannten Reaktionen werden bei Temperaturen zwischen dem Schmelz- und Siedepunkt des Lösungsmittels, insbesondere bei 0 bis 100°C, durchgeführt. Die Reaktionspartner werden dabei vorzugsweise in ca. äquimolaren Mengen oder in einem Überschuß bis etwa zur zehnfachen molaren Menge, bezogen auf die Menge an Carbonsäure II, dessen Säurechlorid oder an Alkoxyamid III, eingesetzt.
B) Wittig-Reaktion eines 3-Pyrazolylbenzaldehyds V mit einem Ylid VI oder einem Phosphonat VII in Gegenwart einer Base {vgl. z. Houben-Weyl, Methoden der organischen Chemie, Bd. 5/lb, Georg Thieme Verlag, Stuttgart 1972, S. 383 ff.}: 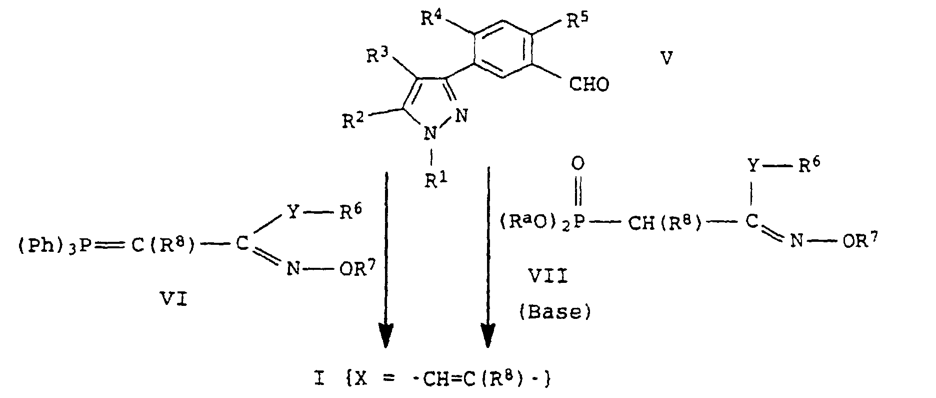
   Ph steht für die bei Wittig-Reagenzien üblichen Substituenten am Phosphor, insbesondere für Phenyl;
   R^{a} steht insbesondere für Niederalkyl wie Methyl und Ethyl.
   Die Umsetzung mit VII erfolgt in der Regel in Gegenwart einer Base, beispielsweise einem Alkalimetallhydrid wie Natriumhydrid, einem Alkalimetallhydroxid wie Natriumhydroxid und Kaliumhydroxid, einem Alkoholat wie Kalium-tert.-butanolat oder Butyllithium.
   Als Lösungsmittel kommen beispielsweise aliphatische oder aromatische Kohlenwasserstoffe wie n-Hexan und Toluol, Halogenkohlenwasserstoffe wie Dichlormethan, Ether wie Diethylether und Tetrahydrofuran, aprotische Lösungsmittel wie Dimethylformamid und Acetonitril in Betracht.
   Die Reaktionstemperatur liegt üblicherweise zwischen dem Schmelz- und Siedepunkt des Reaktionsgemisches, vorzugsweise bei 0 bis 100°C.
   Base und der Reaktionspartner VI oder VII werden dabei allgemein in ca. äquimolaren Mengen oder in einem Überschuß bis etwa zur zehnfachen molaren Menge, bezogen auf die Menge an V, eingesetzt.
   Weitere Möglichkeiten zur Herstellung verschiedener Verbindungen I aus den 3-Pyrazolylbenzaldehyden V schließen die an sich bekannte Aldolkondensation ein {hierfür geeignete Bedingungen sind z.B. Nielsen, Org. React. 16, 1ff. (1968) zu entnehmen}, sowie Kondensationsreaktionen nach Knoevenagel oder Perkin. Geeignete Reaktionsbedingungen sind beispielsweise in Org. React. 15, 204ff. (1967) {nach Knoevenagel} und Johnson, Org. React. 1, 210ff. (1942) {nach Perkin} beschrieben.
C) Diazotierung eines 3-Pyrazolylanilins VIII und Arylierung der erhaltenen Diazonium-Verbindung nach Meerwein {siehe hierzu z.B. Org. Reactions 11, 189-260 (1960) und J. Org. Chem. 42, 2431 (1977)}: 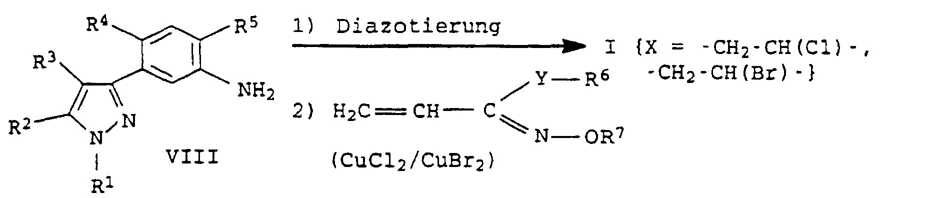
   Das Diazoniumsalz ist vorteilhaft auf an sich bekannte Weise durch Umsetzung eines 3-Pyrazolylanilins VIII in einer wässrigen Säurelösung, z.B. in Salzsäure, Bromwasserstoffsäure oder Schwefelsäure, mit einem Nitrit wie Natriumnitrit und Kaliumnitrit erhältlich.
   Es besteht aber auch die Möglichkeit, wasserfrei, z.B. in chlorwasserstoffhaltigem Eisessig, in absolutem Alkohol, in Dioxan oder Tetrahydrofuran, in Acetonitril oder in Aceton zu arbeiten und hierbei das Anilin VIII mit einem Salpetrigsäureester wie tert.-Butylnitrit und Isopentylnitrit umzusetzen.
   Die anschließende Reaktion des Diazoniumsalzes mit einem Olefin oder Alkin in Gegenwart eines Kupfersalzes, vorzugsweise eines Kupferhalogenids wie Kupfer(I)chlorid, Kupfer(II)chlorid, Kupfer(I)- und Kupfer(II)bromid, nach Meerwein kann z.B. in Wasser, in einem Keton wie Aceton, Diethylketon und Methylethylketon, in einem Nitril wie Acetonitril, in einem Ether wie Dioxan und Tetrahydrofuran oder in einem Alkohol wie Methanol und Ethanol erfolgen.
   Diazotierung und Arylierung können bei Temperaturen von (-30) bis +50°C durchgeführt werden. Üblicherweise werden die Komponenten der Diazotierungsreaktion in etwa stöchiometrischem Verhältnis eingesetzt, jedoch kann ein Überschuß der einen oder anderen Komponente von Vorteil sein.
   In der Regel werden die zu arylierenden Olefine in großem Überschuß eingesetzt; es ist aber auch möglich, nur einen geringen Überschuß, eine stöchiometrische Menge oder einen Unterschuß zu verwenden.
   Das Kupferhalogenid wird üblicherweise in etwa stöchiometrischer Menge, bezogen auf das Diazoniumsalz oder, sofern dieses nicht isoliert wurde, auf das Anilin VIII, eingesetzt, jedoch ist auch ein Über- oder Unterschuß möglich.
   Diazotierung und Arylierung können auch in einer Stufe vorgenommen werden. In diesem Fall diazotiert man in Gegenwart der Olefin- oder Alkinkomponente und des Kupferhalogenids.
D) Basische Eliminierung von Halogenwasserstoff aus 3-Phenylpyrazolen der Formel I, bei denen X -CH₂-CH(Halogen)- oder -CH=C(Halogen)- bedeutet: 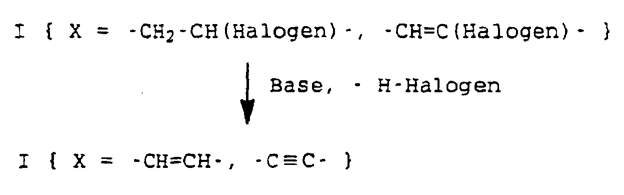
   Als Basen kommen hierfür beispielsweise Amine wie Triethylamin und Pyridin, Alkalimetallhydroxide wie Natriumhydroxid, Alkalimetallcarbonate wie Natrium- und Kaliumcarbonat oder Alkalimetallhydride wie Natriumhydrid in Betracht.
   Als Lösungsmittel eignen sich beispielsweise aliphatische oder aromatische Kohlenwasserstoffe wie n-Hexan und Toluol, Halogenkohlenwasserstoffe wie Dichlormethan, Ether wie Diethylether und Tetrahydrofuran, aprotische Lösungsmittel wie Dimethylformamid und Acetonitril sowie Mischungen der genannten Solventien.
   Gegebenenfalls kann die Reaktion gleichzeitig mit anderen basenkatalysierten Reaktionen - wie oben unter A) bis C) beschrieben - ablaufen, beispielsweise bei der Bildung von I aus III (Verfahren A), bei der Bildung von III aus II über den Säurechlorid-Weg (Verfahren A), bei der Bildung von I aus V (Verfahren B) oder auch bei der Bildung von I aus VIII (Verfahren C).
   Die bei den Verfahren A) bis C) als Vorstufen genannten 3-Pyrazolylphenylcarbonsäuren II, 3-Pyrazolylbenzaldehyde V und 3-Pyrazolylaniline VIII sind im allgemeinen bekannt oder in Analogie zu bekannten Verfahren herstellbar. Diejenigen Ausgangsprodukte II, V und VIII, bei denen R³ Fluor bedeutet, sind z.B. durch Fluorierung der entsprechenden Verbindungen mit R³ = Wasserstoff zugänglich: 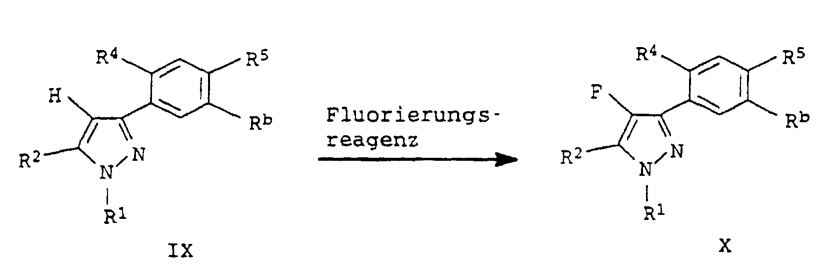 R^{b} steht für Wasserstoff, Halogen oder eine gegenüber Fluorierung inerte Gruppe wie C₁-C₄-Alkyl und (C₁-C₄-Alkoxy)carbonyl.
   Als Fluorierungsreagenzien können beispielsweise Fluor, Schwefeltetrafluorid, N-Fluor-2-pyridon, N-Fluorpyridiniumfluorid, N-Fluor-N-methyltoluolsulfonamid, Stickstofftrifluorid, SELECTFLUOR® {1-Chlormethyl-4-fluor-1,4-diazabicyclo[2.2.2]octanbis(tetrafluoroborat)}, N-Fluorbenzolsulfonimid, 1-Fluorchinuclidiniumtriflat, Acetylhypofluorit oder weitere gebräuchliche Fluorierungsmittel {siehe z.B. D.T. Meshri, ACS Monogr. 187, 25 (1995)} in Betracht.
   Als Lösungsmittel eignen sich insbesondere aliphatische oder aromatische, gegebenenfalls partiell oder vollständig fluorierte oder chlorierte Kohlenwasserstoffe wie n-Hexan, Toluol, Dichlormethan und Fluortrichlormethan, Ether wie Diethylether und Tetrahydrofuran, Nitrile wie Acetonitril sowie Mischungenn der genannten Solventien.
   Die Verfahrensprodukte der Formel X können anschließend nach an sich bekannten Verfahren, wie sie beispielsweise in der WO 97/02251 beschrieben sind, in die Verbindungen der Formel II, V oder VIII (mit jeweils R³ = Fluor) übergeführt werden.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Normalerweise sind die substituierten 3-Phenylpyrazole I nach einem der vorstehend genannten Syntheseverfahren herstellbar. Aus wirtschaftlichen oder verfahrenstechnischen Gründen kann es jedoch zweckmäßiger sein, einige Verbindungen I aus ähnlichen 3-Phenylpyrazolen, die sich jedoch insbesondere in der Bedeutung des Restes R⁶ oder R⁷ unterscheiden, herzustellen, und zwar auf an sich bekannte Weise, z.B. durch Alkylierung, Acetalhydrolyse, Acetalisierung, Amidierung, Esterhydrolyse, Kondensationsreaktion, Oxidation, Peterson-Olefinierung, Reduktion, Veretherung, Veresterung oder Wittig-Reaktion.

Die für die einzelnen Verfahren angegebenen Ausgangsverbindungen sind entweder bekannt oder auf an sich bekannte Weise oder in Analogie zu einem der beschriebenen Verfahren erhältlich.

Die Aufarbeitung der Reaktionsgemische erfolgt in der Regel nach an sich bekannten Methoden, beispielsweise
durch Verdünnen der Reaktionslösung mit Wasser und anschließender Isolierung des Produktes mittels Filtration, Kristallisation oder Lösungsmittelextraktion, oder
durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten 3-Phenylpyrazole I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die weitgehend reinen Isomeren getrennt werden können. Reine optisch aktive Isomere lassen sich vorteilhaft aus entsprechenden optisch aktiven Ausgangsprodukten herstellen.

Landwirtschftlich brauchbare Salze der Verbindungen I können durch Reaktion mit einer Base des entprechenden Kations, vorzugsweise einem Alkalimetallhydroxid oder -hydrid, oder durch Reaktion mit einer Säure des entprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat; Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Ia.689 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ia.1322 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. Ia.1331 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. Ig.1310 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. Ia.1334 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. Ia.2270 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. Ik.1310 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die substituierten 3-Phenylpyrazole I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### 2-Chlor-5-(4-chlor-5-difluormethoxy-1-methyl-1H-pyrazol-3-yl)-4-fluorbenzoesäuremethylester(methoxy)imid (Nr. Ia.2)

Zu einer Lösung von 1 g (2,6 mmol) 2-Chlor-5-(4-chlor-5-difluormethoxy-1-methyl-1H-pyrazol-3-yl)-4-fluorbenzoesäure(methoxy)amid in 50 ml Aceton wurden 0,43 g (3,1 mmol) Kaliumcarbonat und 0,39 g (3,1 mmol) Dimethylsulfat gegeben. Nach 3 Tagen engte man ein. Der Rückstand wurde mit Wasser versetzt, wonach man zweimal mit Ethylacetat extrahierte. Die vereinigten organischen Phasen wurden mit Wasser und gesättigter wäßriger Kochsalzlösung gewaschen, über Magnesiumsulfat getrocknet und schließlich eingeengt. Die Reinigung des Rohproduktes erfolgte mittels Säulenchromatographie an Kieselgel (Eluens: Hexan/Ethylacetat = 2:1).
Ausbeute: 0,2 g.
¹H-NMR (270 MHz, in CDCl₃): δ [ppm] = 3,73 (s,3H), 3,85 (s,3H), 3,90 (s,3H), 6,71 (t,1H), 7,32 (d,1H), 7,68 (d,1H).

### Vorstufe 1.1

### 2-Chlor-5-(4-chlor-5-difluormethoxy-1-methyl-1H-pyrazol-3-yl)-4-fluorbenzoesäurechlorid

Zu einer Lösung von 8 g (22 mmol) 2-Chlor-5-(4-chlor-5-difluormethoxy-1-methyl-1H-pyrazol-3-yl)-4-fluorbenzoesäure in 100 ml Toluol wurden 1 Tropfen Dimethylformamid und 8,6 g (67 mmol) Oxalylchlorid gegeben. Nach 30 Min. Rühren bei Rückflußtemperatur kühlte man ab und engte ein. Ausbeute: quantitativ.

### Vorstufe 1.2

### 2-Chlor-5-(4-chlor-5-difluormethoxy-1-methyl-1H-pyrazol-3-yl)-4-fluorbenzoesäure(methoxy)amid

Zu einer Lösung von 8,4 g (22 mmol) 2-Chlor-5-(4-chlor-5-difluormethoxy-1-methyl-1H-pyrazol-3-yl)-4-fluorbenzoesäurechlorid in 150 ml Toluol wurden 34 g (0,24 mol) Kaliumcarbonat gegeben und anschließend 16 g (48 mmol) einer 25 %igen wäßrigen Methoxyamin-Hydrochlorid-Lösung getropft. Nach 4 Std. Rühren verdünnte man die Reaktionsmischung mit 0,5 l Wasser. Dann wurde die organische Phase abgetrennt, mit gesättigter wäßriger Kochsalzlösung gewaschen, über Magnesiumsulfat getrocknet und schließlich eingeengt. Ausbeute: 8,3 g.
¹H-NMR (270 MHz, in CDCl₃): δ [ppm] = 3,83 (s,3H), 3,90 (s,3H), 6,71 (t,1H) 7,25 (d,1H), 7,79 (d,1H), 9,20 (s,1H).

### Beispiel 2

### 2-Chlor-5-(4-chlor-5-difluormethoxy-1-methyl-1H-pyrazol-3-yl)-4-fluorbenzoesäure(methoxy)imid(essigsäure)anhydrid (Nr. Ia.686)

Zu einer Lösung von 1 g (2,6 mmol) 2-Chlor-5-(4-chlor-5-difluormethoxy-1-methyl-1H-pyrazol-3-yl)-4-fluorbenzoesäure(methoxy)amid in 46 ml Dichlormethan wurden nacheinander 0,26 g (2,6 mmol) Triethylamin und 0,23 g (2,9 mmol) Acetylchlorid getropft. Nach 16 Std. Rühren versetzte man die Reaktionsmischung mit Wasser. Anschließend wurde die organische Phase abgetrennt, über Magnesiumsulfat getrocknet und schließlich eingeengt. Die Reinigung des Rohprodukts erfolgte mittels Kieselgelchromatographie (Eluens: Hexan/Ethylacetat = 1:2). Ausbeute: 0,3 g.

### Beispiel 3

### 2-Chlor-5-(4-chlor-1-methyl-5-trifluormethyl-1H-pyrazol-3-yl)-4-fluorbenzoesäureallylester(methoxy)imid (Nr. Ic.590)

Zu einer Lösung von 1,5 g (3,9 mmol) 2-Chlor-5-(4-chlor-1-methyl-5-trifluormethyl-1H-pyrazol-3-yl)-4-fluorbenzoesäure(methoxy)amid in 50 ml Aceton wurden 0,6 g (4,6 mmol) Kaliumcarbonat und 0,6 g (4,6 mmol) Allylbromid gegeben. Nach 16 Std. Rühren engte man ein. Der Rückstand wurde mit Wasser versetzt. Anschließend extrahierte man das Wertprodukt zweimal mit Ethylacetat. Die vereinigten organischen Phasen wurden mit Wasser und gesättigter wäßriger Kochsalzlösung gewaschen, über Magnesiumsulfat getrocknet und schließlich eingeengt. Die Reinigung des Rohprodukts erfolgte mittels Säulenchromatographie an Kieselgel (Eluens: Hexan/Ethylacetat = 2:1). Ausbeute: 0,3 g.

### Vorstufe 3.1

### 2-Chlor-5-(4-chlor-1-methyl-5-trifluormethyl-1H-pyrazol-3-yl)-4-fluorbenzoesäurechlorid

14 g (39 mmol) 2-Chlor-5-(9-chlor-1-methyl-5-trifluormethyl-1H-pyrazol-3-yl)-4-fluorbenzoesäure wurden analog zu Vorstufe 1.1 umgesetzt. Ausbeute: 13,3 g.

### Vorstufe 3.2

### 2-Chlor-5-(4-chlor-1-methyl-5-trifluormethyl-1H-pyrazol-3-yl)-4-fluorbenzoesäure(methoxy)amid

13,7 g (36 mmol) 2-Chlor-5-(4-chlor-1-methyl-5-trifluormethyl-1H-pyrazol-3-yl)-4-fluorbenzoesäurechlorid wurden analog zu Vorstufe 1.2 umgesetzt. Ausbeute: 14 g.
¹H-NMR (270 MHz, in CDCl₃): δ [ppm] = 3,90 (s,3H), 4,06 (s,3H), 7,27 (d,1H), 7,79 (d,1H), 9,05 (s,1H).

### Beispiel 4

### 2-Chlor-3-(2-chlor-5-(4-chlor-5-difluormethoxy-1-methyl-1Hpyrazol-3-yl)-4-fluorphenyl)acrylsäuremethylester(methoxy)imid (Nr. Ig.2)

Zu einer Lösung von 1 g 2-Chlor-3-(2-chlor-5-(4-chlor-5-difluormethoxy-1-methyl-1H-pyrazol-3-yl)-4-fluorphenyl)acrylsäure(methoxy)amid (2,3 mmol) in 30 ml Aceton wurden 0,4 g (2,7 mmol) gepulvertes Kaliumcarbonat und 0,35 g (2,5 mmol) Methyliodid gegeben. Nach 16 Std. rühren engte man ein. Der Rückstand wurde mit Wasser und Ethylacetat versetzt. Die organische Phase wurde über Magnesiumsulfat getrocknet und dann eingeengt. Die Reinigung des Rückstandes erfolgte mittels Säulenchromatographie an Kieselgel (Eluens: Hexan/Ethylacetat = 4:1). Ausbeute: 0,2 g.

### Vorstufe 4.1

### 2-Chlor-3-(2-chlor-5-(4-chlor-5-difluormethoxy-1-methyl-1H-pyrazol-3-yl)-4-fluorphenyl)acrylsäure-tert.-butylester

Zu einer Lösung von 32 g 2-Chlor-5-(4-chlor-5-difluormethoxy-lmethyl-lH-pyrazol-3-yl)-4-fluorbenzaldehyd (94 mmol) in 200 ml Toluol wurden 72 g (0,14 mol) tert.-Butoxycarbonyl(chlor)-methylen(triphenyl)phosphoran gegeben. Nach 16 Std. Rühren engte man ein. Der Rückstand wurde mit Ethylacetat versetzt. Dann erhitzte man auf Rückflußtemperatur und filtrierte heiß. Der abgetrennte Feststoffanteil wurde mehrmals mit Ethylacetat nachgewaschen. Das Filtrat versetzte man mit etwa dem doppelten Volumen Hexan, wonach abermals filtriert wurde. Schließlich engte man das Filtrat ein. Die Reinigung des verbliebenen Rohprodukts von Triphenylphosphinoxid-Resten erfolgte durch Lösen in Hexan/Ethylacetat (4:1) und anschließender Filtration über ein Kieselgelbett. Ausbeute: 44 g.
¹H-NMR (250 MHz, in CDCl₃): δ [ppm] = 1,58 (s,9H), 3,84 (s,3H), 6,73 (t,1H), 7,32 (d,1H), 8,03 (s,1H), 8,24 (d,1H).

### Vorstufe 4.2

### 2-Chlor-3-(2-chlor-5-(4-chlor-5-difluormethoxy-1-methyl-1Hpyrazol-3-yl)-4-fluorphenyl)acrylsäure

Zu einer Lösung von 44 g 2-Chlor-3-(2-chlor-5-(4-chlor-5-difluormethoxy-1-methyl-1H-pyrazol-3-yl)-4-fluorphenyl)acrylsäure-tert.-butylester (93 mmol) in 200 ml Dichlormethan wurden 200 ml Trifluoressigsäure gegeben. Nach 16 Std. Rühren engte man ein. Der Rückstand wurde aus Hexan/Essigester (2:1) umkristallisiert. Ausbeute: 23 g; durch Konzentrieren der Mutterlauge weitere 4 g.
¹H-NMR (270 MHz, in CDCl₃): δ [ppm] = 3,87 (s,3H), 6,72 (t,1H), 7,34 (d,1H) 8,26 (s,1H), 8,31 (d,1H).

### Vorstufe 4.3

### 2-Chlor-3-(2-chlor-5-(4-chlor-5-difluormethoxy-1-methyl-1H-pyrazol-3-yl)-4-fluorphenyl)acrylsäurechlorid

Zu einer Lösung von 27 g 2-Chlor-3-(2-chlor-5-(4-chlor-5-difluormethoxy-1-methyl-1H-pyrazol-3-yl)-4-fluorphenyl)acrylsäure (64 mmol) in 200 ml Toluol wurden 3 Tropfen Dimethylformamid und 33 g (0,26 mol) Oxalylchlorid gegeben. Anschließend rührte man bei 90°C, bis die starke Gasentwicklung aufhörte (nach ca. 2,5 Std.). Dann wurde die Reaktionsmischung eingeengt.
Ausbeute: 26 g Rohprodukt, das ohne Reinigung weiter umgesetzt wurde.

### Vorstufe 4.4

### 2-Chlor-3-(2-chlor-5-(4-chlor-5-difluormethoxy-1-methyl-1H-pyrazol-3-yl)-4-fluorphenyl)acrylsäure(methoxy)amid

Zu einer Lösung von 4,5 g (10 mmol) 2-Chlor-3-(2-chlor-5-(4-chlor-5-difluormethoxy-1-methyl-1H-pyrazol-3-yl)-4-fluorphenyl)acrylsäurechlorid in 25 ml Toluol wurden 25 ml Wasser, 14,4 g (0,1 mol) Kaliumcarbonat und 1,7 g (20 mmol) Methoxyamin-Hydrochlorid gegeben. Nach 16 Stunden Rühren bei ca. 20°C erwärmte man noch für 2 Std. auf 40°C. Anschließend wurde die Reaktionsmischung mit 200 ml Toluol und 200 ml Wasser verdünnt. Dann filtrierte man den Feststoffanteil ab. Die organische Phase wurde über Magnesiumsulfat getrocknet und schließlich eingeengt.
Ausbeute: 3,1 g.
¹H-NMR (270 MHz, in CDCl₃): δ [ppm] = 3,85 (s,3H), 3,90 (s,3H), 6,73 (t,1H), 7,33 (d,1H), 8,04 (d,1H), 8,20 (s,1H), 9,33 (s,1H).

### Beispiel 5

### 2-Chlor-3-[2-chlor-5-(4-chlor-1-methyl-5-trifluormethyl-1H-pyrazol-3-yl)-4-fluorphenyl]acrylsäure(methoxycarbonylmethyl)ester(methoxy)imid (Nr. Ik.1310)

Zu einer Lösung von 1,2 g (2,6 mmol) 2-Chlor-3-(2-chlor-5-(4-chlor-1-methyl-5-trifluormethyl-1H-pyrazol-3-yl)-4-fluorphenyl)acrylsäure(methoxy)amid in 50 ml Aceton wurden 0,4 g (3 mmol) Kaliumcarbonat und 0,4 g (2,9 mmol) Bromessigsäuremethylester gegeben. Nach 16 Std. Rühren engte man ein. Der Rückstand wurde mit Wasser versetzt. Dann extrahierte man das Produkt mit Diethylether. Die organische Phase wurde über Magnesiumsulfat getrocknet und schließlich eingeengt. Die Reinigung des Rohproduktes erfolgte mittels Kieselgelchromatographie (Eluens: Hexan/Ethylacetat = 4:1). Ausbeute: 0,6 g.

### Vorstufe 5.1

### 2-Chlor-3-[2-chlor-5-(4-chlor-1-methyl-5-trifluormethyl-1H-pyrazol-3-yl)-4-fluorphenyl]acrylsäure-tert.-butylester

6 g (18 mmol) 2-Chlor-5-(4-chlor-1-methyl-5-trifluormethyl-1H-pyrazol-3-yl)-4-fluorbenzaldehyd wurden analog zu Vorstufe 4.1 umgesetzt. Ausbeute: 6,4 g.
¹H-NMR (270 MHz, in CDCl₃): δ [ppm] = 1,58 (s,9H), 4,07 (s,3H), 7,33 (d,1H), 8,03 (s,1H), 8,20 (d,1H).

### Vorstufe 5.2

### 2-Chlor-3-[2-chlor-5-(4-chlor-1-methyl-5-trifluormethyl-1H-pyrazol-3-yl)-4-fluorphenyl]acrylsäure

4,8 g (10 mmol) 2-Chlor-3-[2-chlor-5-(4-chlor-1-methyl-5-trifluormethyl-1H-pyrazol-3-yl)-4-fluorphenyl]acrylsäure wurden analog zu Vorstufe 4.2 umgesetzt. Ausbeute: 2,4 g.
Smp.: 195-196°C.

### Vorstufe 5.3

### 2-Chlor-3-[2-chlor-5-(4-chlor-1-methyl-5-trifluormethyl-1H-pyrazol-3-yl)-4-fluorphenyl]acrylsäurechlorid

1,7 g (4 mmol) 2-Chlor-3-[2-chlor-5-(4-chlor-1-methyl-5-trifluormethyl-1H-pyrazol-3-yl)-4-fluorphenyl]acrylsäure wurden analog zu Vorstufe 4.3 umgesetzt. Ausbeute: 1,7 g.

### Vorstufe 5.4

### 2-Chlor-3-[2-chlor-5-(9-chlor-1-methyl-5-trifluormethyl-1H-pyrazol-3-yl)-4-fluorphenyl]acrylsäure(methoxy)amid

1,7 g (3,9 mmol) 2-Chlor-3-[2-chlor-5-(4-chlor-1-methyl-5-trifluormethyl-1H-pyrazol-3-yl)-4-fluorphenyl]acrylsäurechlorid wurden analog zu Vorstufe 4.4 umgesetzt. Ausbeute: 1,2 g.
¹H-NMR (270 MHz, in CDCl₃): δ [ppm] = 3,90 (s,3H), 4,08 (s,3H), 7,34 (d,1H), 8,00 (d,1H), 8,21 (s,1H), 9,36 (s,1H).

### Beispiel 6

### 2-Chlor-5-(4-chlor-1-methyl-5-methylsulfonyl-1H-pyrazol-3-yl)-4-fluorbenzoesäuremethylester(methoxy)amid (Nr. Ie.2)

Auf die in Beispiel 1 beschriebene Weise erhielt man unter Verwendung von 1,4 g (3,5 mmol) 2-Chlor-5-(4-chlor-1-methyl-5-methylsulfonyl-1H-pyrazol-3-yl)-4-fluorbenzoesäure(methoxy)amid, 0,58 g (4,2 mmol) Kaliumcarbonat und 0,53 g (4,2 mmol) Dimethylsulfat 0,2 g Wertprodukt.

### Vorstufe 6.1

### 2-Chlor-5-(4-chlor-1-methyl-5-methylsulfonyl-1H-pyrazol-3-yl)-4-fluorbenzoesäurechlorid

5 g (14 mmol) 2-Chlor-5-(4-chlor-1-methyl-5-methylsulfonyl-1H-pyrazol-3-yl)-4-fluorbenzoesäure wurden analog zu Vorstufe 1.1 umgesetzt. Ausbeute: quantitativ.

### Vorstufe 6.2

### 2-Chlor-5-(4-chlor-1-methyl-5-methylsulfonyl-1H-pyrazol-3-yl)-4-fluorbenzoesäure(methoxy)amid

5 g (13 mmol) 2-Chlor-5-(4-chlor-1-methyl-5-methylsulfonyl-1H-pyrazol-3-yl)-4-fluorbenzoesäurechlorid wurden analog zu Vorstufe 1.2 umgesetzt. Ausbeute: 2,8 g.
¹H-NMR (250 MHz, in CDCl₃): δ [ppm] = 3,30 (s,3H), 3,91 (s,3H), 4,15 (s,3H), 7,29 (d,1H), 7,82 (d,1H), 8,95 (s,1H).

### Beispiel 7

### 3-[2-Chlor-5-(4-chlor-5-difluormethoxy-1-methyl-1H-pyrazol-3-yl)-4-fluorphenyl]propiolsäure-[2-(methoxyimino)ethyl)ester(methoxy)imid (Nr. Ip.1094)

Zu einer Lösung von 1 g (2,3 mmol) 2-Chlor-3-[2-chlor-5-(4-chlor-5-difluormethoxy-1-methyl-1H-pyrazol-3-yl)-4-fluorphenyl]acrylsäure(methoxy)amid in 50 ml Aceton wurden 0,4 g (2,7 mmol) Kaliumcarbonat und 0,27 g (2,5 mmol) (O-Methyl)-chloracetaldehydoxim gegeben. Nach 3 Tagen Rühren engte man ein. Der Rückstand wurde mit 50 ml Dimethylformamid versetzt. Anschließend erhitzte man noch 4 Stunden auf 100°C, bevor das Gemisch erneut eingeengt wurde. Danach versetzte man den Rückstand mit 20 ml Ethylacetat. Die erhaltene organische Phase wurde noch mit Wasser gewaschen, über Magnesiumsulfat getrocknet und schließlich eingeengt. Die Reinigung des Rohprodukts erfolgte mittels Kieselgelchromatographie (Eluens: Hexan/Ethylacetat = 2:1). Ausbeute: 0,2 g.

### Beispiel 8

### 2,4-Dichlor-5-(5-difluormethoxy-4-fluor-1-methyl-1H-pyrazol-3-yl)benzoesäuremethylester(methoxy)imid (Nr. Ib.3)

Auf die in Beispiel 1 beschriebene Weise erhielt man unter Verwendung von 0,5 g (1,3 mmol) 2,4-Dichlor-5-(5-difluormethoxy-4-fluor-1-methyl-1H-pyrazol-3-yl)benzoesäure(methoxy)amid, 0,21 g (1,6 mmol) Kaliumcarbonat und 0,20 g (1,6 mmol) Dimethylsulfat 0,17 g des gewünschten Wertprodukts.

### Vorstufe 8.1

### 3-(2,4-Dichlor-5-methylphenyl)-5-difluormethoxy-4-fluor-1-methyl-1H-pyrazol

Zu einer Lösung von 40,2 g (131 mmol) 3-(2,4-Dichlor-5-methylphenyl)-5-difluormethoxy-1-methyl-1H-pyrazol in 0,5 1 Acetonitril wurden 46,3 g (131 mmol) Selectfluor™ gegeben. Nach 12 Stunden Rühren bei Raumtemperatur erhitzte man noch eine Stunde lang auf Rückflußtemperatur. Danach wurden nach und nach dreimal je 6,9 g (20 mmol) Selectfluor™ in die Reaktionsmischung gegeben und jeweils für eine Stunde auf Rückflußtemperatur erhitzt. Anschließend versetzte man das erhaltene Gemisch mit 0,5 l Methyl-tert.butylether. Die organische Phase wurde noch mit Wasser, gesättigter wässriger Natriumhydrogencarbonat-Lösung und wieder Wasser gewaschen, dann über Magnesiumsulfat getrocknet und schließlich eingeengt. Die Reinigung des Rohprodukts erfolgte mittels Kieselgelchromatographie (Eluens: Hexan/Ethylacetat = 9:1). Ausbeute: 20,3 g.
¹H-NMR (400 MHz, in CDCl₃): δ [ppm] = 2,36 (s,3H), 3,78 (s,3H), 6,66 (t,1H), 7,37 (s,1H), 7,46 (s,1H).

### Vorstufe 8.2

### 2,4-Dichlor-5-(5-difluormethoxy-4-fluor-1-methyl-1H-pyrazol-3-yl)benzaldehyd

Eine Lösung von 20,3 g (62 mmol) 3-(2,4-Dichlor-5-methylphenyl)-5-difluormethoxy-4-fluor-1-methyl-1H-pyrazol und 11,1 g (62 mmol) N-Bromsuccinimid in 0,27 l Tetrachlormethan wurde unter Bestrahlung mit einer UV-Lampe für zwei Stunden auf Rückflußtemperatur erhitzt. Zweimal gab man dann nach und nach 2,2 g (12 mmol) N-Bromsuccinimid in das Reaktionsgemisch, wonach jedesmal für eine Stunde unter weiterer Bestrahlung auf Rückflußtemperatur erhitzt wurde. Dann ließ man die Mischung abkühlen und filtrierte den Feststoffanteil ab. Das Filtrat wurde eingeengt.

Zu der erhaltenen Substanz, gelöst in 500 ml Acetonitril, gab man anschließend 14,2 g (120 mmol) N-Methylmorpholin-N-oxid. Nach 3 Stunden Rühren wurde eingeengt. Den Rückstand versetzte man mit 500 ml Wasser und 500 ml Dichlormethan. Zur Isolierung des Produkts wurde die organische Phase abgetrennt, noch dreimal mit Wasser gewaschen, dann über Magnesiumsulfat getrocknet und schließlich eingeengt. Die Reinigung des Rückstandes erfolgte mittels MPLC an Kieselgel (Eluens: Cyclohexan/Ethylacetat = 9:1).
Ausbeute: 7,4 g.
¹H-NMR (270 MHz, in CDCl₃): δ [ppm] = 3,81 (s,3H), 6,67 (t,1H), 7,63 (s,1H), 8,09 (s,1H), 10,43 (s,1H).

### Vorstufe 8.3

### 2,4-Dichlor-5-(5-difluormethoxy-4-fluor-1-methyl-1H-pyrazol-3-yl)benzoesäure

Zu einer Lösung von 7,4 g (22 mmol) 2,4-Dichlor-5-(5-difluormethoxy-4-fluor-1-methyl-1H-pyrazol-3-yl)benzaldehyd in 200 ml Acetonitril wurden zuerst eine Lösung von 0,85 g (5,5 mmol) Natriumdihydrogenphosphatdihydrat in 10 ml Wasser und dann 2,7 ml einer 30 %igen wäßrigen Wasserstoffperoxid-Lösung getropft. Danach tropfte man innerhalb von 20 Minuten eine Lösung von 2,9 g (33 mmol) Natriumchlorit in 25 ml Wasser in die Reaktionsmischung. Nach einer Stunde Rühren wurde das erhaltene Gemisch mit verd. Salzsäure angesäuert. Schließlich filtrierte man das entstandene feste Wertprodukt ab und trocknete es. Ausbeute: 6,3 g.
¹H-NMR (250 MHz, in CDCl₃): δ [ppm] = 3,83 (s,3H), 6,67 (t,1H), 7,64 (s,1H), 8,21 (s,1H).

### Vorstufe 8.4

### 2,4-Dichlor-5-(5-difluormethoxy-4-fluor-1-methyl-1H-pyrazol-3-yl)benzoylchlorid

5,8 g (16 mmol) 2,4-Dichlor-5-(5-difluormethoxy-4-fluor-1-methyl-1H-pyrazol-3-yl)benzoesäure und 10,4 g (82 mmol) Oxalylchlorid wurden auf die in Vorstufe 1.1 beschriebene Weise umgesetzt.
Ausbeute: 5,2 g.

### Vorstufe 8.5

### 2,4-Dichlor-5-(5-difluormethoxy-4-fluor-1-methyl-1H-pyrazol-3-yl)benzoesäure(methoxy)amid

Auf die in Vorstufe 1.2 beschriebene Weise erhielt man unter Verwendung von 5,2 g (14 mmol) 2,4-Dichlor-5-(5-difluormethoxy-4-fluor-1-methyl-1H-pyrazol-3-yl)benzoylchlorid, 19,2 g (0,14 mol) Kaliumcarbonat und 9,2 g (28 mmol) einer 25 %igen wäßrigen Methoxyaminhydrochlorid-Lösung ein Rohprodukt, dessen Reinigung mittels Kieselgelchromatographie (Eluens: Hexan/Ethylacetat = 4:1) 1,5 g des gewünschten Wertprodukts ergab.
¹H-NMR (250 MHz, in CDCl₃): δ [ppm] = 3,79 (s,3H), 3,90 (s,3H), 6,66 (t,1H), 7,55 (s,1H), 7,74 (s,1H), 9,00 (s,1H).

In der folgenden Tabelle 2 sind neben den vorstehend beschriebenen Wirkstoffen noch weitere substituierte 3-Phenylpyrazole I aufgeführt, die auf analoge Weise hergestellt wurden oder herstellbar sind:

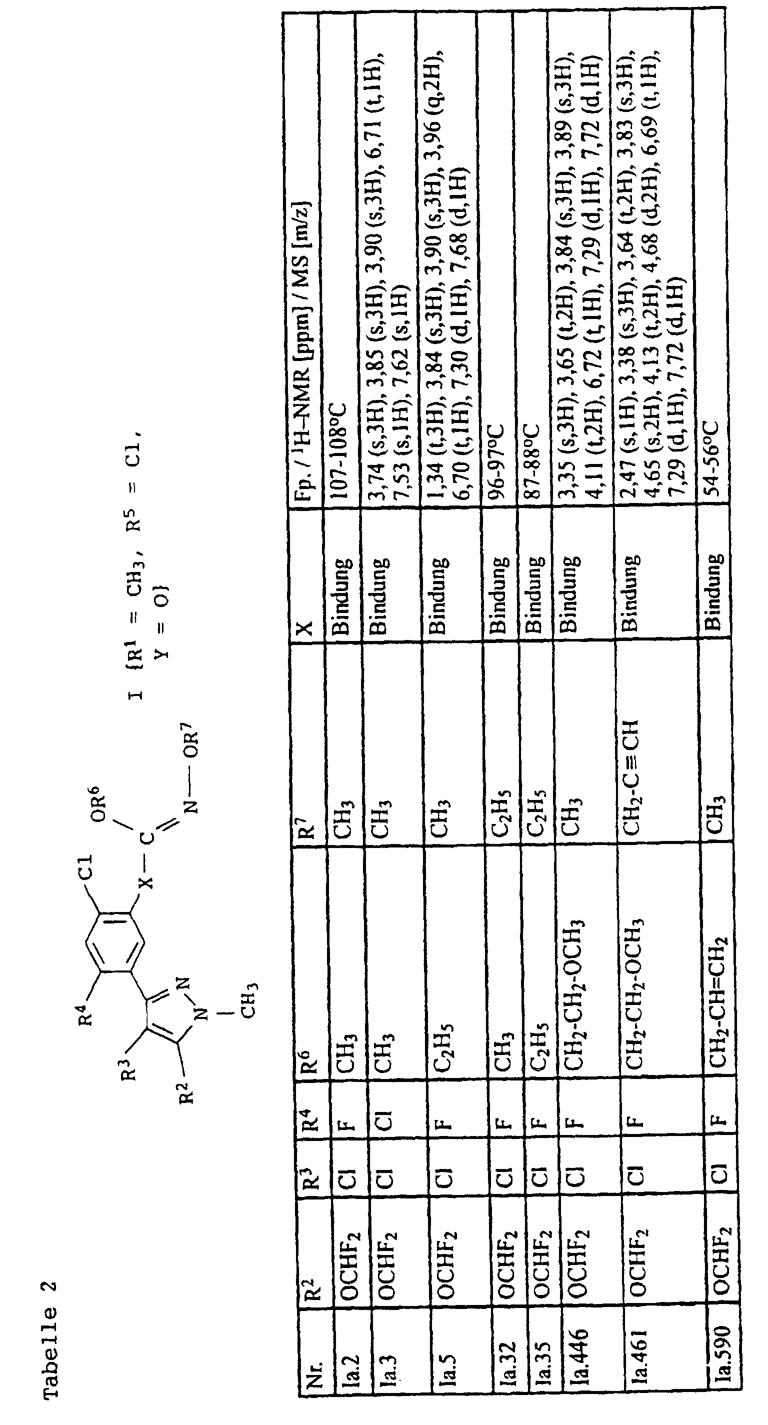

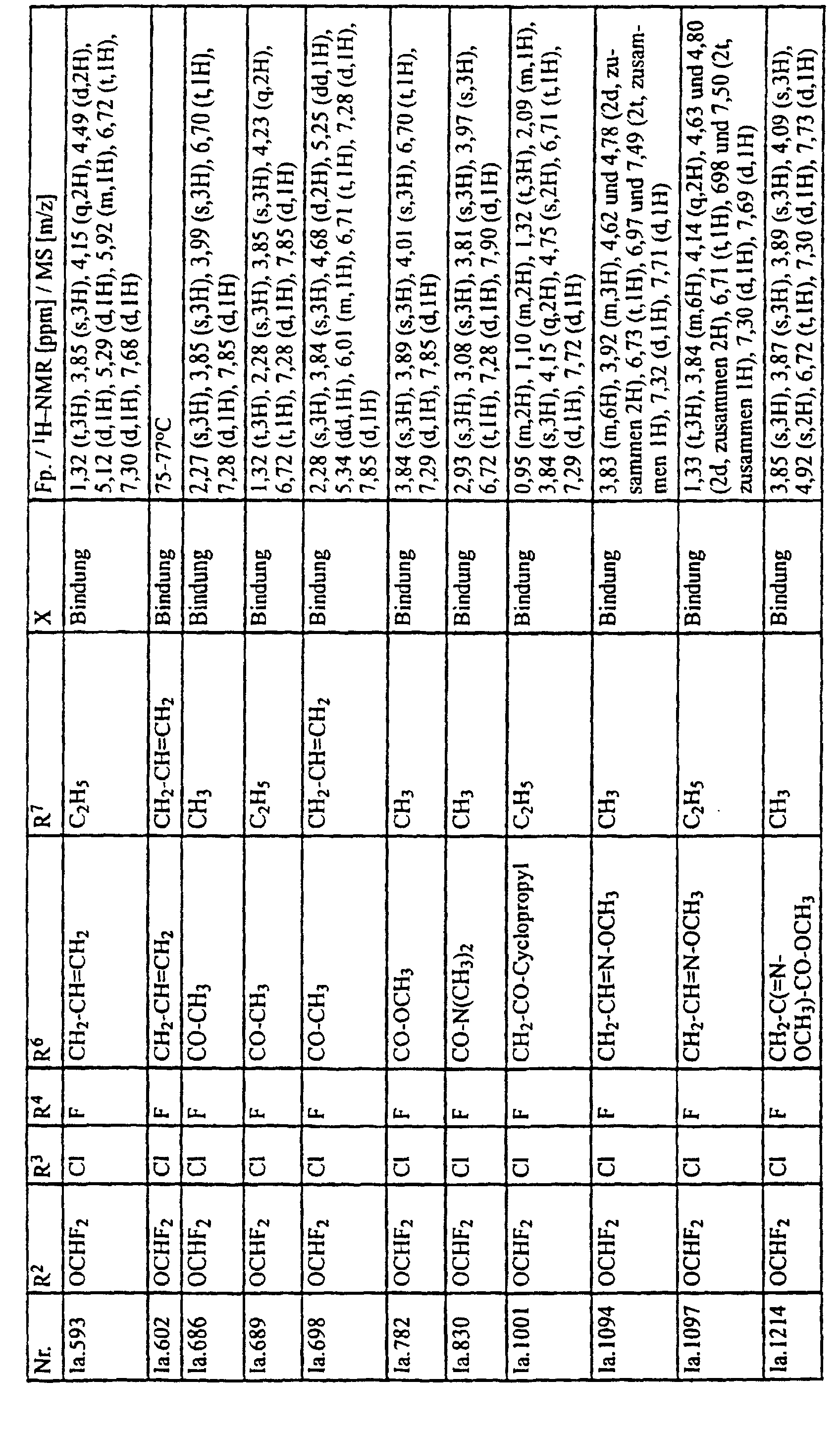

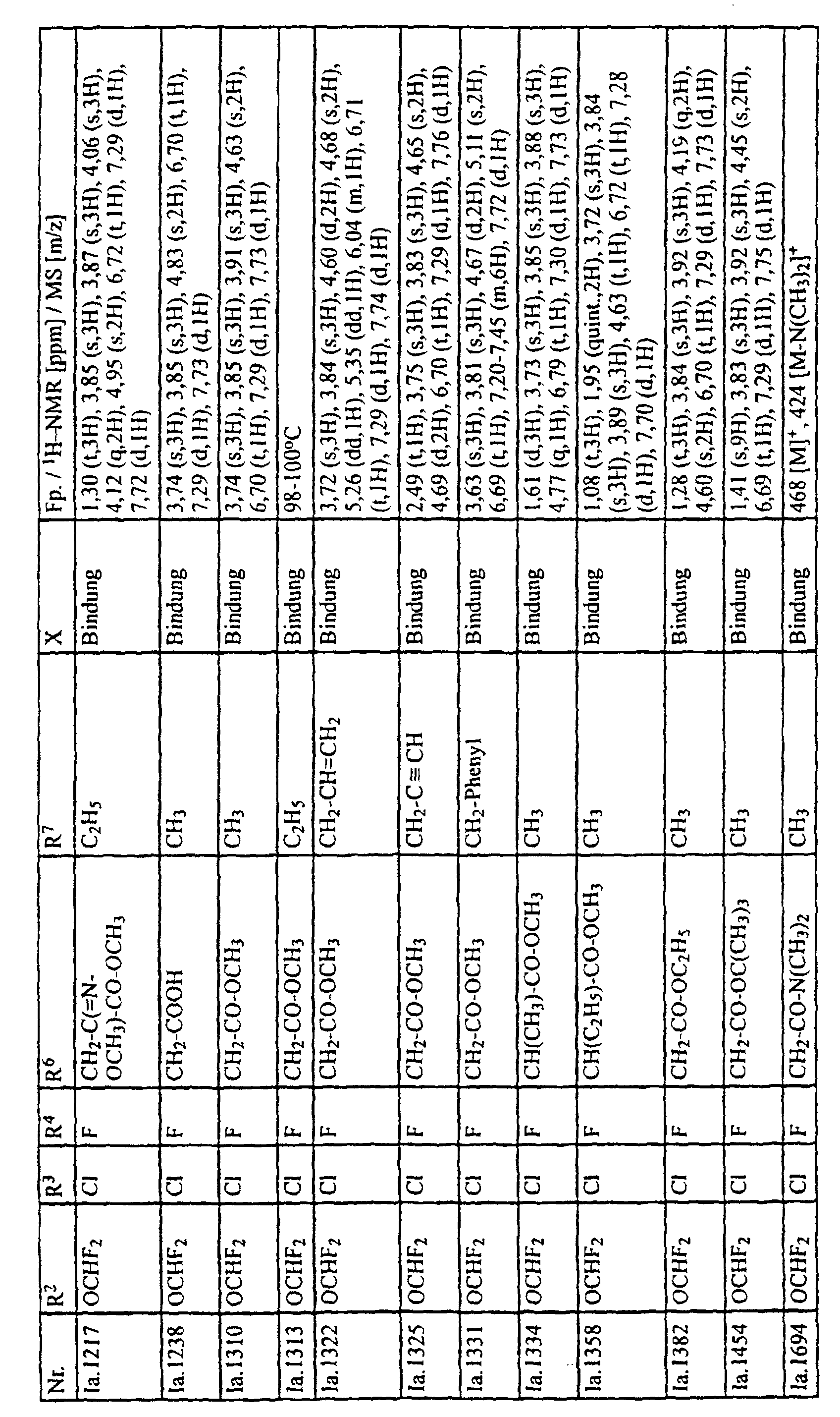

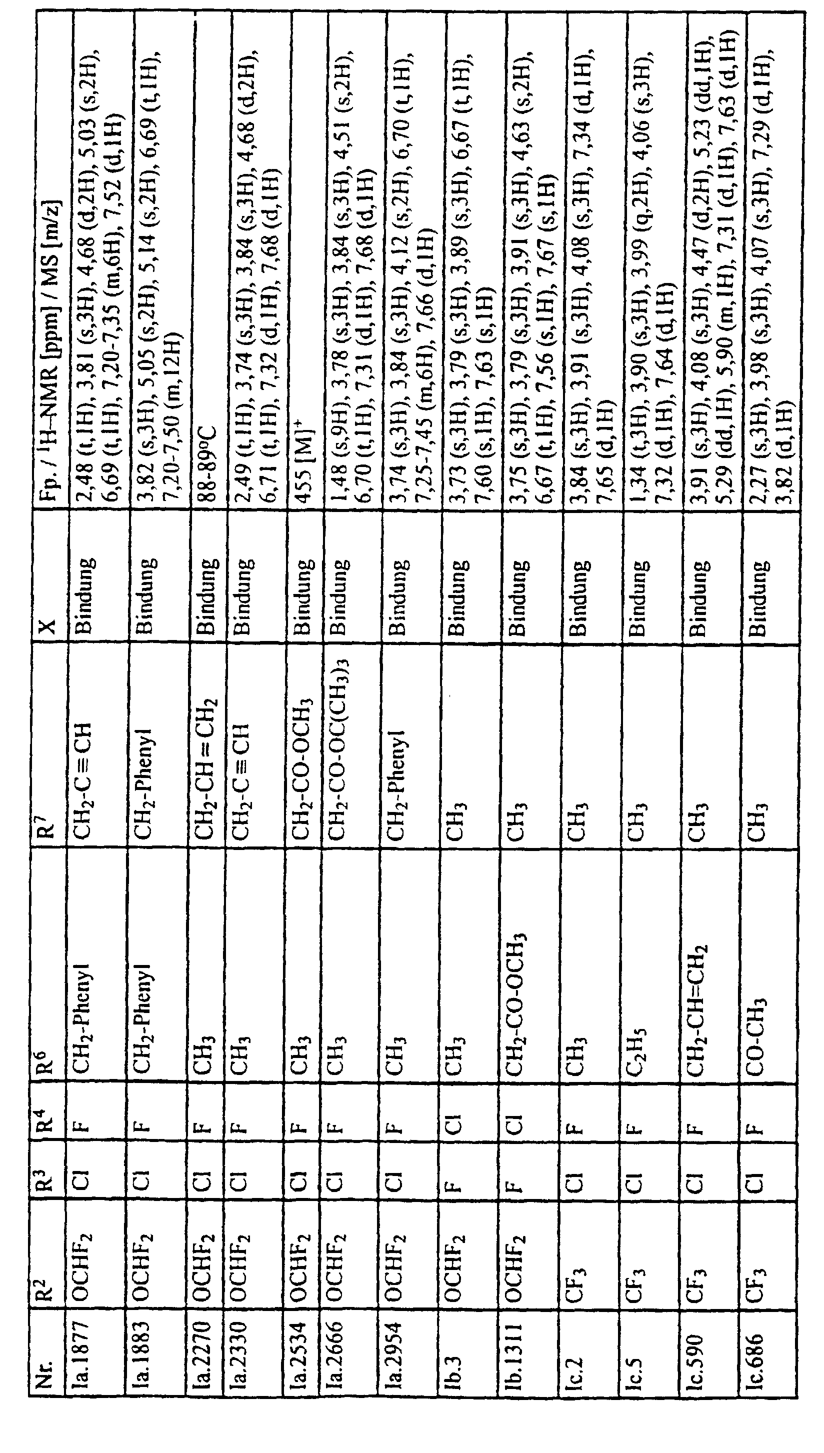

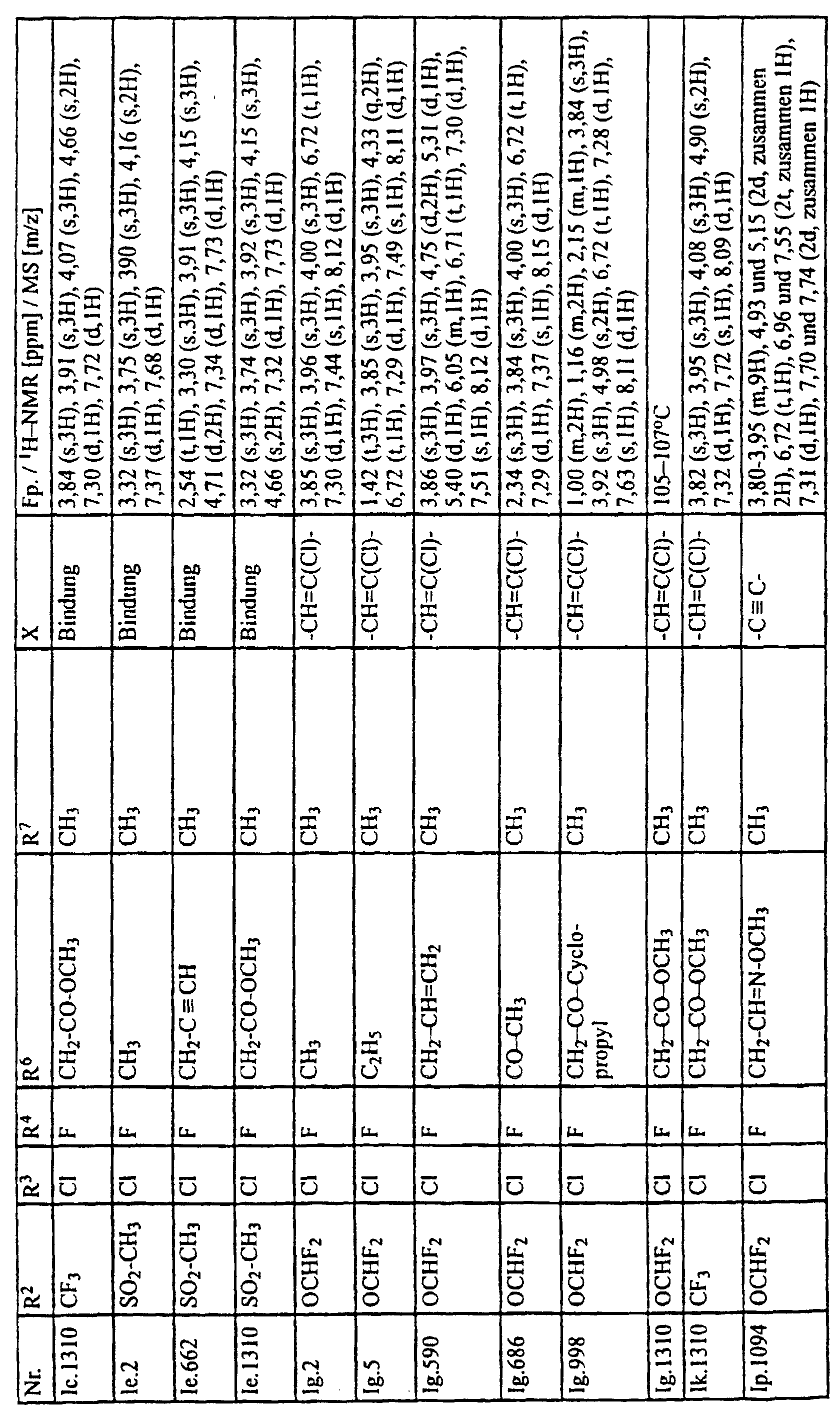

### Anwendungsbeispiele

Die herbizide Wirkung der substituierten 3-Phenylpyrazole I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 3,9 oder 1,9 g/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Amaranthus retroflexus | Zurückgekrümmter Fuchsschwanz | redroot pigweed |
| Galium aparine | Klettenlabkraut | catchweed bedstraw |
| Sinapis alba | Weißer Senf | white mustard |
| Solanum nigrum | Schwarzer Nachtschatten | black nightshade |

Bei Aufwandmengen von 3,9 und 1,9 g/ha a.S. zeigten die Verbindungen Nr. Ig.1310 und Ik.1310 im Nachauflaufverfahren eine sehr gute herbizide Wirkung gegen die o.g. breitblättrigen Pflanzen.

## Patentansprüche

1. Substituierte 3-Phenylpyrazole der Formel I 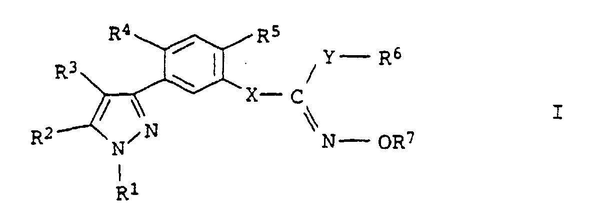 in der die Variablen folgende Bedeutungen haben:
R¹ C₁-C₄-Alkyl oder C₁-C₄-Halogenalkyl;
R² Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
R³ Wasserstoff, Cyano, Halogen, C₁-C₄-Alkyl oder C₁-C₄-Halogenalkyl;
R⁴ Wasserstoff oder Halogen;
R⁵ Cyano, Halogen, C₁-C₄-Alkyl, C₁-C₄-Halogenalkyl, C₁-C₄-Alkoxy oder C₁-C₄-Halogenalkoxy;
R⁶, R⁷ unabhängig voneinander C₁-C₆-Alkyl, C₁-C₆-Halogenalkyl, Hydroxy-C₁-C₄-alkyl, Cyano-C₁-C₄-alkyl, C₁-C₄-Alkoxy-C₁-C₄-alkyl, C₁-C₄-Halogenalkoxy-C₁-C₄-alkyl, C₃-C₄-Alkenyloxy-C₁-C₄-alkyl, C₃-C₄-Alkinyloxy-C₁-C₄-alkyl, C₃-C₈-Cycloalkyloxy-C₁-C₄-alkyl, Amino-C₁-C₄-alkyl, C₁-C₄-Alkylamino-C₁-C₄-alkyl, Di-(C₁-C₄-alkyl)amino-C₁-C₄-alkyl, C₁-C₄-Alkylthio-C₁-C₄-alkyl, C₁-C₄-Halogenalkylthio-C₁-C₄-alkyl, C₃-C₄-Alkenylthio-C₁-C₄-alkyl C₃-C₄-Alkinylthio-C₁-C₄-alkyl, C₁-C₄-Alkylsulfinyl-C₁-C₄-alkyl, C₁-C₄-Halogenalkylsulfinyl-C₁-C₄-alkyl, C₃-C₄-Alkenylsulfinyl-C₁-C₄-alkyl, C₃-C₄-Alkinylsulfinyl-C₁-C₄-alkyl, C₁-C₄-Alkylsulfonyl-C₁-C₄-alkyl, C₁-C₄-Halogenalkylsulfonyl-C₁-C₄-alkyl, C₃-C₄-Alkenylsulfonyl-C₁-C₄-alkyl, C₃-C₄-Alkinylsulfonyl-C₁-C₄-alkyl, C₃-C₆-Alkenyl, Cyano-C₃-C₆-alkenyl, C₃-C₆-Halogenalkenyl, C₃-C₆-Alkinyl, Cyano-C₃-C₆-alkinyl, C₃-C₆-Halogenalkinyl, (C₁-C₄-Alkyl)carbonyl, (C₁-C₄-Halogenalkyl)carbonyl, (C₁-C₄-Alkoxy)carbonyl, Aminocarbonyl, (C₁-C₄-Alkyl)aminocarbonyl, Di(C₁-C₄-alkyl)aminocarbonyl, (C₁-C₄-Alkoxy-C₁-C₄-alkyl)carbonyl, C₁-C₄-Alkylsulfonyl, C₁-C₄-Halogenalkylsulfonyl, (C₁-C₄-Alkyl)carbonyl-C₁-C₄-alkyl, (C₁-C₄-Halogenalkyl)carbonyl-C₁-C₄-alkyl, (C₃-C₈-Cycloalkyl)carbonyl-C₁-C₄-alkyl, (C₁-C₄-Alkoxy)imino-C₁-C₄-alkyl, (C₃-C₄-Alkenyloxy)imino-C₁-C₄-alkyl, Hydroxycarbonyl-C₁-C₄-alkyl, (C₁-C₄-Alkoxy)carbonyl-C₁-C₄-alkyl, das eine (C₁-C₄-Alkoxy)imino-Gruppe tragen kann, (C₁-C₄-Halogenalkoxy)carbonyl-C₁-C₄-alkyl (C₁-C₄-Alkylthio)carbonyl-C₁-C₄-alkyl, Aminocarbonyl-C₁-C₄-alkyl, (C₁-C₄-Alkylamino)carbonyl-C₁-C₄-alkyl, Di(C₁-C₄-alkylamino)carbonyl-C₁-C₄-alkyl,
C₃-C₈-Cycloalkyl, C₃-C₈-Cycloalkyl-C₁-C₄-alkyl, Phenyl, Phenyl-C₁-C₄-alkyl oder 3- bis 7-gliedriges Heterocyclyl oder Heterocyclyl-C₁-C₄-alkyl, wobei alle Cycloalkylringe und Heterocyclen gewünschtenfalls ein Carbonyl- oder Thiocarbonyl-Ringglied enthalten können,
und wobei jeder Cycloalkyl-, Phenyl- oder Heterocyclylring unsubstituiert sein oder ein bis vier Substituenten tragen kann, jeweils ausgewählt aus der Gruppe bestehend aus Cyano, Nitro, Amino, Hydroxy, Carboxy, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy und Di(C₁-C₄-alkyl)amino;
X eine chemische Bindung, eine 1,2-Ethindiyl-Gruppe oder eine über die mit Stern gekennzeichnete Bindung an den Phenylring gebundene Gruppe *-CH₂-CH(R⁸)- oder *-CH=C(R⁸)-, wobei R⁸ für Wasserstoff, Cyano, Nitro, Halogen, C₁-C₄-Alkyl oder C₁-C₄-Halogenalkyl steht;
Y Sauerstoff oder Schwefel;
sowie die landwirtschaftlich brauchbaren Salze der Verbindungen I.

2. Verwendung der substituierten 3-Phenylpyrazole I und ihrer landwirtschaftlich brauchbaren Salze, gemäß Anspruch 1, als Herbizide.

3. Herbizides Mittel, enthaltend eine herbizid wirksame Menge mindestens eines substituierten 3-Phenylpyrazols der Formel I oder eines landwirtschaftlich brauchbaren Salzes von I, gemäß Anspruch 1, und mindestens einen flüssigen und/oder festen Trägerstoff sowie gewünschtenfalls mindestens einen oberflächenaktiven Stoff.

4. Verfahren zur Herstellung von herbizid wirksamen Mitteln, **dadurch gekennzeichnet, daß** man eine herbizid wirksame Menge mindestens eines substituierten 3-Phenylpyrazols der Formel I oder eines landwirtschaftlich brauchbaren Salzes von I, gemäß Anspruch 1, mit mindestens einem flüssigen und/oder festen Trägerstoff sowie gewünschtenfalls mindestens einem oberflächenaktiven Stoff mischt.

5. Verfahren zur Bekämpfung von unerwünschtem Pflanzenwuchs, **dadurch gekennzeichnet, daß** man eine herbizid wirksame Menge mindestens eines substituierten 3-Phenylpyrazols der Formel I oder eines landwirtschaftlich brauchbaren Salzes von I, gemäß Anspruch 1, auf Pflanzen, deren Lebensraum oder auf Saatgut einwirken läßt.

6. Verfahren zur Herstellung von substituierten 3-Phenylpyrazolen der Formel I gemäß Anspruch 1, **dadurch gekennzeichnet, daß** man Alkoxyamide der Formel III 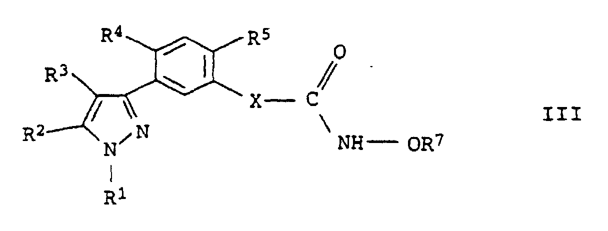 in Gegenwart einer Base mit einem Alkylierungs- oder Acylierungsmittel L-R⁶ umsetzt,
wobei L für eine übliche Abgangsgruppe steht und R¹ bis R⁷ die in Anspruch 1 angegebenen Bedeutungen haben.

## Claims

1. A substituted 3-phenylpyrazole of the formula I 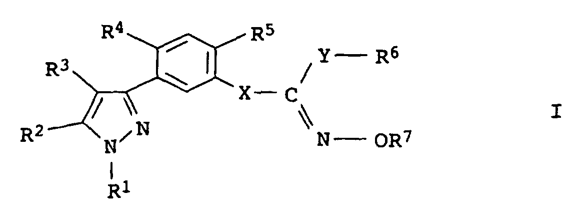 in which the variables have the following meanings:
R¹ is C₁-C₄-alkyl or C₁-C₄-haloalkyl;
R² is cyano, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-haloalkylsulfinyl, C₁-C₄-alkylsulfonyl or C₁-C₄-haloalkylsulfonyl;
R³ is hydrogen, cyano, halogen, C₁-C₄-alkyl or C₁-C₄-haloalkyl;
R⁴ is hydrogen or halogen;
R⁵ is cyano, halogen, C₁-C₄-alkyl, C₁-C₄-haloalkyl, C₁-C₄-alkoxy or C₁-C₄-haloalkoxy;
R⁶, R⁷ independently of one another are C₁-C₆-alkyl, C₁-C₆-haloalkyl, hydroxy-C₁-C₄-alkyl, cyano-C₁-C₄-alkyl, C₁-C₄-alkoxy-C₁-C₄-alkyl, C₁-C₄-haloalkoxy-C₁-C₄-alkyl, C₃-C₄-alkenyloxy-C₁-C₄-alkyl, C₃-C₄-alkynyloxy-C₁-C₄-alkyl, C₃-C₈-cycloalkyloxy-C₁-C₄-alkyl, amino-C₁-C₄-alkyl, C₁-C₄-alkylamino-C₁-C₄-alkyl, di(C₁-C₄-alkyl)amino-C₁-C₄-alkyl, C₁-C₄-alkylthio-C₁-C₄-alkyl, C₁-C₄-haloalkylthio-C₁-C₄-alkyl, C₃-C₄-alkenylthio-C₁-C₄-alkyl, C₃-C₄-alkynylthio-C₁-C₄-alkyl, C₁-C₄-alkylsulfinyl-C₁-C₄-alkyl, C₁-C₄-haloalkylsulfinyl-C₁-C₄-alkyl, C₃-C₄-alkenylsulfinyl-C₁-C₄-alkyl, C₃-C₄-alkynylsulfinyl-C₁-C₄-alkyl, C₁-C₄-alkylsulfonyl-C₁-C₄-alkyl, C₁-C₄-haloalkylsulfonyl-C₁-C₄-alkyl, C₃-C₄-alkenylsulfonyl-C₁-C₄-alkyl, C₃-C₄-alkynylsulfonyl-C₁-C₄-alkyl, C₃-C₆-alkenyl, cyano-C₃-C₆-alkenyl, C₃-C₆-haloalkenyl, C₃-C₆-alkynyl, cyano-C₃-C₆-alkynyl, C₃-C₆-haloalkynyl, (C₁-C₄-alkyl)-carbonyl, (C₁-C₄-haloalkyl)carbonyl, (C₁-C₄-alkoxy)carbonyl, aminocarbonyl, (C₁-C₄-alkyl)aminocarbonyl, di(C₁-C₄-alkyl)aminocarbonyl, (C₁-C₄-alkoxy-C₁-C₄-alkyl)carbonyl, C₁-C₄-alkylsulfonyl, C₁-C₄-haloalkylsulfonyl, (C₁-C₄-alkyl)carbonyl-C₁-C₄-alkyl, (C₁-C₄-haloalkyl)carbonyl-C₁-C₄-alkyl, (C₃-C₈-cycloalkyl)carbonyl-C₁-C₄-alkyl, (C₁-C₄-alkoxy)imino-C₁-C₄-alkyl, (C₃-C₄-alkenyloxy)imino-C₁-C₄-alkyl, hydroxycarbonyl-C₁-C₄-alkyl, (C₁-C₄-alkoxy)carbonyl-C₁-C₄-alkyl, which may bear a (C₁-C₄-alkoxy)imino group, (C₁-C₄-haloalkoxy)carbonyl-C₁-C₄-alkyl, (C₁-C₄-alkylthio)carbonyl-C₁-C₄-alkyl, aminocarbonyl-C₁-C₄-alkyl, (C₁-C₄-alkylamino)carbonyl-C₁-C₄-alkyl, di(C₁-C₄-alkyl-amino)carbonyl-C₁-C₄-alkyl, C₃-C₈-cycloalkyl, C₃-C₈-cycloalkyl-C₁-C₄-alkyl, phenyl, phenyl-C₁-C₄-alkyl or 3- to 7-membered heterocyclyl or heterocyclyl-C₁-C₄-alkyl, it being possible, if desired, for all cycloalkyl rings and heterocycles to contain a carbonyl or thiocarbonyl ring member,
and it being possible for each cycloalkyl, phenyl or heterocyclyl ring to be unsubstituted or to have attached to it one to four substituents, in each case selected from the group consisting of cyano, nitro, amino, hydroxyl, carboxyl, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfonyl, C₁-C₄-haloalkylsulfonyl, (C₁-C₄-alkoxy)carbonyl, (C₁-C₄-alkyl)carbonyl, (C₁-C₄-haloalkyl)carbonyl, (C₁-C₄-alkyl)carbonyloxy, (C₁-C₄-haloalkyl)carbonyloxy and di(C₁-C₄-alkyl)amino;
X is a chemical bond, a 1,2-ethynediyl group or a group *-CH₂-CH(R⁸)- or *-CH=C(R⁸)- which is bonded to the phenyl ring via the bond characterized with an asterisk, where R⁸ is hydrogen, cyano, nitro, halogen, C₁-C₄-alkyl or C₁-C₄-haloalkyl;
Y is oxygen or sulfur;
or an agriculturally useful salt thereof.

2. The use of a substituted 3-phenylpyrazole I or an agriculturally useful salt thereof as claimed in claim 1 as a herbicide.

3. A herbicidal composition comprising a herbicidally active amount of at least one substituted 3-phenylpyrazole of the formula I or of an agriculturally useful salt of I as claimed in claim 1 and at least one liquid and/or solid carrier and, if desired, at least one surfactant.

4. A process for the preparation of herbicidally active compositions which comprises mixing a herbicidally active amount of at least one substituted 3-phenylpyrazole of the formula I or of an agriculturally useful salt of I as claimed in claim 1 with at least one liquid and/or solid carrier and, if desired, at least one surfactant.

5. A method of controlling undesirable vegetation, which comprises allowing a herbicidally active amount of at least one substituted 3-phenolpyrazole of the formula I or of an agriculturally useful salt of I as claimed in claim 1 to act on the plants, their environment or on seed.

6. A process for the preparation of substituted 3-phenylpyroazoles of the formula I as claimed in claim 1, which comprises reacting alkoxyamides of the formula III 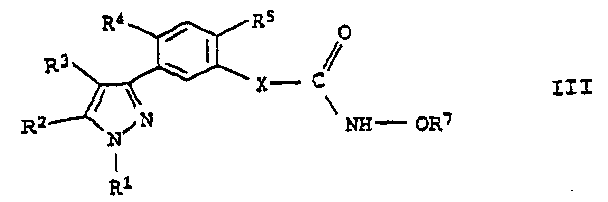 with an alkylating or acylating agent L-R⁶, where L is a customary leaving group and R¹ to R⁷ are as defined in claim 1, in the presence of a base.

## Revendications

1. 3-phénylpyrazoles substitués de formule I 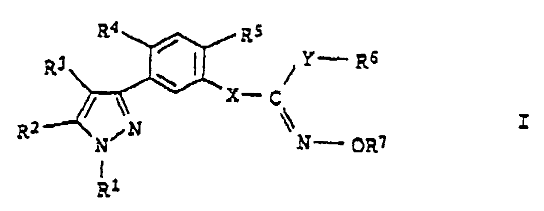 dans laquelle les symboles ont les significations suivantes :
R¹ : un groupe alkyle en C1-C4 ou halogénoalkyle en C1-C4 ;
R² : un groupe cyano, alkyle en C1-C4, halogénoalkyle en C1-C4, alcoxy en C1-C4, halogénoalcoxy en C1-C4, alkylthio en C1-C4, halogénoalkylthio en C1-C4, alkylsulfinyle en C1-C4, halogénoalkylsulfinyle en C1-C4, alkylsulfonyle en C1-C4 ou halogénoalkylsulfonyle en C1-C4 ;
R³ . l'hydrogène, un groupe cyano, un halogène, un groupe alkyle en C1-C4 ou halogénoalkyle en C1-C4 ;
R⁴ : l'hydrogène ou un halogène ;
R⁵ : un groupe cyano, un halogène, un groupe alkyle en C1-C4, halogénoalkyle en C1-C4, alcoxy en C1-C4 ou halogénoalcoxy en C1-C4 ;
R6, R7, chacun, indépendamment l'un de l'autre :
un groupe alkyle en C1-C6, halogénoalkyle en C1-C6, hydroxyalkyle en C1-C4, cyano-alkyle en C1-C4, (alcoxy en C1-C4)alkyle en C1-C4, (halogénoalcoxy en C1-C4)-alkyle en C1-C4, (alcényloxy en C3-C4)alkyle en C1-C4, (alcynyloxy en C3-C4)alkyle en C1-C4, (cycloalcoxy en C3-C8)alkyle en C1-C4, aminoalkyle en C1-C4, (alkyle en C1-C4)aminoalkyle en C1-C4, di-(alkyle en C1-C4)amino-alkyle en C1-C4, (alkylthio en C1-C4)alkyle en C1-C4, (halogénoalkylthio en C1-C4)alkyle en C1-C4, (alcénylthio en C3-C4)alkyle en C1-C4, (alcynyle en C3-C4)thioalkyle en C1-C4, (alkylsulfonyle en C1-C4)alkyle en C1-C4, (halogénoalkylsulfinyle en C1-C4)alkyle en C1-C4, (alcénylsulfinyle en C3-C4)alkyle en C1-C4, (alcynylsulfinyle en C3-C4)alkyle en C1-C4, (alkylsulfonyle en C1-C4)alkyle en C1-C4, (halogénoalkylsulfonyle en C1-C4)alkyle en C1-C4, (alcénylsulfonyle en C3-C4)alkyle en C1-C4, (alcynylsulfonyle en C1-C4)alkyle en C1-C4, alcényle en C3-C6, cyano-alcényle en C3-C6, halogénoalcényle en C3-C6, alcynyle en C3-C6, cyano-alcynyle en C3-C6, halogénoalcynyle en C3-C6, (alkyle en C1-C4)carbonyle, (halogénoalkyle en C1-C4)carbonyle, (alcoxy en C1-C4)carbonyle, aminocarbonyle, (alkyle en C1-C4)aminocarbonyle, di-(alkyle en C1-C4)aminocarbonyle, (alcoxy en C1-C4)-(alkyle en C1-C4)-carbonyle, alkylsulfonyle en C1-C4, halogénoalkylsulfonyle en C1-C4, (alkyle en C1-C4)carbonyl-alkyle en C1-C4, (halogénoalkyle en C1-C4)-carbonyl-alkyle en C1-C4, (cycloalkyle en C3-C8)carbonyl-alkyle en C1-C4, (alcoxy en C1-C4)imino-alkyle en C1-C4, (alcényloxy en C3-C4)imino-alkyle en C1-C4, hydroxycarbonyl-alkyle en C1-C4, (alcoxy en C1-C4)carbonylalkyle en C1-C4, qui peut porter un groupe (alcoxy en C1-C4)imino, (halogénoalcoxy en C1-C4)carbonyl-alkyle en C1-C4, (alkylthio en C1-C4)carbonyle en C1-C4, aminocarbonyl-alkyle en C1-C4, (alkylamino en C1-C4)carbonylalkyle en C1-C4, di-(alkylamino en C1-C4)carbonylalkyle en C1-C4,
un groupe cycloalkyle en C3-C8, (cycloalkyle en C3-C8)alkyle en C1-C4, phényle, phényl-alkyle en C1-C4 ou hétérocyclyle de trois à sept chaînons ou hétérocyclyl-alkyle en C1-C4, tous les noyaux cycloalkyle et hétérocycliques pouvant contenir éventuellement un chaînon carbonyle ou thiocarbonyle,
chacun des noyaux cycloalkyle, phényle ou hétérocyclyle pouvant être non substitué ou pouvant porter un à quatre substituants choisis parmi les groupes cyano, nitro, amino, hydroxy, carboxy, les halogènes, les groupes alkyle en C1-C4, halogénoalkyle en C1-C4, alcoxy en C1-C4, halogénoalcoxy en C1-C4, alkylthio en C1-C4, halogénoalkylthio en C1-C4, alkylsulfonyle en C1-C4, halogénoalkylsulfonyle en C1-C4, (alcoxy en C1-C4)carbonyle, (alkyle en C1-C4)carbonyle, (halogénoalkyle en C1-C4)carbonyle, (alkyle en C1-C4)carbonyloxy, (halogénoalkyle en C1-C4)carbonyloxy et di-(alkyle en C1-C4)amino ;
X représente une liaison chimique, un groupe 1,2-éthynediyle ou un groupe *-CH 2-CH(R⁸) ou *-CH=C(R⁸)- relié au cycle phényle par la liaison signalée par l'astérisque,
R⁸ représetnant l'hydrogène, un groupe cyano, nitro, un halogène, un groupe alkyle en C1-C4 ou halogénoalkyle en C1-C4 ;
Y représente l'oxygène ou le soufre ;
et les sels des composés I aptes aux applications agricoles.

2. Utilisation des 3-phénylpyraoles substitués I et de leurs sels aptes aux applications agricoles selon la revendication 1 en tant qu'herbicides.

3. Produit herbicide contenant une quantité herbicide efficace d'au moins un 3-phénylpyrazole substitué de formule I ou d'un sel de I apte aux applications agricoles selon la revendication 1 et au moins un véhicule solide et/ou liquide et le cas échéant au moins un agent tensio-actif.

4. Procédé pour la préparation de produits à activité herbicide **caractérisé en ce que** l'on mélange une quantité herbicide efficace d'au moins un 3-phénylpyrazole substitué de formule I ou d'un sel de I apte aux applications agricoles selon revendication 1, avec au moins un véhicule solide et/ou liquide et le cas échéant au moins un agent tensio-actif.

5. Procédé pour combattre les croissances de végétaux indésirables **caractérisé par le fait que** l'on fait agir une quantité herbicide efficace d'au moins un 3-phénylpyrazole substitué de formule I ou d'un sel de I apte aux :applications agricoles selon la revendication 1 sur les végétaux, leur habitat ou sur les semences.

6. Procédé pour la préparation des 3-phénylpyrazoles substitués de formule I selon la revendication 1, **caractérisé par le fait que** l'on fait réagir des alcoxyamides de formule III 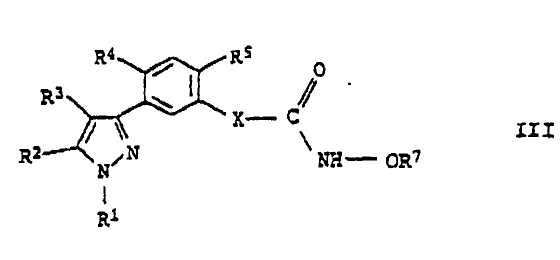 en présence d'une base avec un agent alkylant ou acylant L-R⁶,
L représentant un groupe éliminable usuel et R¹ à R⁷ ayant les significations indiquées dans la revendication 1.
